# EUROPÄISCHE PATENTANMELDUNG

(11) **EP 4 810 156 A1**
(43) Veröffentlichungstag der Anmeldung: **23.09.2026**
(21) Anmeldenummer: 25164634.5
(22) Anmeldetag: 19.03.2025
(51) Int. Cl.: B01J 31/18, C07C 45/50

(54) **LIGANDEN ZUR HYDROFORMYLIERUNG**

(71) Anmelder: Evonik Oxeno GmbH & Co. KG, 45772 Marl (DE)
(72) Erfinder: FRANKE, Robert, 45772 Marl (DE); BÜKER, Julia, 44803 Bochum (DE); SALE, Anna Chiara, 40474 Düsseldorf (DE); BÖRNER, Armin, 18059 Rostock (DE); HOLZ, Jens, 18196 Kessin (DE); ROMEIKE, Kerstin, 18059 Rostock (DE); WENZEL, Gudrun, 18196 Kessin (DE)
(74) Vertreter: Evonik Patent Association

(57) **Zusammenfassung**

Die Erfindung betrifft Verbindungen der Formeln (I-1) bis (I-44). Die Erfindung betrifft zudem die Verwendung der Verbindungen als Ligand eines Katalysators in einer Hydroformylierungsreaktion. Die vorliegende Erfindung betrifft zudem eine Substanz umfassend eine Verbindung der Formeln (I-1) bis (I-44) und die Verwendung dieser Substanz als Katalysator in einer Hydroformylierungsreaktion. Die vorliegende Erfindung betrifft darüber hinaus ein katalytisches Verfahren zur Herstellung von Aldehyden.

## Beschreibung

Die vorliegende Erfindung betrifft eine Verbindung gemäß den Formeln (I-1) bis (I-44) und die Verwendung der Verbindung als Ligand eines Katalysators in einer Hydroformylierungsreaktion. Die vorliegende Erfindung betrifft zudem eine Substanz und die Verwendung dieser Substanz als Katalysator in einer Hydroformylierungsreaktion. Die vorliegende Erfindung betrifft darüber hinaus ein Verfahren zur Herstellung von Aldehyden.

Die Hydroformylierung (auch Oxosynthese oder Roelen-Reaktion genannt) war die erste großtechnisch genutzte und heutzutage mit über 10 Mio. t/a mengenmäßig wichtigste Synthese, welche mit metallorganischen Katalysatoren durchgeführt wurde/wird. Bei der Hydroformylierung reagiert ein Synthesegas aus Wasserstoff und Kohlenstoffmonoxid mit Kohlenstoff-Doppelbindungen von Olefinen unter Bildung von Aldehyden; die Reaktion wird vorzugsweise durch Cobalt- oder Rhodiumkatalysatoren katalysiert.

Durch die Auswahl der Komponenten, insbesondere durch elektronische und sterische Eigenschaften von Metallatom und Liganden des metallorganischen Katalysators, kann die Hydroformylierung von Olefinen gesteuert und optimiert werden. Insbesondere kann durch die gezielte Auswahl der Liganden in metallorganischen Katalysatoren der Umsatz und auch die Selektivität gesteigert werden.

Als Liganden des Katalysators spielen insbesondere die Organobisphosphite eine herausragende Rolle (siehe R. Franke, D. Selent, A. Börner Chem. Rev. 2012, 112, 11, 5675-5732, https://doi.org/10.1021/cr3001803).

Die als Ligand eingesetzten Verbindungen sollen hierbei insbesondere einen hohen Umsatz der Olefine in Aldehyde gewährleisten.

Demnach liegt der vorliegenden Erfindung die primäre Aufgabe zu Grunde, neue Verbindungen herzustellen, die als Liganden in Katalysatorsystemen für die Hydroformylierung geeignet sind.

Entsprechend basiert die vorliegende Erfindung auch auf der Aufgabe, eine Substanz enthaltend die zuvor genannten Verbindungen anzugeben, welche katalytisch aktiv ist.

Die vorliegende Erfindung basiert zudem auf der Aufgabe, ein (katalytisches) Verfahren zur Herstellung von Aldehyden aus Olefinen anzugeben, wobei insbesondere der Umsatz optimiert werden soll.

Diese Aufgaben sind eng verknüpft mit der ergänzenden Aufgabenstellung, eine Verwendung für die zuvor genannten Verbindungen und/oder die zuvor genannten Substanzen anzugeben.

Weitere Aufgaben ergeben sich aus der nachfolgenden Beschreibung und den Patentansprüchen.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele. Soweit für einen erfindungsgemäßen Aspekt (Verbindung; Substanz; Verfahren zur Herstellung von Aldehyden; Verwendung einer erfindungsgemäßen Verbindung als Ligand eines Katalysators, Verwendung einer erfindungsgemäßen Substanz als Katalysator) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Der Gegenstand der vorliegenden Erfindung wird in den beigefügten Patentansprüchen und der vorliegenden Beschreibung definiert.

Der hierin verwendete Begriff "Ligand" hat die in der Komplexchemie übliche Bedeutung und bezeichnet ein Atom oder Molekül, welches sich über eine koordinative Bindung an ein zentrales Metallatom bzw. im Falle von verbrückenden und chelatisierenden Liganden an mehrere Metallatome binden kann.

Der hierin verwendete Begriff "Katalysator" hat die übliche Bedeutung und wird für Substanzen verwendet, die die Aktivierungsenergie einer bestimmten Reaktion herabsetzen und dadurch die Reaktionsgeschwindigkeit erhöhen, ohne bei der Reaktion verbraucht zu werden und ohne im Endprodukt zu erscheinen. Katalysatoren gehen definitionsgemäß unverändert aus der Reaktion hervor.

Die hierin beschriebenen Katalysatoren sind bevorzugt Metallkomplexe, die eines oder mehrere Metallatome (in Komplexen auch oft als "Zentralatom" bezeichnet) und einen oder mehrere Liganden enthalten.

Der hierin verwendete Begriff "Selektivität" bezeichnet den prozentualen Anteil eines spezifischen Produkts in einem Gemisch aus mehreren möglichen Produkten. Werden beispielsweise 100 Moleküle A als eingesetzter Ausgangsstoff sowohl in 25 Moleküle B als auch in 25 Moleküle C und 50 Moleküle D umgesetzt, so beträgt die Selektivität für die Moleküle B und C jeweils 25%, während die Selektivität für das Molekül D 50% beträgt.

Die vorliegende Erfindung betrifft insbesondere eine Verbindung, wobei die Verbindung ausgewählt ist aus den folgenden Formeln (I-1) bis (I-44)

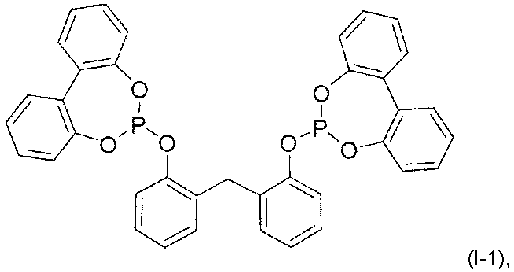

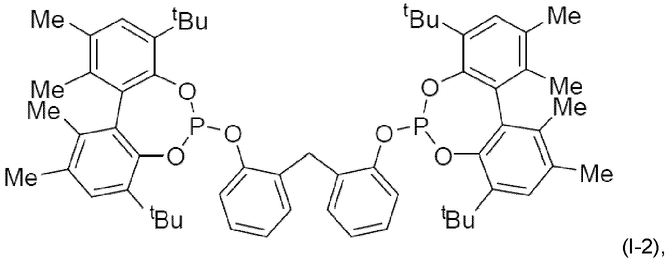

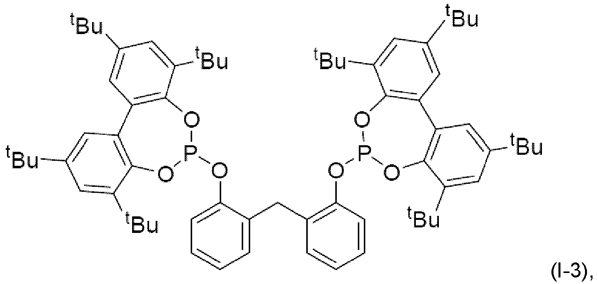

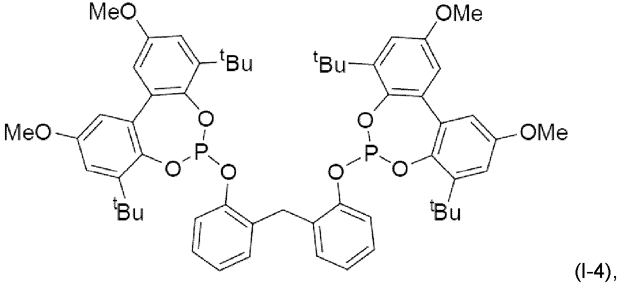

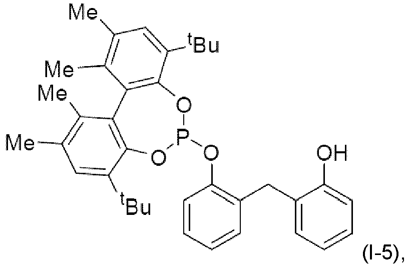

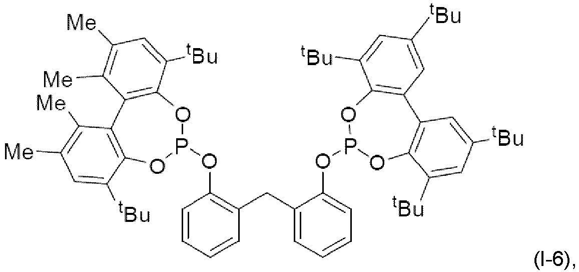

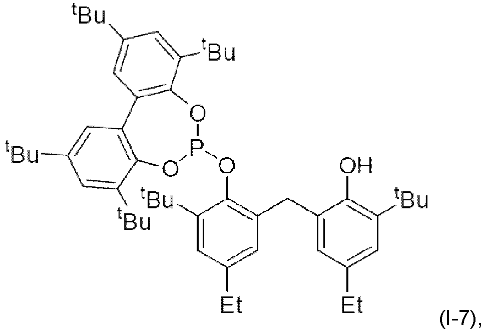

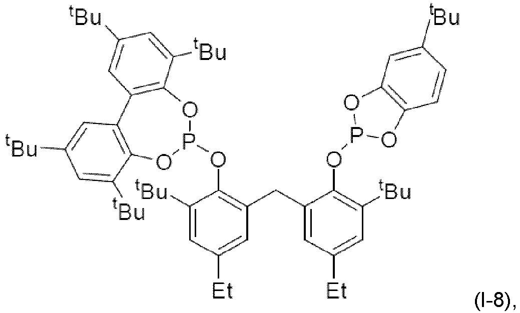

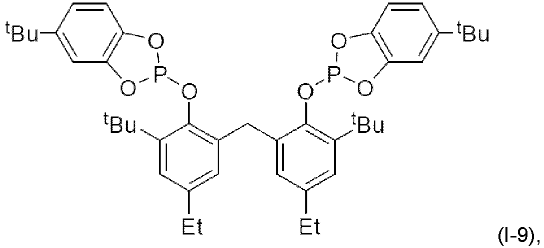

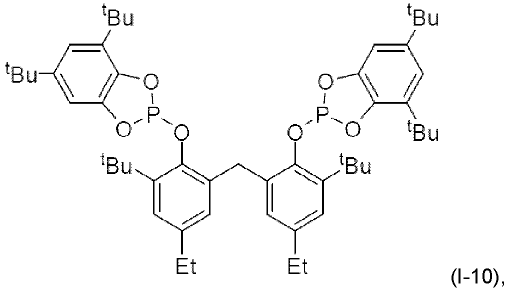

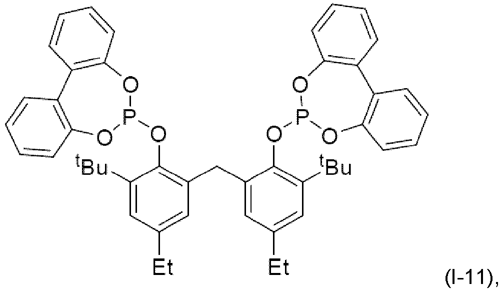

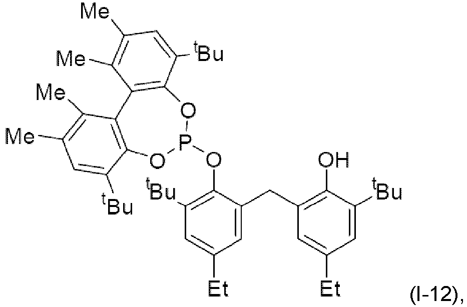

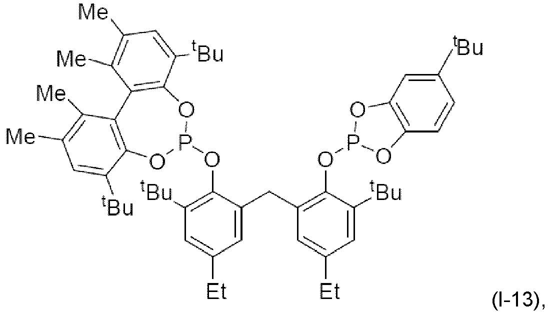

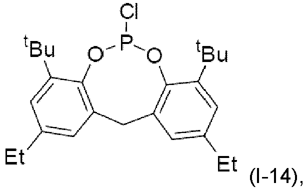

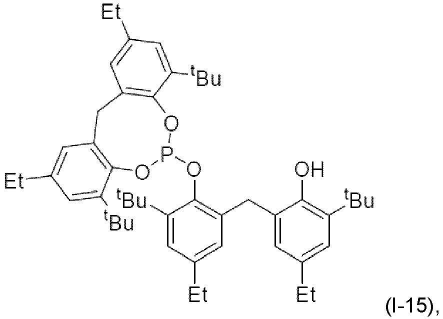

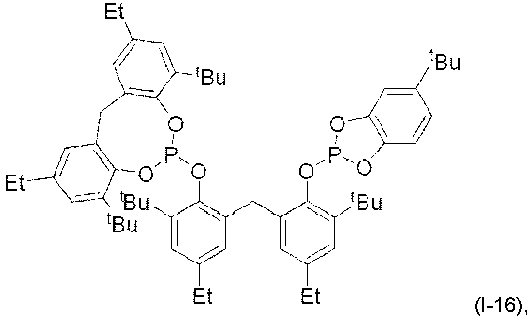

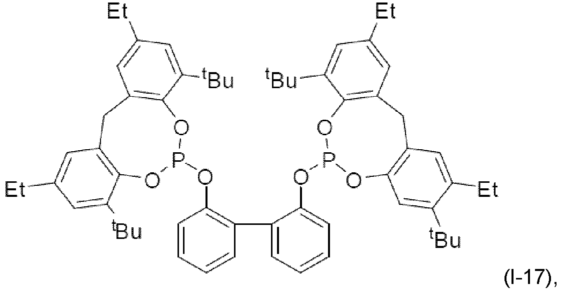

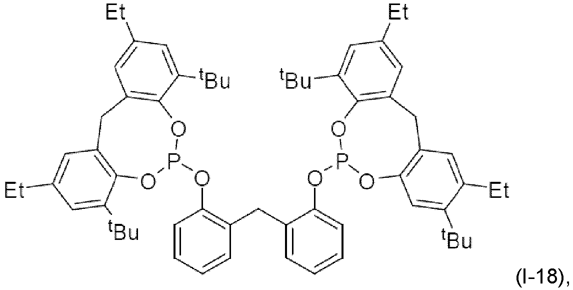

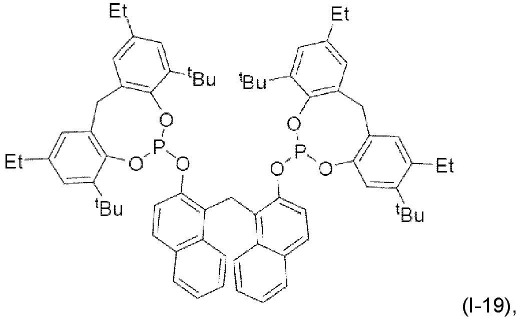

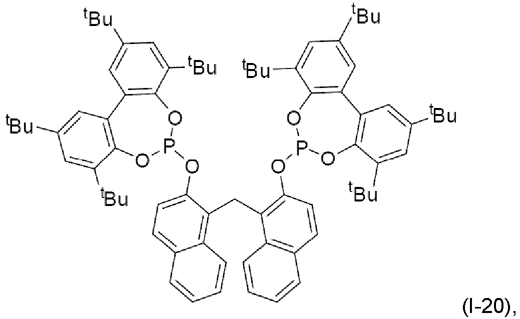

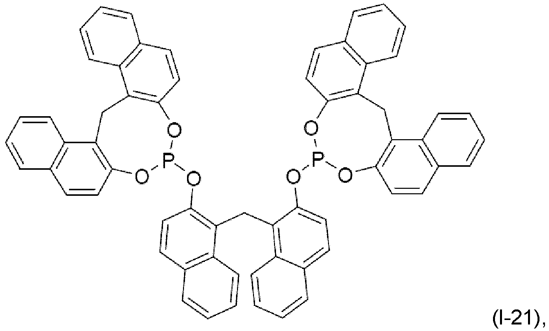

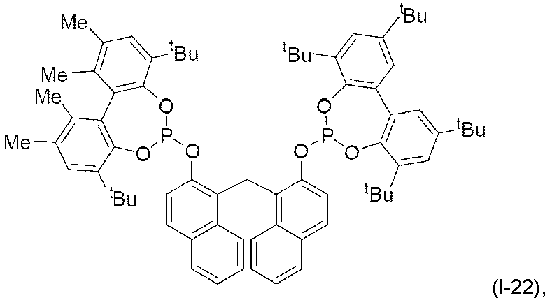

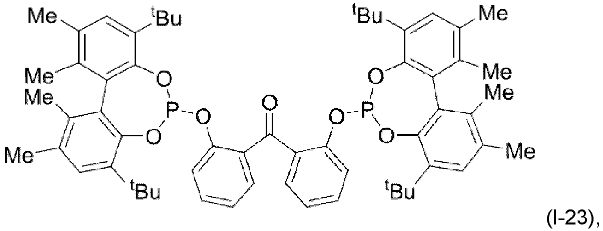

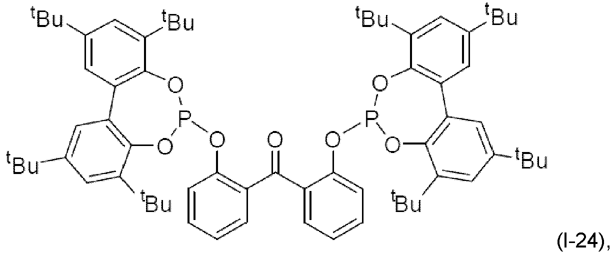

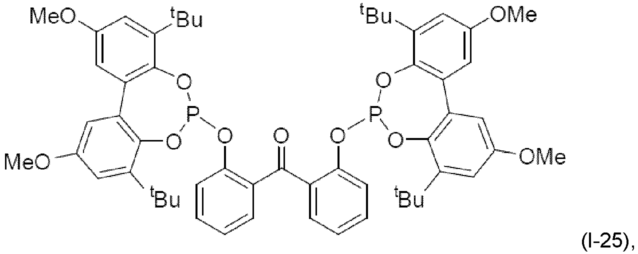

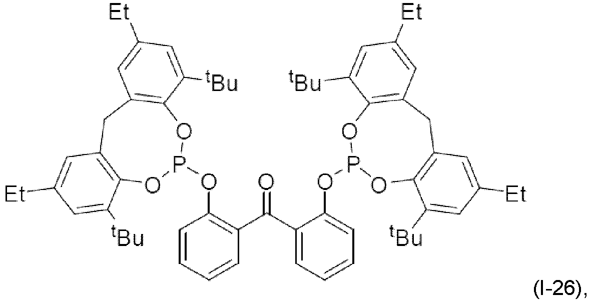

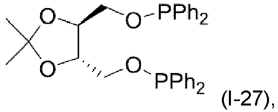

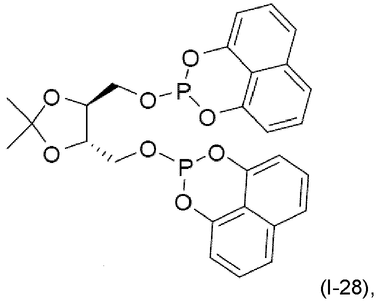

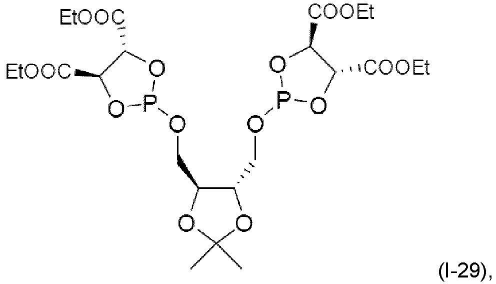

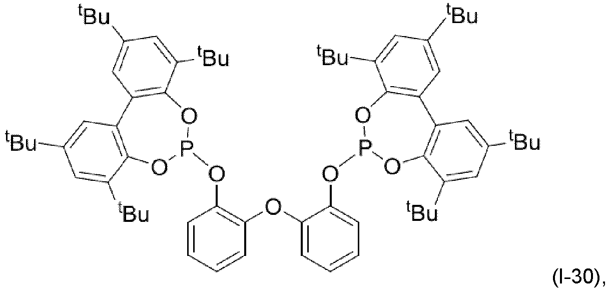

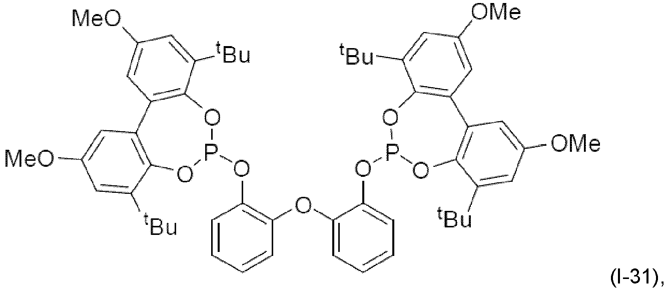

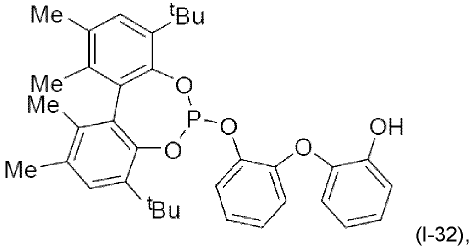

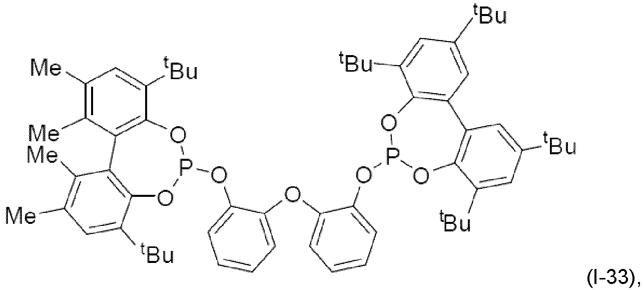

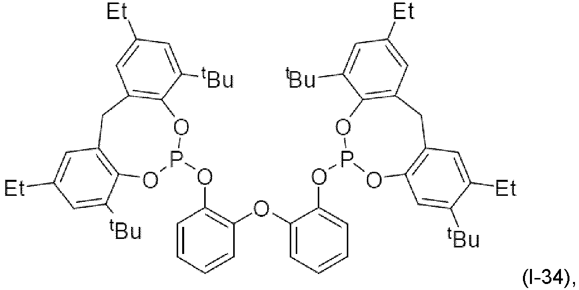

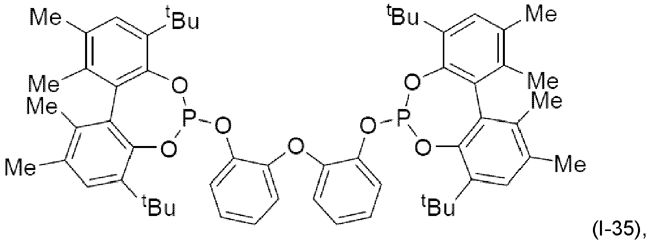

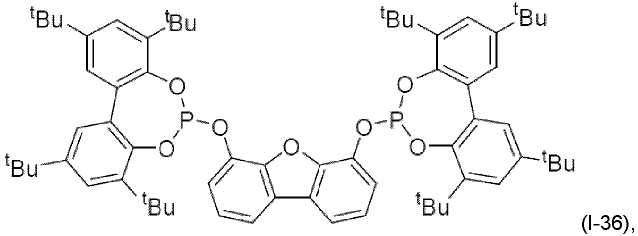

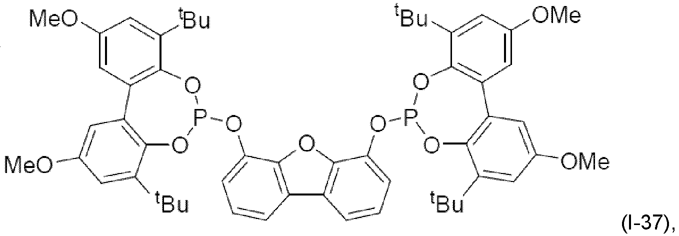

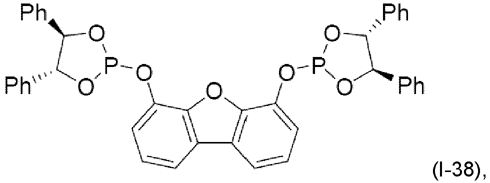

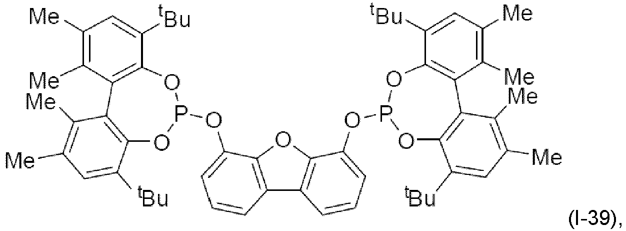

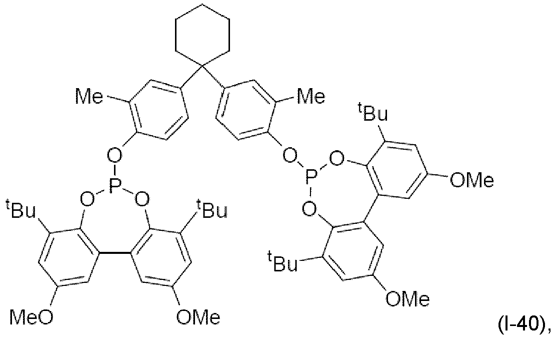

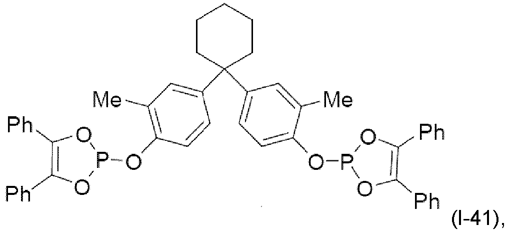

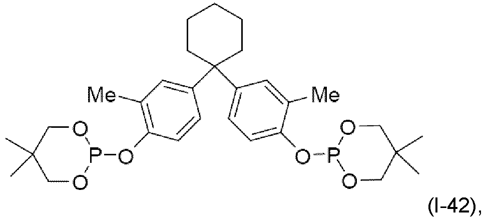

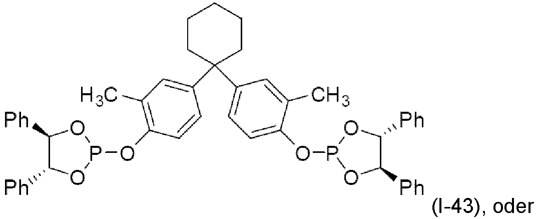

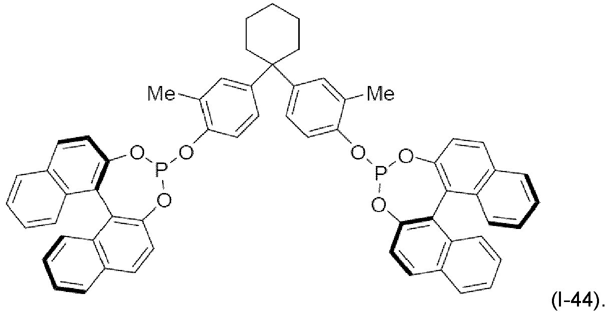

Die erfindungsgemäßen Verbindungen konnten erstmalig synthetisiert werden und haben sich in eigenen Untersuchungen als besonders vorteilhafte Liganden zur katalytischen Herstellung von Aldehyden aus Olefinen in einer Hydroformylierungsreaktion herausgestellt.

Die erfindungsgemäßen Verbindungen gemäß Formeln (I-1) bis (I-44) sind Phosphite, Biphosphite oder Phosphine mit unterschiedlichem Rückgrat und unterschiedlichen Substituenten. Ein Phosphit hat die allgemeine Form P(OR)₃ mit (R = Alkyl-Rest oder Aryl-Rest). Durch geschickte Wahl des Rückgrats und durch weiterhin geschickte Wahl von Substituenten der Phosphitgruppen, konnten im Rahmen der vorliegenden Erfindung für die Hydroformylierung besonders gut geeignete Liganden bereitgestellt werden. Die erfindungsgemäßen Verbindungen gemäß Formeln (I-1) bis (I-44) weisen bevorzugt zwei potentielle Bindungsstellen zu einem Metallzentralatom auf, liegen also in Form zweizähniger Liganden vor, was die daraus resultierende Metallkomplexe durch den sogenannten Chelateffekt besonders stabil und robust macht.

In einer ersten bevorzugten Ausgestaltung der vorliegenden Erfindung sind die erfindungsgemäßen Verbindungen Phosphite mit einem Methylen-verbrücktem Rückgrat, insbesondere mit einem Diphenylmethan-verbrücktem Rückgrat, dies betrifft die Verbindungen (I-1) bis (I-22).

In einer zweiten bevorzugten Ausgestaltung der vorliegenden Erfindung sind die erfindungsgemäßen Verbindungen Phosphite mit einem Hydroxyphenylmethanon-Rückgrat, dies betrifft die Verbindungen (I-23) bis (I-26).

In einer dritten bevorzugten Ausgestaltung der vorliegenden Erfindung sind die erfindungsgemäßen Verbindungen Phosphite oder Phosphine mit einem Dimethyldioxolan-Rückgrat, dies betrifft die Verbindungen (I-27) bis (I-29).

In einer vierten bevorzugten Ausgestaltung der vorliegenden Erfindung sind die erfindungsgemäßen Verbindungen Phosphite mit offenem oder geschlossenem Dibenzofuran-Rückgrat, dies betrifft die Verbindungen (I-30) bis (I-39).

In einer fünften bevorzugten Ausgestaltung der vorliegenden Erfindung sind die erfindungsgemäßen Verbindungen Phosphite mit Cyclohexan-Bis-Methylphenol-Rückgrat, dies betrifft die Verbindungen (I-40) bis (I-44).

Die im Rahmen der vorliegenden Erfindung durchgeführten Untersuchungen, vgl. Auch die unten gezeigten Beispiele zeigen, dass mit den erfindungsgemäßen Verbindungen (Liganden) deutlich bessere Umsätze bei der Synthese von Aldehyden aus Olefinen in einer Hydroformylierungsreaktion erzielt werden können als mit bekannten Systemen, insbesondere bessere Umsätze als mit Organobisphosphinit-Liganden (oft auch als "Bisphosphinit" bezeichnet).

Des Weiteren zeigen die mit diesen Liganden hergestellten Katalysatoren die vorteilhafte Eigenschaft interne Olefine (zum Beispiel 2-Penten) in einem nicht unerheblichen Anteil zu terminalen Aldehyden zu hydroformylieren, sogenannte n-Selektivität.

Die vorliegende Erfindung betrifft zudem eine Substanz, wobei die Substanz
i) eine Mischung ist, die Mischung umfassend
   - eine oder mehrere erfindungsgemäße Verbindungen,
      und
   - eines oder mehrere Metallatome,
   und/oder
ii) ein Metallkomplex ist oder einen Metallkomplex enthält, der Metallkomplex umfassend
   - eine oder mehrere erfindungsgemäße Verbindungen,
      und
   - eines oder mehrere Metallatome.

Erfindungsgemäße Substanzen in Form von Mischungen oder Metallkomplexen wie vorstehend und nachstehend definiert eignen sich insbesondere als Katalysatorsysteme in Hydroformylierungsreaktionen.

In erfindungsgemäßen Substanzen sind neben den erfindungsgemäßen Verbindungen zusätzlich eines oder mehrere Metallatome umfasst. Die erfindungsgemäßen Verbindungen, d.h. die Liganden, und die Metallatome (vor der Komplexbildung oft auch als "Metallprecursor" bezeichnet) bilden bevorzugt einen Metallkomplex, der katalytisch aktiv ist, insbesondere in einer Hydroformylierungsreaktion.

Der Metallkomplex kann *in situ* durch Zusammengeben des erfindungsgemäßen Liganden und eines Metallprecursors erzeugt werden (Variante i).

Der Metallkomplex mit dem entsprechenden Liganden kann jedoch auch gesondert hergestellt, isoliert und weiterverwendet, insbesondere als Katalysator eingesetzt werden, oder an Dritte verkauft werden (Variante ii).

Bevorzugt ist eine erfindungsgemäße Substanz, wobei das molare Verhältnis von Metallatomen zu Verbindungen gemäß Formeln (I-1) bis (I-44), d.h. den Liganden, in der Mischung und/oder im Metallkomplex im Bereich von 0,5:1 bis 1:4 liegt, bevorzugt im Bereich von 0,9:1 bis 1:2.1 liegt, besonders bevorzugt 1:1 oder 1:2 ist.

Eine solche erfindungsgemäße Substanz in Form einer Mischung oder eines Metallkomplexes eignet sich insbesondere als Katalysator zur Hydroformylierung. Die zuvor angegebenen bevorzugten molaren Verhältnisse von Metallatomen zu Verbindungen gemäß Formeln (I-1) bis (I-44) erlauben hierbei eine besonders effiziente Nutzung der Liganden und/oder Metallatome. Das molare Verhältnis sollte hierbei ausgeglichen sein. Ein hoher Überschuss an Liganden oder Metallatomen hingegen wäre in den meisten Fällen ineffizient, da die katalytische Aktivität der erfindungsgemäßen Substanzen meist aus dem Zusammenwirken eines Liganden mit einem Metallatom oder zweier Liganden mit einem Metallatom oder eines Liganden mit zwei Metallatomen oder entsprechender Mischfälle beruht.

Ebenfalls bevorzugt ist eine erfindungsgemäße Substanz, wobei das eine oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn, bevorzugt ist das eine oder eines der mehreren Metallatome Rhodium.

Bevorzugt ist das eine oder eines der mehreren Metallatome im Rahmen der vorliegenden Erfindung Cobalt oder Rhodium. Cobalt und Rhodium haben sich als Metallatom in Katalysatoren für die Hydroformylierung als besonders geeignet erwiesen.

Ebenfalls bevorzugt ist eine erfindungsgemäße Substanz in Form einer Mischung, wobei die Mischung als weiteren Bestandteil umfasst
- ein oder mehrere Olefine mit jeweils einer oder mehreren Kohlenstoff-Doppelbindungen, bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
   und/oder (bevorzugt "und)
- Lösungsmittel, wobei das Lösungsmittel vorzugsweise Toluol umfasst oder Toluol ist.

Vorgenannte bevorzugte Ausgestaltungen der erfindungsgemäßen Substanz in Form einer Mischung, zusätzlich umfassend eines oder mehrere Olefine mit jeweils einer oder mehreren Kohlenstoff-Doppelbindungen können direkt in einer Hydroformylierungsreaktion eingesetzt werden. Sie umfassen sowohl einen wie zuvor beschriebenen Katalysator als auch eine oder mehrere olefinische Ausgangsverbindungen. Bevorzugt umfasst die Mischung ebenfalls ein geeignetes Lösungsmittel, insbesondere Toluol, um die Reaktionsführung zu erleichtern.

In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde.

Diese Kohlenstoff-Doppelbindungen können sowohl in internen Olefinen wie zum Beispiel in 2-Penten vorliegen, als auch in endständigen Olefinen wie zum Beispiel in 1,3-Butadien vorliegen. Zudem können die als Ausgangsstoffe eingesetzten Olefine entweder eine oder auch mehrere Kohlenstoff-Doppelbindungen enthalten.

Mit den erfindungsgemäßen Substanzen bzw. Katalysatoren, die entweder bereits als Metallkomplex vorgelegt oder *in situ* gebildet werden, können sowohl Olefine mit einer einfachen Kohlenstoff-Doppelbindung (zum Beispiel 2-Penten) als auch Olefine mit mehreren Kohlenstoff-Doppelbindungen (zum Beispiel 1,3-Butadien) hydroformyliert werden.

Mit den erfindungsgemäßen Substanzen bzw. Katalysatoren können insbesondere die in diesem Text als bevorzugt benannten Olefine effektiv zu Aldehyden umgesetzt werden.

Die vorliegende Erfindung betrifft zudem ein Verfahren zur Herstellung von Aldehyden umfassend die Verfahrensschritte
a) Herstellen oder Bereitstellen eines Reaktionssystems umfassend
   a-1) eine erste Komponente umfassend ein oder mehrere Olefine mit jeweils einer oder mehreren Kohlenstoff-Doppelbindungen,
   a-2) eine Katalysatorkomponente umfassend eine erfindungsgemäße Verbindung und eines oder mehrere Metallatome,
   a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

Aus Olefinen können über Hydroformylierungsreaktionen Aldehyde gewonnen werden. In einer Hydroformylierungsreaktion findet formal die Addition eines Wasserstoffs (-H) und einer Formylgruppe (-CHO) an eine Kohlenstoff-Doppelbindung eines Moleküls statt; somit entstehen Aldehyde. Hierzu muss in Schritt a) ein Reaktionssystem hergestellt oder bereitgestellt werden.

Eine erste Komponente des Reaktionssystems a-1) umfasst eines oder mehrere Olefine mit jeweils einer oder mehreren Kohlenstoff-Doppelbindungen. Diese Komponente umfasst somit die Ausgangsstoffe bzw. Edukte, welche zu Aldehyden umgesetzt werden. Die eingesetzten Olefine können sowohl interne Kohlenstoff-Dippelbindungen (zum Beispiel 2-Penten), als auch endständige Kohlenstoff-Doppelbindungen (zum Beispiel 1,3-Butadien) haben. Zudem können die Olefine eine oder mehrere Kohlenstoff-Doppelbindungen enthalten.

Eine weitere Komponente des Reaktionssystems a-2), die sogenannte Katalysatorkomponente, umfasst eine erfindungsgemäße Verbindung und eines oder mehrere Metallatome bzw. umfasst eine erfindungsgemäße Substanz. Die erfindungsgemäße Verbindung und das eine oder die mehreren Metallatome bilden den Katalysator der Hydroformylierungsreaktion. Werden die erfindungsgemäße Verbindung und das eine oder die mehreren Metallatome in Form eines Metallprecursors separat in das Reaktionssystem gegeben, so bildet sich der Katalysator *in situ.* Es können jedoch auch bereits isolierte Metallkomplexe direkt eingesetzt werden.

Eine zweite Komponente des Reaktionssystems a-3) umfasst H₂ und CO, auch Reaktivkomponente oder Synthesegas genannt. Die Herstellung von Aldehyden aus Olefinen wird formal durch Addition eines Wasserstoffs (-H) und einer Formylgruppe (-CHO) an eine Kohlenstoff-Doppelbindung gewährleistet. In der Praxis erfolgt die Umsetzung durch Zugabe von Wasserstoff (H₂) und Kohlenstoffmonoxid (CO) als Reaktivkomponente bzw. Synthesegas.

Durch das Erwärmen in Schritt b) findet die Reaktion statt und die Olefine werden zu den entsprechenden Aldehyden umgesetzt, wobei die Katalysatorkomponente die Reaktion beschleunigt.

Bevorzugt ist ein erfindungsgemäßes Verfahren, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten.

Das erfindungsgemäße Verfahren eignet sich also zur Umsetzung einer Vielzahl an olefinischen Verbindungen.

Besonders bevorzugt ist ein erfindungsgemäßes Verfahren, wobei das eine oder die mehreren Olefine ausgewählt ist/sind der Gruppe bestehend aus cis- und/oder trans-2-Penten.

Das erfindungsgemäße Verfahren und/oder der Einsatz erfindungsgemäßer Verbindungen ist zudem geeignet zur Herstellung von linearen unverzweigten n-Aldehyden aus Olefinen mit endständigen und innenliegenden Doppelbindungen, sogenannte n-Selektivität.

Beispielsweise kann aus dem Ausgangsstoff 2-Penten mittels eines erfindungsgemäßen Verfahrens und/oder unter Einsatz erfindungsgemäßer Verbindungen und/oder Substanzen durch eine isomerisierende Hydroformylierung auch Hexanal hergestellt werden. Das interne Olefin 2-Penten wird also n-selektiv zu einem linearen unverzweigten n-Aldehyden umgesetzt. In vielen Industrieanwendungen werden diese linearen Aldehyde bevorzugt.

Bevorzugt ist ein erfindungsgemäßes Verfahren (vorzugsweise wie vorstehend als bevorzugt bezeichnet), wobei das eine oder eines der mehreren Metallatome der Katalysatorkomponente ausgewählt ist aus der Gruppe bestehend aus Rhodium, Cobalt, Eisen, Ruthenium, Osmium, Iridium, Platin und Zinn, bevorzugt ist das eine oder eines der mehreren Metallatome Rhodium, besonders bevorzugt wird das Rhodium bereitgestellt aus einer der folgenden Verbindungen: Rh(acac)(CO)₂, [(acac)Rh(COD)] und Rh₄CO₁₂.

Für die Hydroformylierung gemäß dem erfindungsgemäßen Verfahren können verschiedene Übergangsmetalle, wie Rhodium, Cobalt, Eisen, Ruthenium, Osmium, Iridium und Platin als Metallatom verwendet werden. Auch Zinn ist als Metallatom geeignet.

Bevorzugt ist das eine oder eines der mehreren Metallatome im Rahmen des Verfahrens der vorliegenden Erfindung Cobalt oder Rhodium. Cobalt und Rhodium haben sich als Metallatom in Katalysatoren für die Hydroformylierung als besonders geeignet erwiesen.

Das Rhodium wird innerhalb des erfindungsgemäßen Verfahrens bevorzugt aus den kommerziell erhältlichen und gut handhabbaren Verbindungen Rh(acac)(CO)₂, [(acac)Rh(COD)] und/oder Rh₄CO₁₂ bereitgestellt. Diese Verbindungen werden auch Metallprecusoren bezeichnet.

In einer ersten besonders bevorzugten Ausgestaltung der vorliegenden Erfindung wird das Rhodium bereitgestellt aus der Verbindung [(acac)Rh(COD)]; diese Verbindung entspricht (Acetylacetonat)(1,5-cyclooctadien)rhodium(I) mit der CAS-Nr. 12245-39-5.

In einer zweiten besonders bevorzugten Ausgestaltung der vorliegenden Erfindung wird das Rhodium bereitgestellt aus der Verbindung Rh(acac)(CO)₂; diese Verbindung entspricht (Acetylacetonat)dicar-bonylrhodium(I) mit der CAS-Nr. 14874-82-9.

Bevorzugt ist ein erfindungsgemäßes Verfahren (vorzugsweise wie vorstehend als bevorzugt bezeichnet), wobei das molare Verhältnis von Metallatomen zu Verbindungen gemäß Formeln (I-1) bis (I-44) in der Katalysatorkomponente im Bereich von 1:0,8 bis 1:4 liegt, bevorzugt im Bereich von 1:0,9 bis 1:2,1 liegt, besonders bevorzugt 1:1 oder 1:2 ist.

Die zuvor angegebenen bevorzugten molaren Verhältnisse von Metallatomen zu Verbindungen gemäß Formeln (I-1) bis (I-44) erlauben hierbei eine besonders effiziente Nutzung der erfindungsgemäßen Verbindung, d.h. der Liganden, und Metallatome. Das molare Verhältnis sollte hierbei ausgeglichen sein. Ein hoher Überschuss an Liganden oder Metallatomen hingegen wäre in den meisten Fällen ineffizient, da die katalytische Aktivität der erfindungsgemäßen Verbindungen meist aus dem Zusammenwirken eines Liganden mit einem Metallatom oder zweier Liganden mit einem Metallatom oder eines Liganden mit zwei Metallatomen oder entsprechender Mischfälle beruht.

Bevorzugt ist ein erfindungsgemäßes Verfahren (vorzugsweise wie vorstehend als bevorzugt bezeichnet), wobei die Katalysatorkomponente ein Metallkomplex ist oder einen Metallkomplex enthält, der Metallkomplex umfassend eine oder mehrere der erfindungsgemäßen Verbindungen.

Bevorzugt ist ein erfindungsgemäßes Verfahren (vorzugsweise wie vorstehend als bevorzugt bezeichnet), wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

Formal entspricht diese Hydroformylierungsreaktion der Addition eines Wasserstoffs (-H) und einer Formylgruppe (-CHO) an eine Kohlenstoff-Doppelbindung. Hierfür werden in der Praxis in der Regel nahezu äquimolare Gemische von Wasserstoff (H₂) und Kohlenstoffmonoxid (CO) eingesetzt. Das besonders bevorzugte Stoffmengenverhältnis von H₂ zu CO liegt daher im Bereich von 1,1:1 bis 1:1,1, entspricht also einem ausgewogenen Verhältnis der Reaktivkomponenten.

Bevorzugt ist ein erfindungsgemäßes Verfahren (vorzugsweise wie vorstehend als bevorzugt bezeichnet), wobei das Reaktionssystem in Verfahrensschritt b) unter einem Druck im Bereich von 1 bis 30 bar steht, bevorzugt unter einem Druck im Bereich von 1,5 bar bis 25 bar steht.

Bevorzugt ist ein erfindungsgemäßes Verfahren (vorzugsweise wie vorstehend als bevorzugt bezeichnet), wobei das Reaktionssystem in Verfahrensschritt b) auf eine Temperatur im Bereich von 80 °C bis 160 °C erwärmt wird, bevorzugt auf eine Temperatur im Bereich von 100 °C bis 140 °C erwärmt wird.

Bevorzugt ist ein erfindungsgemäßes Verfahren (vorzugsweise wie vorstehend als bevorzugt bezeichnet), wobei das Reaktionssystem zusätzlich ein Lösungsmittel umfasst, wobei das Lösungsmittel vorzugsweise Toluol umfasst oder Toluol ist.

Bevorzugt ist ein erfindungsgemäßes Verfahren (vorzugsweise wie vorstehend als bevorzugt bezeichnet), wobei der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem.

Bevorzugt ist ein erfindungsgemäßes Verfahren (vorzugsweise wie vorstehend als bevorzugt bezeichnet), wobei das Stoffmengenverhältnis von Rhodium zu den erfindungsgemäßen Verbindungen (vorzugsweise wie vorstehend als bevorzugt bezeichnet) in der Katalysatorkomponente im Bereich von 1:1 bis 1:3 liegt, bevorzugt im Bereich von 1:1,5 bis 1:2,5 liegt, besonders bevorzugt im Bereich von 1:1,9 bis 1:2,1 liegt.

Bevorzugt ist ein erfindungsgemäßes Verfahren (vorzugsweise wie vorstehend als bevorzugt bezeichnet), umfassend die Verfahrensschritte
V1) Vorlegen eines Olefins;
V2) Zugabe einer erfindungsgemäßen Verbindung und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

Die bevorzugte Abfolge des erfindungsgemäßen Verfahrens gemäß Verfahrensschritten V1) bis V4) hat sich als besonders effektiv und effizient erwiesen. Die Erfindung ist jedoch nicht auf die zuvor genannte Reihenfolge der Verfahrensschritte beschränkt.

Die vorliegende Erfindung betrifft zudem die Verwendung einer erfindungsgemäßen Verbindung (vorzugsweise wie vorstehend als bevorzugt bezeichnet) als Ligand eines Katalysators oder einer erfindungsgemäßen Substanz (vorzugsweise wie vorstehend als bevorzugt bezeichnet) als Katalysator in einer Hydroformylierungsreaktion, bevorzugt in einer Hydroformylierungsreaktion gemäß einem erfindungsgemäßen Verfahren (vorzugsweise wie vorstehend als bevorzugt bezeichnet).

Die erfindungsgemäßen Verbindungen werden bevorzugt als Liganden für Katalysatoren oder erfindungsgemäße Substanzen verwendet. Katalysatoren sind, wie oben definiert, bestimmte Metallkomplexe, die ein Metallatom und einen oder mehrere Liganden enthalten. Katalysatoren setzen die Aktivierungsenergie einer bestimmten Reaktion herab und erhöhen dadurch die Reaktionsgeschwindigkeit, ohne bei der Reaktion verbraucht zu werden. Sie gehen definitionsgemäß unverändert aus der Reaktion hervor.

Durch Verwendung der erfindungsgemäßen Verbindungen als Ligand eines Katalysators konnte in Hydroformylierungsreaktionen ein hoher Umsatz erreicht werden. Des Weiteren zeigen die mit diesen Liganden hergestellten Katalysatoren die Eigenschaft, interne Olefine (zum Beispiel 2-Penten) in einem nicht unerheblichen Teil zu terminalen Aldehyden zu hydroformylieren, sogenannte n-Selektivität.

Auch die erfindungsgemäßen Substanzen können als Katalysator in einer Hydroformylierungsreaktion verwendet werden. Der Katalysator ist ein Metallkomplex wie vorstehend definiert. Dieser Metallkomplex wird bevorzugt *in situ* durch Zusammengeben der erfindungsgemäßen Verbindung, d.h. des Liganden, und des Metallprecursors erzeugt.

### BEISPIELE

Die folgenden Beispiele dienen der weiteren Erläuterung und Veranschaulichung der vorliegenden Erfindung, ohne deren Anwendungsbereich einzuschränken.

### 1) Synthese von erfindungsgemäßen Verbindungen:

### Verbindung I-1:

### Bis(2-(dibenzo[d,f][1,3,2]dioxaphosphepin-6-yloxy)phenyl)methan

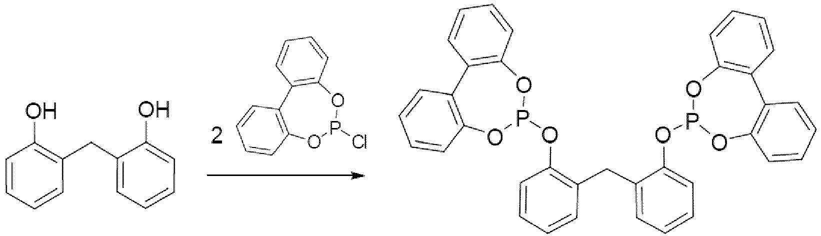

Das Bis(2-hydroxyphenyl)-methan (200 mg, 1,00 mmol) und Triethylamin (304 mg, 3,00 mmol) in THF (4 ml) wird bei Raumtemperatur mit 6-Chlorodibenzo[*d*,*f*][1,3,2]dioxaphosphepin (501 mg, 2,00 mmol) in THF (4 ml) tropfenweise versetzt. Nach dem Rühren über Nacht wird vom ausgefallenen Niederschlag abfiltriert und das Lösemittel unter Vakuum entfernt. Reinigungsversuche mittels Kristallisation scheiterten, so dass die Verbindung durch Säulenchromatographie (Eluent: Heptan/Dichlormethan 1/2) isoliert wurde.

Ausbeute: 330 mg (52%), farbloser, zäher Sirup.

³¹P-NMR (CD₂Cl₂): □ +144,1 ppm (Produkt).

¹H-NMR(CD₂Cl₂): □ 7,59-7,46 (4H, m, 4xHₐᵣ), 7,38-7,06 (20H, m, 20xHₐᵣ), 4,14 (2H, s, CH₂).

¹³C-NMR (CD₂Cl₂): □ 150,5 (d, *J* 7,6 Hz, 2xCₐᵣ), 149,4 (d, *J* 5,5 Hz, 4xCₐᵣ), 132,0 (d, *J* 3,0 Hz, 2xCₐᵣ), 131,6 (2xCₐᵣH), 131,5 (d, *J* 3,2 Hz, 4xCₐᵣ), 130,4 (4xCₐᵣH), 129,7 (4xCₐᵣH), 128,0 (2xCₐᵣH), 125,9 (4xCₐᵣH), 124,9 (2xCₐᵣH), 122,5 (4xCₐᵣH), 120,3 (d, *J* 12,2 Hz, 2xCₐᵣH), 31,0 (CH₂).

HRMS (ESI-TOF) [M+H]⁺: *m*/*z* berechnet: für C₃₇H₂₆O₆P₂ 629,1283, gefunden: 629,1295.

HRMS (ESI-TOF) [M+Na]⁺: *m*/*z* berechnet: für C₃₇H₂₆O₆P₂ 651,1097, gefunden: 651,1115.

### Verbindung I-2:

### Bis(2-((rac-4,8-di-tert-butyl-1,2,10,11-tetramethyldibenzo[d,f][1,3,2]dioxaphosphepin-6-yl)oxy)-phenyl)methane

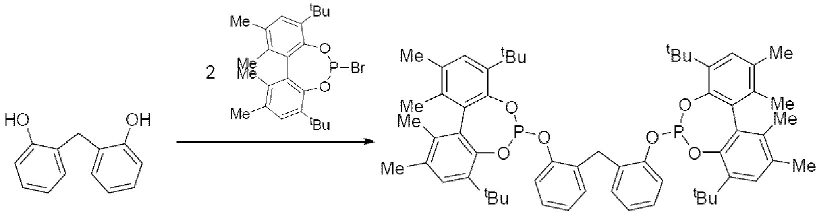

Zu einer Lösung des *rac*-6-Bromo-4,8-di-*tert*-butyl-1,2,10,11-tetramethyldibenzo[*d*,*f*][1,3,2]dioxa-phosphepins (835 mg, 1,80 mmol) in Toluol (7 ml) werden bei Raumtemperatur zunächst Triethylamin (273 mg, 2,70 mmol) und anschließend 2,2'-Methylendiphenol (180 mg, 0,90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 740 mg weißer Feststoff (85%).

Schmelzpunkt: 108-110°C.

³¹P-NMR (CD₂Cl₂): □ +135,8 ppm (52%) und +134,0 ppm (48%), Gemisch aus (*R,R*)/(*S,S*)- und (*R,S*)-Verbindung.

¹H-NMR (CD₂Cl₂): Diastereomerengemisch, □ 7,23 (2H, s, 2xHₐᵣ), 7,21 (2H, s, 2xHₐᵣ), 7,20 (2H, s, 2xHₐᵣ), 7,18 (2H, s, 2xHₐᵣ), 7,06-6,93 (8H, m, 8xHₐᵣ), 7,06-6,93 (8H, m, 8xHₐᵣ), 6,83-6,77 (8H, m, 8xHₐᵣ), 6,74 (2H, m, 2xHₐᵣ), 6,69 (2H, m, 2xHₐᵣ), 3,84 (1H, d, *J* 14,8 Hz, Hₐ-CH₂), 3,63 (2H, s,CH₂), 3,63 (1H, d, *J* 14,8 Hz, H_{b}-CH₂), 2,28 (12H, s, 4xCH₃), 2,28 (6H, s, 2xCH₃) 2,23 (6H, s, 2xCH₃) 2,23 (6H, s, 2xCH₃), 1,85 (6H, s, 2xCH₃), 1,84 (6H, s, 2xCH₃), 1,84 (6H, s, 2xCH₃) 1,81 (6H, s, 2xCH₃), 1,38 (18H, s, 2xC(CH₃)₃), 1,38 (18H, s, 2xC(CH₃)₃), 1,31 (18H, s, 2xC(CH₃)₃), 1,31 (18H, s, 2xC(CH₃)₃).

¹³C-NMR (CD₂Cl₂): Diastereomerengemisch, □ 150,4 (d, *J* 3,5 Hz, 2xCₐᵣ-), 150,4 (d, *J* 3,5 Hz, 2xCₐᵣ), 145,3 (d, *J* 6,1 Hz, 2xCₐᵣ), 145,2 (d, *J* 5,8 Hz, 2xCₐᵣ), 145,1 (d, *J* 2,1 Hz, 2xCₐᵣ), 145,1 (d, *J* 2,1 Hz, 2xCₐᵣ), 138,6 (d, *J* 2,9 Hz, 2xCₐᵣ), 138,6 (d, *J* 2,9 Hz, 2xCₐᵣ), 138,0 (2xCₐᵣ), 137,9 (2xCₐᵣ), 135,6 (4xCₐᵣ), 134,8 (2xCₐᵣ), 134,8 (2xCₐᵣ), 133,1 (2xCₐᵣ), 133,1 (2xCₐᵣ), 133,0 (2xCₐᵣ), 132,6 (d, *J* 2,0 Hz, 2xCₐᵣ), 132,5 (4xCₐᵣ), 132,4 (2xCₐᵣ), 132,1 (2xCₐᵣH) 132,0 (2xCₐᵣH), 131,1 (d, *J* 2,9 Hz, 2xCₐᵣ), 131,1 (d, *J* 3,1 Hz, 2xCₐᵣ), 128,7 (4xCₐᵣH) 128,2 (2xCₐᵣH), 128,2 (2xCₐᵣH), 127,2 (2xCₐᵣH), 127,1 (2xCₐᵣH), 124,2 (2xCₐᵣH), 124,2 (2xCₐᵣH), 120,4 (d, *J* 12,3 Hz, 2xCₐᵣH), 120,3 (d, *J* 13,4 Hz, 2xCₐᵣH) 35,2 (4x^{t}C), 34,9 (2x^{t}C), 34,9 (2x^{t}C), 31,8 (6xCH₃), 31,7 (6xCH₃), 31,4 (6xCH₃), 31,3 (6xCH₃), 29,5 (CH₂), 29,5 (CH₂), 20,5 (4xCH₃), 20,5 (4xCH₃), 16,9 (2xCH₃), 16,8 (2xCH₃), 16,6 (4xCH₃).

HRMS (ESI-TOF) [M+H]⁺: *m*/*z* berechnet: für C₆₁H₇₄O₆P₂ 965,5038, gefunden: 965,5027.

HRMS (ESI-TOF) [M+Na]⁺: *m*/*z* berechnet: für C₆₁H₇₄O₆P₂ 987,4852, gefunden: 987,4846.

### Verbindung I-3:

### Bis(2-((2,4,8,10-tetra-tert-butyldibenzo[d,f][1,3,2]dioxaphosphepin-6-yl)oxy)phenyl)methan

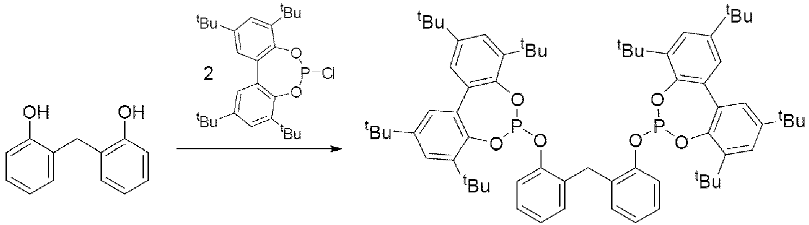

Zu einer auf 0°C abgekühlten Lösung von Bis(2-hydroxyphenyl)methan (100 mg, 0,50 mmol) und Triethylamin (152 mg, 1,50 mmol) in Toluol (4 ml) wird langsam eine Lösung des 2,4,8,10-Tetra-*tert*-butyl-6-chlorodibenzo[*d*,*f*][1,3,2]dioxaphosphepins (523 mg, 1,10 mmol) in Toluol (2 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird durch Säulenchromatographie (Eluent: Cyclohexan/AcOEt 100/0 bis 99/1) gereinigt.

Ausbeute: 130 mg farbloser Sirup (24%).

³¹P-NMR (CD₂Cl₂): □ +139,4 ppm.

¹H-NMR (CD₂Cl₂): □ 7,48 (4H, d, *J* 2,5 Hz, 4xHₐᵣ), 7,25 (4H, d, *J* 2,5 Hz, 4xHₐᵣ), 7,03 (2H, m, 2xHₐᵣ), 6,97 (2H, m, 2xHₐᵣ), 6,91 (2H, dd, *J* 7,7, 1,7 Hz, 2xHₐᵣ), 6,81 (2H, m, 2xHₐᵣ), 3,97 (2H, br s, CH₂), 1,38 (36H, s, 4xC(CH₃)₃), 1,38 (36H, s, 4xC(CH₃)₃).

¹³C-NMR (CD₂Cl₂): □ 150,4 (d, *J* 3,4 Hz, 2xCₐᵣ), 147,4 (4xCₐᵣ), 145,9 (d, *J* 6,0 Hz, 2xCₐᵣ), 140,8 (4xCₐᵣ), 133,2 (d, *J* 4,0 Hz, 2xCₐᵣ), 132,4 (d, *J* 2,4 Hz, 4xCₐᵣ), 131,9 (2xCₐᵣH), 127,3 (2xCₐᵣH), 127,0 (4xCₐᵣH), 124,9 (4xCₐᵣH), 124,4 (2xCₐᵣH), 120,7 (d, *J* 12,8 Hz, 2xCₐᵣH), 35,7 (4x^{t}C), 35,1 (4x^{t}C), 31,7 (12xCH₃), 31,3 (d, *J* 2,6 Hz, 12xCH₃), 30,8 (CH₂).

HRMS (ESI-TOF) [M+2O+H]⁺: m/z berechnet: für C₆₉H₉₀O₆P₂ 1109,6184, gefunden: 1109,6210.

HRMS (ESI-TOF) [M+20+Na]⁺: m/z berechnet: für C₆₉H₉₀O₆P₂ 1131,6003, gefunden: 1131,6033.

### Verbindung I-4:

### Bis(2-((4,8-di-tert-butyl-2,10-dimethoxydibenzo[d,f][1,3,2]dioxaphosphepin-6-yl)oxy)phenyl)-methan

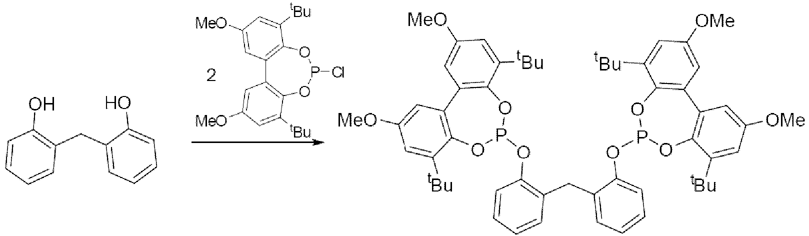

Das Bis(2-hydroxyphenyl)-methan (148 mg, 0,74 mmol) und Triethylamin (222 mg, 2,22 mmol) in THF (3 ml) wird bei Raumtemperatur mit 4,8-Di-*tert*-butyl-6-chloro-2,10-dimethoxydibenzo[*d,f*][1,3,2]di-oxa-phosphepin (626 mg, 1,48 mmol) in THF (6 ml) tropfenweise versetzt. Nach dem Rühren über Nacht wird vom ausgefallenen Niederschlag abfiltriert und das Lösemittel unter Vakuum entfernt. Reinigungsversuche mittels Kristallisation scheiterten, so dass das Bisphosphit durch Säulenchromatographie (Eluent: Dichlormethan) isoliert wurde.

Ausbeute: 290 mg (40%), weißer Feststoff.

³¹P-NMR (CD₂Cl₂): □ +140,2 ppm.

¹H-NMR (CD₂Cl₂): □ 7,12-7,05 (4H, m, 4xHₐᵣ), 7,01 (4H, d, *J* 3,1 Hz, 4xHₐᵣ), 6,92 (2H, m, 2xHₐᵣ), 6,88-6,81 (2H, m, 2xHₐᵣ), 6,79 (4H, d, *J* 3,1 Hz, 4xHₐᵣ), 3,98 (2H, s, CH₂), 3,83 (12H, s, 4xOCH₃), 1,36 (36H, s, 4xC(CH₃)₃).

¹³C-NMR (CD₂Cl₂): □ 156,4 (4xCₐᵣ), 150,4 (d, *J* 4,7 Hz, 2xCₐᵣ), 143,1 (d, *J* 1,3 Hz, 4xCₐᵣ), 141,8 (d, *J* 6,2 Hz, 4xCₐᵣ), 143,2 (d, *J* 1,2 Hz, 4xCₐᵣ), 132,4 (d, *J* 2,3 Hz, 2xCₐᵣ), 131,9 (2xCₐᵣH), 127,4 (2xCₐᵣH), 124,4 (2xCₐᵣH), 120,5 (d, *J* 13,3 Hz, 2xCₐᵣH), 114,8 (4xCₐᵣH), 113,3 (4xCₐᵣH), 35,7 (4x^{t}C), 31,1 (d, *J* 2,7 Hz, 12xCH₃), 30,6 (CH₂).

HRMS (ESI-TOF) [M+H]⁺: *m*/*z* berechnet: für C₅₇H₆₆O₁₀P₂ 973,4210, gefunden: 973,4222.

HRMS (ESI-TOF) [M+Na]⁺: *m*/*z* berechnet: für C₅₇H₆₆O₁₀P₂ 995,4023, gefunden: 959,4041.

### Verbindung I-5:

### 2-(2-((rac-4,8-Di-tert-butyl-1,2,10,11-tetramethyldibenzo[d,f][1,3,2]dioxaphosphepin-6-yl)oxy)benzyl)phenol

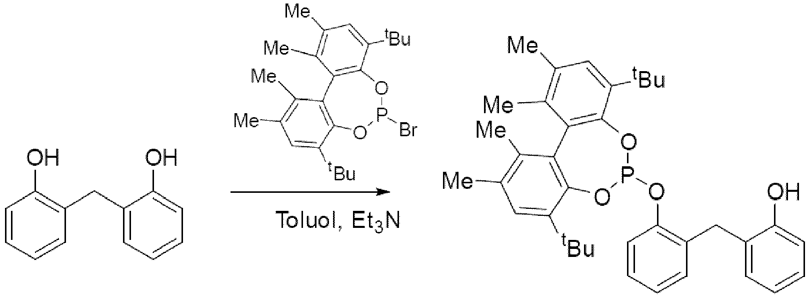

Zu einer auf 0°C abgekühlten Lösung von Bis(2-hydroxyphenyl)methan (400 mg, 2,00 mmol) und Triethylamin (607 mg, 6,00 mmol) in Toluol (5 ml) wird langsam eine Lösung des rac-6-Bromo-4,8-di-tert-butyl-1,2,10,11-tetramethyldibenzo[*d*,*f*][1,3,2]dioxa-phosphepins (618 mg, 1,33 mmol) in Toluol (3 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird durch Säulenchromatographie (Eluent: Cyclohexan/AcOEt 98/2) gereinigt.

Ausbeute: 620 mg weißer Feststoff (80%).

³¹P-NMR (CD₂Cl₂): □ +134,6 ppm.

¹H-NMR (CD₂Cl₂): □ 7,21 (2H, br d, *J* 1,4 Hz, 2xHₐᵣ), 7,17-6,93 (6H, m, 6xHₐᵣ), 6,76 (1H, dt, *J* 7,4, 1,3 Hz, Hₐᵣ), 6,76 (1H, dd, *J* 8,1, 1,2 Hz, Hₐᵣ), 4,92 (1H, s, OH), 3,60 (1H, d, *J* 16,0 Hz, Hₐ-CH₂), 3,44 (1H, d, *J* 16,0 Hz, H_{b}-CH₂), 2,28 (3H, s, CH₃), 2,11 (3H, s, CH₃), 1,84 (3H, s, CH₃), 1,78 (3H, s, CH₃), 1,42 (9H, s, C(CH₃)₃), 1,32 (9H, s, C(CH₃)₃).

¹³C-NMR (CD₂Cl₂): □ 154,3 (Cₐᵣ), 149,8 (Cₐᵣ), 144,9 (Cₐᵣ), 144,5 (Cₐᵣ), 138,7 (Cₐᵣ), 138,1 (Cₐᵣ), 135,7 (Cₐᵣ), 135,1 (Cₐᵣ), 133,8 (Cₐᵣ), 133,0 (Cₐᵣ), 132,4 (d, *J* 5,6 Hz, Cₐᵣ), 132,2 (d, *J* 1,6 Hz, Cₐᵣ), 131,5 (CₐᵣH), 130,9 (d, *J* 3,2 Hz, Cₐᵣ), 130,6 (CₐᵣH), 128,4 (CₐᵣH), 128,8 (CₐᵣH), 128,1 (CₐᵣH), 127,9 (CₐᵣH), 126,2 (Cₐᵣ), 124,7 (CₐᵣH), 120,7 (CₐᵣH), 120,7 (d, *J* 11,7 Hz, CₐᵣH), 116,2 (CₐᵣH), 35,2 (^{t}C), 34,9 (^{t}C), 31,8 (3xCH₃), 31,3 (d, *J* 5,1 Hz, 3xCH₃), 30,0 (CH₂), 20,5 (CH₃), 20,3 (CH₃), 16,7 (CH₃), 16,6 (CH₃).

HRMS (ESI-TOF) [M+H]⁺: *m*/*z* berechnet: für C₃₇H₄₃O₄P 583,2972, gefunden: 583,2983.

HRMS (ESI-TOF) [M+Na]⁺: *m*/*z* berechnet: für C₃₇H₄₃O₄P 605,2791, gefunden: 605,2803.

### Verbindung I-6:

### rac-4,8-Di-tert-butyl-1,2,10,11-tetramethyl-6-(2-(2-((2,4,8,10-tetra-tert-butyldibenzo[d,f][1,3,2]dioxaphosphepin-6-yl)oxy)benzyl)phenoxy)dibenzo[d,f][1,3,2]dioxaphosphepin

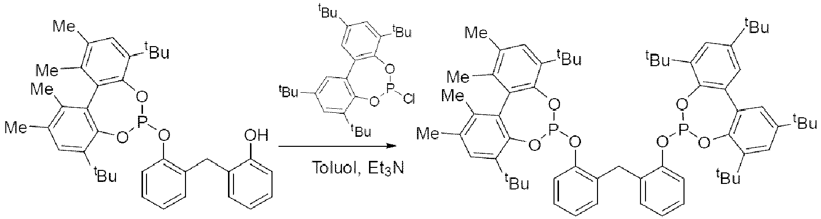

Eine Lösung von 2-(2-((*rac*-4,8-Di-*tert*-butyl-1,2,10,11-tetramethyldibenzo[*d*,*f*][1,3,2]dioxaphosphepin-6-yl)oxy)benzyl)phenol (300 mg, 0,51 mmol) und Triethylamin (78 mg, 0,77 mmol) in Toluol (4 ml) wird auf 0°C abgekühlt und langsam mit einer Lösung des 2,4,8,10-Tetra-*tert*-butyl-6-chlorodibenzo[*d*,*f*][1,3,2]dioxaphosphepins (267 mg, 0,56 mmol) in Toluol (2 ml) versetzt. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 410 mg weißer Feststoff (79%).

Schmelzpunkt: 114-116°C.

³¹P-NMR (CD₂Cl₂): □ +139,5 ppm und +134,7 ppm.

¹H-NMR (CD₂Cl₂): □ 7,47 (2H, dd, *J* 2,7, 2,7 Hz, 2xHₐᵣ), 7,23 (2H, d, *J* 2,5 Hz, 2xHₐᵣ), 7,21 (1H, s, Hₐᵣ), 7,18 (1H, s, Hₐᵣ), 7,06-6,92 (4H, m, 4xHₐᵣ), 6,87 (1H, dd, *J* 7,7, 1,8 Hz, Hₐᵣ), 6,82-6,74 (3H, m, 3xHₐᵣ), 3,88 (1H, d, *J* 14,9 Hz, Hₐ-CH₂), 3,77 (1H, d, *J* 14,9 Hz, H_{b}-CH₂), 2,27 (3H, s, CH₃), 2,24 (3H, s, CH₃), 1,84 (6H, s, 2xCH₃), 1,37 (18H, s, 2xC(CH₃)₃), 1,36 (18H, s, 2xC(CH₃)₃), 1,36 (9H, s, C(CH₃)₃), 1,30 (9H, s, C(CH₃)₃).

¹³C-NMR (CD₂Cl₂): □ 150,4-133,4 (18xCₐᵣ), 132,0 (CₐᵣH), 131,8 (CₐᵣH), 128,7 (CₐᵣH), 128,2 (CₐᵣH), 127,2 (2xCₐᵣH), 127,0 (2xCₐᵣH), 124,9 (CₐᵣH), 124,9 (CₐᵣH), 124,3 (CₐᵣH), 124,2 (CₐᵣH), 120,6 (d, *J* 11,6 Hz, CₐᵣH), 120,4 (d, *J* 11,7 Hz, CₐᵣH), 35,7 (2x^{t}C), 35,1 (^{t}C), 35,0 (2x^{t}C), 34,9 (^{t}C), 31,7 (3xCH₃), 31,6 (6xCH₃), 31,4 (3xCH₃), 31,3 (d, *J* 2,7 Hz, 6xCH₃), 31,2 (3xCH₃), 30,3 (CH₂), 20,5 (2xCH₃), 16,7 (CH₃), 16,6 (CH₃).

HRMS (ESI-TOF) [M+H]⁺: *m*/*z* berechnet: für C₆₅H₈₂O₆P₂ 1021,5660, gefunden: 1021,5681.

HRMS (ESI-TOF) [M+Na]⁺: *m*/*z* berechnet: für C₆₅H₈₂O₆P₂ 1043,5479, gefunden: 1043,5499.

### Verbindung I-7:

### 2-(tert-Butyl)-6-(3-(tert-butyl)-5-ethyl-2-((2,4,8,10-tetra-tert-butyldibenzo[d,f][1,3,2]dioxaphosphepin-6-yl)oxy)benzyl)-4-ethylphenol

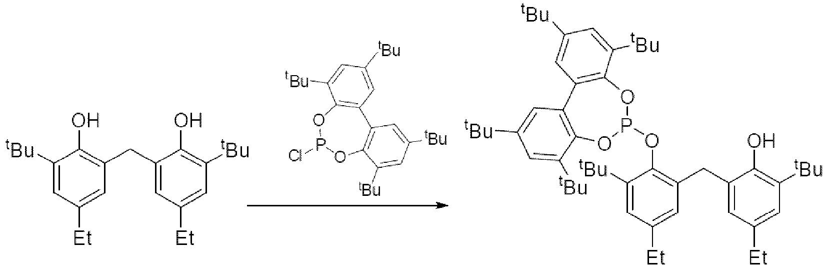

Das 2,4,8,10-Tetra-*tert*-butyl-6-chlorodibenzo[*d*,*f*[1,3,2]dioxaphosphepin (238 mg, 0,5 mmol) in Toluol (2 ml) wird bei 0°C langsam zu einer Lösung von 6,6'-Methylenbis(2-(*tert*-butyl)-4-ethylphenol) (240 mg, 0,65 mmol) und Triethylamin (151 mg, 1,50 mmol) in Toluol (4 ml) getropft. Nach 30 Minuten lässt man auf Raumtemperatur erwärmen und rührt über Nacht weiter. Anschließend wird vom ausgefallenen Niederschlag abfiltriert und das Filtrat unter Vakuum eingeengt. Die Reinigung des verbliebenen Rückstandes erfolgt durch Säulenchromatographie (Eluent: Cyclohexan/AcOEt 99/1).

Ausbeute: 320 mg weißer Feststoff (79%).

Schmelzpunkt = 96-98°C.

³¹P-NMR (CD₂Cl₂): □ +141,3 ppm (24%, sehr breites Singulett) und +140,8 ppm (breites Singulett), zwei Konformationsisomere.

¹H- und ¹³C-NMR: Durch die hohe Flexibilität im Wechsel der Konformationen sind die meisten Signale stark verbreitert und lassen sich nicht identifizieren bzw. zuordnen.

HRMS (ESI-TOF) [M+H]⁺: *m*/*z* berechnet: für C₅₃H₇₅O₄P 807,5481, gefunden: 807,5488.

HRMS (ESI-TOF) [M+Na]⁺: *m*/*z* berechnet: für C₅₃H₇₅O₄P 829,5295, gefunden: 829,5308.

### Verbindung I-8:

### 2,4,8,10-Tetra-tert-butyl-6-(2-(tert-butyl)-6-(3-(tert-butyl)-2-((5-(tert-butyl)benzo[d][1,3,2]dioxaphosphol-2-yl)oxy)-5-ethylbenzyl)-4-ethylphenoxy)dibenzo[d,f][1,3,2]dioxaphosphepin

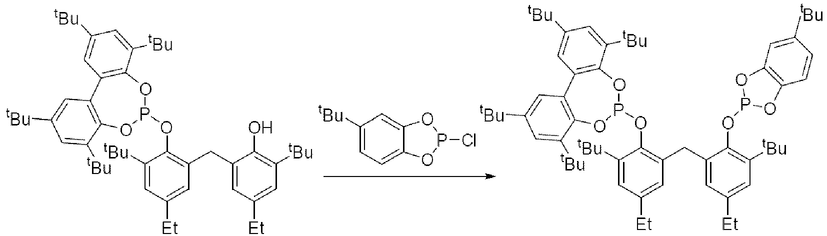

Zu einer Lösung des 2-(*tert*-Butyl)-6-(3-(*tert*-butyl)-5-ethyl-2-((2,4,8,10-tetra-*tert*-butyldibenzo[*d*,*f*][1,3,2]dioxaphosphepin-6-yl)oxy)benzyl)-4-ethylphenols (185 mg, 0,23 mmol) in Toluol (3 ml) gibt man bei 0°C zunächst Triethylamin (35 mg, 0,35 mmol) und anschließend eine Lösung des 5-(*tert*-Butyl)-2-chloro-benzo[*d*][1,3,2]dioxaphosphols (58 mg, 0,25 mmol) in Toluol (2 ml) hinzu. Nach 30 Minuten lässt man auf Raumtemperatur erwärmen und rührt über Nacht weiter. Die Reaktionslösung wird filtriert und das Filtrat unter Vakuum eingeengt. Der verbleibende Rückstand wird in wenig Hexan aufgenommen und im Kühlschrank aufbewahrt. Der ausgefallene Niederschlag (Reste Triethylammoniumchlorid und Hydrolyseverbindung der P-CI-Verbindung) wird abgetrennt, der Rückstand aus dem Filtrat wird getrocknet und das Bisphosphit ohne weitere Reinigung verwendet.

Ausbeute: 120 mg weißer Feststoff (52%).

Schmelzpunkt: 145-149°C.

³¹P-NMR (CD₂Cl₂): □ +143,1 ppm (32%, br d, *J* 19 Hz) und +141,2 ppm (34%, br d, *J* 19 Hz), Diastereomer A; +140,5 ppm (15%, br s) und 14% +139,4 ppm (br s), Diastereomer B.

¹H- und ¹³C-NMR: Durch die hohe Flexibilität im Wechsel der Konformationen sind die meisten Signale stark verbreitert und lassen sich nicht identifizieren bzw. zuordnen.

HRMS (ESI-TOF) [M+H]⁺: *m*/*z* berechnet: für C₆₃H₈₆O₆P₂ 1001,5978, gefunden: 1001,5948.

HRMS (ESI-TOF) [M+Na]⁺: *m*/*z* berechnet: für C₆₃H₈₆O₆P₂ 1023,5792, gefunden: 1023,5769.

### Verbindung I-9:

### Bis(3-(tert-butyl)-2-((5-(tert-butyl)benzo[d][1,3,2]dioxaphosphol-2-yl)oxy)-5-ethylphenyl)methan

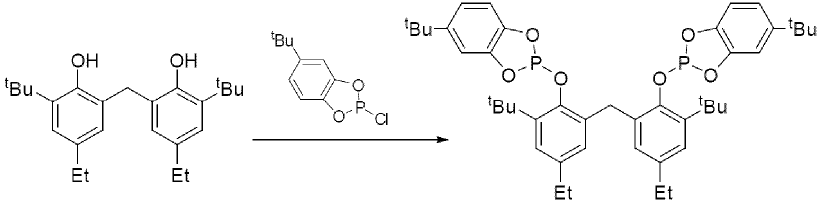

Zu einer Lösung von 6,6'-Methylenbis(2-(*tert*-butyl)-4-ethylphenol) (184 mg, 0,50 mmol) in Toluol (5 ml) gibt man bei Raumtemperatur zunächst Triethylamin (152 mg, 1,50 mmol) und anschließend eine Lösung des 5-(*tert*-Butyl)-2-chlorobenzo[*d*][1,3,2]dioxaphosphols (277 mg, 1,20 mmol) in Toluol (2 ml) langsam hinzu. Man lässt über Nacht weiterrühren und filtriert dann die Reaktionslösung. Das Filtrat wird unter Vakuum eingeengt und der verbleibende Rückstand wird in wenig Acetonitril aufgenommen und im Kühlschrank zur Kristallisation gebracht. Nach der Filtration und dem Trocknen unter Vakuum wird das Bisphosphit in guter Reinheit erhalten.

Ausbeute: 300 mg weißer Feststoff (79%).

Schmelzpunkt: 85-88°C.

³¹P-NMR (CD₂Cl₂): □ +140,4 ppm und +140,3 ppm (2x Diastereomere).

¹H-NMR (CD₂Cl₂): Diastereomerengemisch, □ 7,18 (2H, m, 2xHₐᵣ), 7,16-7,13 (6H, m, 6xHₐᵣ), 7,04-6,93 (8H, m, 8xHₐᵣ), 6,66 (4H, m, 4xHₐᵣ), 4,36 (1H, d, *J* 16,7 Hz, Hₐ-CH₂), 4,31 (2H, s, CH₂), 4,29 (1H, d, *J* 16,7 Hz, H_{b}-CH₂), 2,50 (8H, q, *J* 7,6 Hz, 4xCH₂), 1,47 (18H, s, 2xC(CH₃)₃), 1,46 (18H, s, 2xC(CH₃)₃), 1,29 (18H, s, 2xC(CH₃)₃), 1,28 (18H, s, 2xC(CH₃)₃), 1,09 (6H, t, *J* 7,6 Hz, 2xCH₃), 1,08 (6H, t, *J* 7,6 Hz, 2xCH₃).

¹³C-NMR (CD₂Cl₂): Diastereomerengemisch, □ 147,5 (d, *J* 6,3 Hz, 4xCₐᵣ), 147,2 (4xCₐᵣ), 145,1 (d, *J* 7,7 Hz, 4xCₐᵣ), 142,7 (d, *J* 7,9 Hz, 4xCₐᵣ), 142,0 (m, 4xCₐᵣ), 140,4 (4xCₐᵣ), 128,9 (4xCₐᵣH), 126,1 (4xCₐᵣH), 120,0 (4xCₐᵣH), 112,1 (d, *J* 6,8 *Hz,* 4xCₐᵣH), 110,5 (4xCₐᵣH), 35,2 (CH₂), 35,2 (CH₂), 35,1 (4x^{t}C), 35,0 (4x^{t}C), 31,8 (12xCH₃), 31,2 (6xCH₃), 31,0 (6xCH₃), 28,9 (4xCH₂), 15,8 (4xCH₃).

HRMS (ESI-TOF) [M+H]⁺: *m*/*z* berechnet: für C₄₅H₅₈O₆P₂ 757,3787, gefunden: 757,3772.

HRMS (ESI-TOF) [M+Na]⁺: *m*/*z* berechnet: für C₄₅H₅₈O₆P₂ 779,3600, gefunden: 779,3586.

### Verbindung I-10:

### Bis(3-(tert-butyl)-2-((4,6-di-tert-butylbenzo[d][1,3,2]dioxaphosphol-2-yl)oxy)-5-ethylphenyl)-methan

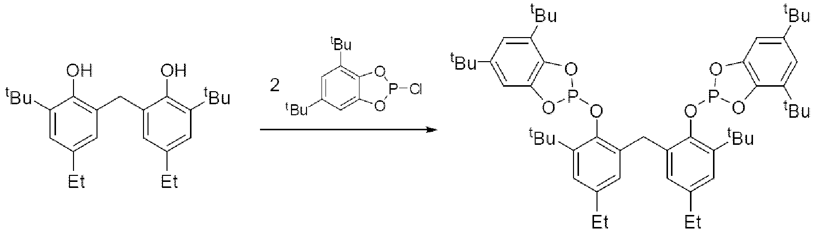

Zu einer Lösung von 6,6'-Methylenbis(2-(*tert*-butyl)-4-ethylphenol) (159 mg, 0,43 mmol) und Triethylamin (131 mg, 1,29 mmol) in THF (3 ml) tropft man bei 0°C eine Lösung von 4,6-Di-tert-butyl-2-chloro-benzo[*d*][1,3,2]dioxaphosphol (247 mg, 0,86 mmol) in THF (4 ml). Man lässt über Nacht bei Raumtemperatur weiterrühren und filtriert anschließend vom ausgefallenen Ammoniumsalz ab. Nach dem Einengen des Filtrats unter Vakuum und dem Trocknen bei 60°C wurde das Bisphosphit bereits in hoher Reinheit (³¹P-NMR: 98%) erhalten.

Ausbeute: 340 mg weißer Feststoff (91%).

³¹P-NMR (CD₂Cl₂): □ +139,0 ppm und +138,9 ppm (2x Diastereomere).

¹H-NMR (CD₂Cl₂): Diastereomerengemisch, □ 7,16 (4H, m, 4xHₐᵣ), 7,03 (2H, d, *J* 1,9 Hz, 2xHₐᵣ), 7,02 (2H, d, *J* 2,0 Hz, 2xHₐᵣ), 6,99 (2H, d, *J* 2,0 Hz, 2xHₐᵣ), 6,97 (2H, d, *J* 2,0 Hz, 2xHₐᵣ), 6,70 (4H, m, 4xHₐᵣ), 4,46 (2H, br s, CH₂), 4,42 (2H, br s, CH₂), 2,58-2,46 (8H, m, 4xCH₂), 1,51 (36H, s, 4xC(CH₃)₃), 1,45 (18H, s, 2xC(CH₃)₃), 1,43 (18H, s, 2xC(CH₃)₃), 1,28 (18H, s, 2xC(CH₃)₃), 1,26 (18H, s, 2xC(CH₃)₃), 1,11 (6H, t, *J* 7,6 Hz, 2xCH₃), 1,09 (6H, t, *J* 7,6 Hz, 2xCH₃).

¹³C-NMR (CD₂Cl₂): Diastereomerengemisch, □ 147,8 (d, *J* 4,4 Hz, 2xCₐᵣ), 147,7 (d, *J* 4,0 Hz, 2xCₐᵣ), 146,3 (2xCₐᵣ), 146,2 (2xCₐᵣ), 145,2 (2xCₐᵣ), 145,1 (2xCₐᵣ), 142,0 (d, *J* 2,9 Hz, 2xCₐᵣ), 142,0 (d, *J* 2,9 Hz, 2xCₐᵣ), 140,8 (2xCₐᵣ), 140,7 (2xCₐᵣ), 140,4 (4xCₐᵣ), 135,3 (4xCₐᵣ), 133,2 (d, *J* 4,3 Hz, 2xCₐᵣ), 133,1 (d, *J* 4,0 Hz, 2xCₐᵣ), 129,0 (4xCₐᵣH), 126,0 (4xCₐᵣH), 117,4 (2xCₐᵣH), 117,4 (2xCₐᵣH), 108,5 (2xCₐᵣH), 108,4 (2xCₐᵣH), 35,8 (m, CH₂), 35,5 (m, CH₂), 35,2 (4x^{t}C), 35,2 (4x^{t}C), 34,9 (2x^{t}C), 34,8 (2x^{t}C), 31,9 (12xCH₃), 31,1 (6xCH₃), 31,1 (6xCH₃), 30,1 (6xCH₃), 30,1 (6xCH₃), 28,9 (4xCH₂), 15,8 (2xCH₃), 15,8 (2xCH₃).

HRMS (ESI-TOF) [M+H]⁺: *m*/*z* berechnet: für C₅₃H₇₄O₆P₂ 869,5038, gefunden: 869,5054.

HRMS (ESI-TOF) [M+Na]⁺: *m*/*z* berechnet: für C₅₃H₇₄O₆P₂ 891,4852, gefunden: 891,4865.

| | | | | |
|---|---|---|---|---|
| Elementaranalyse: | berechnet: | C 73,24% | H 8,58% | P 7,13% |
| | gefunden: | C 72,92% | H 8,19% | P 7,05%. |

### Verbindung I-11:

### Bis(3-(tert-butyl)-2-(dibenzo[d,f][1,3,2]dioxaphosphepin-6-yloxy)-5-ethylphenyl)methan

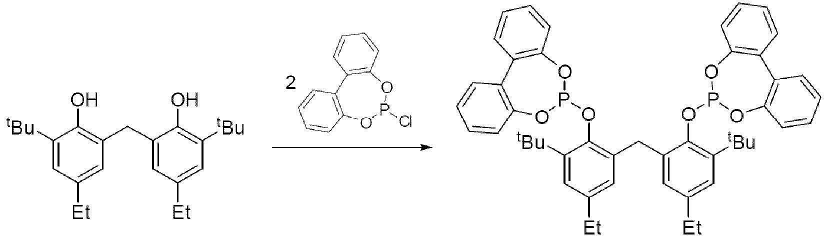

Zu einer Lösung von 6,6'-Methylenbis(2-(*tert*-butyl)-4-ethylphenol) (322 mg, 0,87 mmol) und Triethylamin (265 mg, 2,62 mmol) in Tetrahydrofuran (3 ml) wird bei 0°C langsam eine Lösung 6-Chlordi-benzo[*d*,*f*][1,3,2]dioxaphosphepins (503 mg, 2,00 mmol) in Tetrahydrofuran (6 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Die Reinigung erfolgte durch Erhitzen des Rohproduktes in Heptan (10 ml) und anschließendem Rühren über mehrere Stunden. Die unlöslichen Bestandteile enthalten neben dem Produkt noch Reste des Hydrochlorids. Durch Aufnehmen mit Toluol wird letzteres nach der Filtration entfernt und das Filtrat erneut unter Vakuum eingeengt. Das verbleibende Produkt wird abschließend unter Vakuum getrocknet.

Ausbeute: 250 mg weißer Feststoff (36%).

³¹P-NMR (CD₂Cl₂): □ +146,8 ppm.

¹H-NMR (CD₂Cl₂): □ 7,51-7,44 (4H, m, 4xHₐᵣ), 7,30-7,19 (10H, m, 10xHₐᵣ), 6,98-6,93 (4H, m, 4xHₐᵣ), 6,76 (2H, d, *J* 2,0 Hz, 2xHₐᵣ), 4,65 (2H, s, CH₂), 2,57 (4H, q, *J* 7,6 Hz, 2xCH₂), 1,55 (18H, s, 2xC(CH₃)₃), 1,16 (6H, t, *J* 7,6 Hz, 2xCH₃).

¹³C-NMR (CD₂Cl₂): □ 149,5 (d, *J* 6,0 Hz, 4xCₐᵣ), 148,3 (d, *J* 8,7 Hz, 2xCₐᵣ), 142,3 (d, *J* 3,0 Hz, 2xCₐᵣ), 140,8 (2xCₐᵣ), 133,6 (d, *J* 3,6 Hz, 2xCₐᵣ), 131,4 (d, *J* 3,1 Hz, 4xCₐᵣ), 130,1 (4xCₐᵣH), 129,5 (4xCₐᵣH), 129,1 (2xCₐᵣH), 126,1 (2xCₐᵣH), 125,6 (4xCₐᵣH), 122,7 (4xCₐᵣH), 35,8 (m, CH₂), 35,4 (2x^{t}C), 31,2 (d, *J* 2,4 Hz, 6xCH₃), 28,9 (2xCH₂), 16,1 (2xCH₃).

HRMS (ESI-TOF) [M+H]⁺: *m*/*z* berechnet: für C₄₉H₅₀O₆P₂ 797,3156, gefunden: 797,3152.

HRMS (ESI-TOF) [M+Na]⁺: *m*/*z* berechnet: für C₄₉H₅₀O₆P₂ 819,2975, gefunden: 819,2969.

### Verbindung I-12:

### 2-(tert-Butyl)-6-(3-(tert-butyl)-2-((rac-4,8-di-tert-butyl-1,2,10,11-tetramethyldibenzo[d,f][1,3,2]dioxaphosphepin-6-yl)oxy)-5-ethylbenzyl)-4-ethylphenol

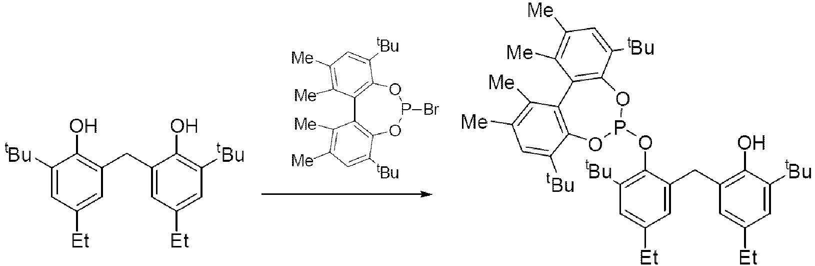

Zu einer Lösung von 6,6'-Methylenbis(2-(*tert*-butyl)-4-ethylphenol) (862 mg, 2,34 mmol) und Triethylamin (546 mg, 5,40 mmol) in Toluol (4 ml) wird bei 0°C langsam eine Lösung des rac-6-Bromo-4,8-di-tert-butyl-1,2,10,11-tetramethyldibenzo[*d*,*f*][1,3,2]dioxaphosphepins (834 mg, 1,80 mmol) in Toluol (4 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Nach der Reinigung durch Säulenchromatographie (Eluent: Cyclohexan/AcOEt 99/1) wird ein weißes, festes Monophosphit erhalten.

Ausbeute: 1,10 g weißer Feststoff (81%).

³¹P-NMR (CD₂Cl₂): □ +134,5 ppm (10%, br s) und +133,9 ppm (90%, s), zwei Isomere.

¹H-NMR und¹³C-NMR (CD₂Cl₂): sehr breite Signale neben scharfen Signale in den Spektren; Zuordnung stark erschwert.

HRMS (ESI-TOF) [M+H]⁺: *m*/*z* berechnet: für C₄₉H₆₇O₄P 751,4855, gefunden: 751,4852.

HRMS (ESI-TOF) [M+Na]⁺: *m*/*z* berechnet: für C₄₉H₆₇O₄P 773,4669, gefunden: 773,4677.

### Verbindung I-13:

### 4,8-Di-tert-butyl-6-(2-(tert-butyl)-6-(3-(tert-butyl)-2-((5-(tert-butyl)benzo[d][1,3,2]dioxaphosphol-2-yl)oxy)-5-ethylbenzyl)-4-ethylphenoxy)-1,2,10,11-tetramethyldibenzo[d,f][1,3,2]dioxaphosphepin

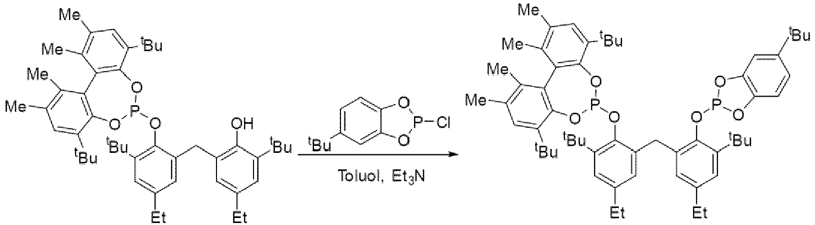

Zu einer auf 0°C abgekühlten Lösung von 2-(*tert*-*Butyl*)-6-(3-(*tert*-butyl)-2-((*rac*-4,8-di-*tert*-butyl-1,2,10,11-tetramethyldibenzo[*d*,*f*][1,3,2]dioxaphosphepin-6-yl)oxy)-5-ethylbenzyl)-4-ethylphenol (400 mg, 0,53 mmol) und Triethylamin (81 mg, 0,80 mmol) in Toluol (5 ml) wird langsam eine Lösung des 5-(*tert-*Butyl)-2-chlorbenzo[*d*][1,3,2]dioxaphosphols (134 mg, 0,58 mmol) in Toluol (2 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird aus Acetonitril umkristallisiert und das Bisphosphit als weißer Feststoff erhalten.

Ausbeute: 350 mg weißer Feststoff (70%).

Schmelzpunkt: 147-149°C.

³¹P-NMR (CD₂Cl₂): □ +141,2, 140,9, 140,6 ppm (50%, drei breite Signale, 1,3,2-Dioxophospholan-Gruppe) und +135,9, 134,7, 134,5 ppm (50%, drei breite Signale, 1,3,2-Dioxaphophepin-Gruppe).

¹H-NMR und¹³C-NMR (CD₂Cl₂): sehr breite Signale; Zuordnung stark erschwert.

HRMS (ESI-TOF) [M+H]⁺: *m*/*z* berechnet: für C₅₉H₇₈O₆P₂ 945,5352, gefunden: 945,5346.

HRMS (ESI-TOF) [M+Na]⁺: *m*/*z* berechnet: für C₄₉H₆₇O₄P 967,5165, gefunden: 967,5170.

### Verbindung I-14:

### 4,8-Di-tert-butyl-6-chloro-2,10-diethyl-12H-dibenzo[d,g][1,3,2]dioxaphosphocin

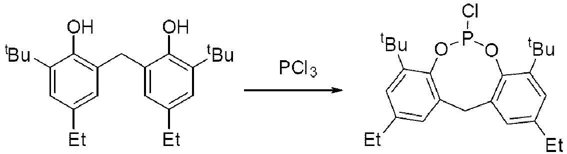

Zu einer Lösung des 6,6'-Methylenbis(2-(*tert*-butyl)-4-ethylphenols) (2,00 g, 5,43 mmol) in THF (40 ml) wird zunächst bei -40°C Phosphortrichlorid (1,19 g, 8,68 mmol) und anschließend Triethylamin (1,65 g, 16,28 mmol) tropfenweise hinzugegeben. Nach dem Rühren über Nacht bei Raumtemperatur wird die Reaktionslösung filtriert und das Filtrat unter Vakuum eingeengt. Der erhaltene Feststoff wird bei 60°C getrocknet und ohne weitere Reinigung verwendet.

Ausbeute: 2,15 g weißer Feststoff (92%).

³¹P-NMR (CD₂Cl₂): □ +155,1 ppm (breites Singulett).

¹H-NMR (CD₂Cl₂): □ 7,18 (2H, d, *J* 2,1 Hz, 2xHₐᵣ), 7,13 (2H, d, *J* 2,1 Hz, 2xHₐᵣ), 4,04 (1H, dd, *J* 13,1, 2,3 Hz, Hₐ-CH₂), 3,77 (1H, dd, *J* 13,1 Hz, H_{b}-CH₂), 2,64 (4H, q, *J* 7,6 Hz, 2xCH₂), 1,43 (18H, s, 2xC(CH₃)₃), 1,26 (6H, t, *J* 7,6 Hz, 2xCH₃).

¹³C-NMR (CD₂Cl₂): 147,8 (2xCₐᵣ), 142,2 (d, *J* 3,9 Hz, 2xCₐᵣ), 141,7 (d, *J* 1,5 Hz, 2xCₐᵣ), 135,7 (d, *J* 3,8 Hz, 2xCₐᵣ), 128,1 (2xCₐᵣH), 126,4 (2xCₐᵣH), 35,4 (CH₂), 35,3 (2x^{t}C), 31,0 (d, *J* 3,3 Hz, 6xCH₃), 28,9 (2xCH₂), 15,9 (2xCH₃).

### Verbindung I-15:

### 2-(tert-Butyl)-6-(3-(tert-butyl)-2-((4,8-di-tert-butyl-2,10-diethyl-12H-dibenzo[d,g][1,3,2]dioxa-phosphocin-6-yl)oxy)-5-ethylbenzyl)-4-ethylphenol

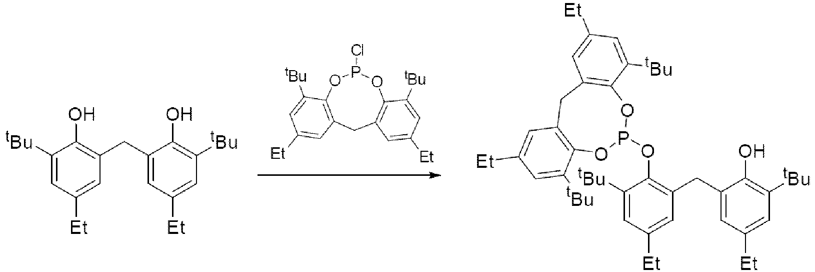

Zu einer Lösung von 6,6'-Methylenbis(2-(*tert*-butyl)-4-ethylphenol) (296 mg, 0,80 mmol) in THF (3 ml) wird bei 0°C zunächst Triethylamin (122 mg, 1,20 mmol) und dann langsam das 4,8-Di-*tert*-butyl-6-chloro-2,10-diethyl-12*H*-dibenzo[*d*,*g*][1,3,2]dioxaphosphocin (348 mg, 0,80 mmol) in THF (5 ml) gegeben. Es wird über Nacht bei Raumtemperatur weitergerührt und anschließend vom ausgefallenen Niederschlag abfiltriert. Das Filtrat wird eingeengt und der verbleibende Rückstand nach dem Trocknen ohne weitere Reinigung (97% Reinheit im ³¹P-NMR) verwendet.

Ausbeute: 560 mg weißer Feststoff (91%).

³¹P-NMR (CD₂Cl₂): □ +137,5 ppm.

¹H-NMR (CD₂Cl₂): □ 7,24 (2H, d, *J* 2,2 Hz, 2xHₐᵣ), 7,18 (1H, d, *J* 2,2 Hz, Hₐᵣ), 7,13 (2H, d, *J* 2,2 Hz, 2xHₐᵣ), 7,01 (1H, d, *J* 2,2 Hz, Hₐᵣ), 7,00 (1H, d, *J* 2,0 Hz, Hₐᵣ), 6,98 (1H, d, *J* 2,2 Hz, Hₐᵣ), 6,24 (1H, br s, OH), 4,52 (1H, dd, *J* 12,8, 3,0 Hz, Hₐ-CH₂), 4,38 (2H, s, CH₂), 3,53 (1H, d, *J* 12,8 Hz, H_{b}-CH₂), 2,70-2,55 (8H, m, 4xCH₂), 1,58 (9H, s, C(CH₃)₃), 1,39 (9H, s, C(CH₃)₃), 1,29 (18H, s, 2xC(CH₃)₃), 1,27 (6H, t, *J* 7,6 Hz, 2xCH₃), 1,25 (3H, t, *J* 7,6 Hz, CH₃), 1,24 (3H, t, *J* 7,6 Hz, CH₃).

¹³C-NMR (CD₂Cl₂): □ 151,6 (Cₐᵣ), 146,0 (d, *J* 8,1 Hz, Cₐᵣ), 145,1 (d, *J* 7,1 Hz, 2xCₐᵣ), 142,8 (d, *J* 3,7 Hz, 2xCₐᵣ), 142,2 (d, *J* 3,0 Hz, Cₐᵣ), 141,3 (d, *J* 1,3 Hz, 2xCₐᵣ), 141,1 (Cₐᵣ), 137,2 (d, *J* 3,8 Hz, 2xCₐᵣ), 136,3 (Cₐᵣ), 135,4 (Cₐᵣ), 133,7 (Cₐᵣ), 128,2 (CₐᵣH), 127,9 (CₐᵣH), 127,9 (2xCₐᵣH), 127,4 (Cₐᵣ), 126,5 (CₐᵣH), 126,2 (2xCₐᵣH), 125,4 (CₐᵣH), 35,7 (^{t}C), 35,3 (2xCH₂), 35,0 (^{t}C), 35,0 (2x^{t}C), 34,3 (CH₂), 34,2 (CH₂), 31,6 (d, *J* 4,4 Hz, 3xCH₃), 31,1 (d, *J* 3,8 Hz, 6xCH₃), 29,8 (3xCH₃), 29,1 (CH₂), 28,9 (2xCH₂), 28,8 (CH₂), 16,3 (CH₃), 16,2 (CH₃), 16,0 (2xCH₃).

### Verbindung I-16:

### 4,8-Di-tert-butyl-6-(2-(tert-butyl)-6-(3-(tert-butyl)-2-((5-(tert-butyl)benzo[d][1,3,2]dioxaphosphol-2-yl)oxy)-5-ethylbenzyl)-4-ethylphenoxy)-2,10-diethyl-12H-dibenzo[d,g][1,3,2]dioxaphosphocin

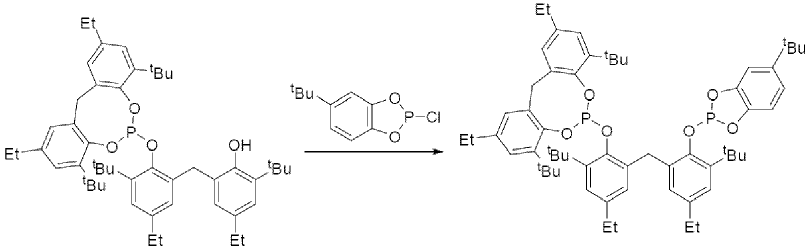

Zu einer Lösung von 2-(*tert*-Butyl)-6-(3-(*tert*-butyl)-2-((4,8-di-*tert*-butyl-2,10-diethyl-12*H-*dibenzo[*d*,*g*][1,3,2]dioxa-phosphocin-6-yl)oxy)-5-ethylbenzyl)-4-ethylphenol (560 mg, 0,73 mmol) in THF (5 ml) wird bei 0°C zunächst Triethylamin (111 mg, 1,10 mmol) und dann langsam das 5-(*tert*-Butyl)-2-chlorobenzo[*d*][1,3,2]dioxaphosphol (169 mg, 0,73 mmol) in THF (3 ml) gegeben. Es wird über Nacht bei Raumtemperatur weitergerührt und anschließend vom ausgefallenen Niederschlag abfiltriert. Das Filtrat wird eingeengt und der verbleibende Rückstand nach dem Trocknen aus Acetonitril umkristallisiert.

Ausbeute: 450 mg weißer Feststoff (64%).

³¹P-NMR (CD₂Cl₂): □ +141,9 ppm (d, *J* 5,2 Hz) und +137,0 ppm (d, *J* 5,2 Hz).

¹H-NMR (CD₂Cl₂): □ 7,20 (1H, d, *J* 2,2 Hz, Hₐᵣ), 7,16-7,10 (3H, m, 3xHₐᵣ), 7,09-7,05 (3H, m, 3xHₐᵣ), 6,99 (1H, dd, *J* 8,4, 2,0 Hz, Hₐᵣ), 6,91 (1H, d, *J* 8,4 Hz, Hₐᵣ), 6,81 (1H, d, *J* 2,1 Hz, Hₐᵣ), 6,68 (1H, d, *J* 2,0 Hz, Hₐᵣ), 4,63 (1H, dd, *J* 16,5, 3,3 Hz, Hₐ-CH₂), 4,51 (1H, dd, *J* 16,5, 3,1 Hz, Hₐ-CH₂), 4,41 (1H, dd, *J* 12,8, 2,9 Hz, Hₐ-CH₂), 3,30 (1H, d, *J* 12,8 Hz, H_{b}-CH₂), 2,62-2,52 (6H, m, 3xCH₂), 2,51 (2H, q, *J* 7,6 Hz, CH₂), 1,64 (9H, s, C(CH₃)₃), 1,40 (9H, s, C(CH₃)₃), 1,30 (9H, s, C(CH₃)₃), 1,26-1,15 (12H, 4xCH₃), 1,20 (18H, s, 2xC(CH₃)₃).

¹³C-NMR (CD₂Cl₂): □ 148,2 (d, *J* 7,3 Hz, Cₐᵣ), 147,0 (Cₐᵣ), 148,2 (d, *J* 6,9 Hz, Cₐᵣ), 145,6 (d, *J* 7,5 Hz, 2xCₐᵣ), 145,1 (d, *J* 7,5 Hz, Cₐᵣ), 142,9 (d, *J* 7,5 Hz, Cₐᵣ), 142,7 (m, 2xCₐᵣ), 142,2 (d, *J* 2,2 Hz, Cₐᵣ), 141,6 (d, *J* 2,8 Hz, Cₐᵣ), 140,8 (2xCₐᵣ), 140,3 (Cₐᵣ), 140,3 (Cₐᵣ), 137,2 (d, *J* 4,0 Hz, Cₐᵣ), 137,1 (d, *J* 3,7 Hz, Cₐᵣ), 133,4 (d, *J* 3,3 Hz, Cₐᵣ), 133,0 (Cₐᵣ), 129,1 (CₐᵣH), 128,0 (CₐᵣH), 127,8 (CₐᵣH), 127,6 (CₐᵣH), 126,6 (CₐᵣH), 125,9 (2xCₐᵣH), 125,6 (CₐᵣH), 119,9 (CₐᵣH), 112,0 (CₐᵣH), 110,3 (CₐᵣH), 36,6 (d, *J* 15,2 Hz, CH₂), 36,5 (d, *J* 15,1 Hz, CH₂), 35,8 (^{t}C), 35,2 (2xCH₂), 35,1 (^{t}C), 35,0 (2x^{t}C), 35,0 (^{t}C), 31,8 (3xCH₃), 31,7 (d, *J* 2,1 Hz, 3xCH₃), 31,1 (d, *J* 2,7 Hz, 3xCH₃), 31,0 (d, *J* 3,0 Hz, 6xCH₃), 29,0 (d, *J* 2,5 Hz, CH₂), 28,9 (CH₂), 16,3 (CH₃), 16,0 (2xCH₃), 15,9 (CH₃).

HRMS (ESI-TOF) [M+H]⁺: *m*/*z* berechnet: für C₆₀H₈₀O₆P₂ 959,5508, gefunden: 959,5497.

HRMS (ESI-TOF) [M+Na]⁺: *m*/*z* berechnet: für C₆₀H₈₀O₆P₂ 981,5322, gefunden: 981,5317.

| | | | | |
|---|---|---|---|---|
| Elementaranalyse: | berechnet: | C 75,13% | H 8,40% | P 6,46% |
| | gefunden: | C 75,06% | H 8,28% | P 6,51%. |

### Verbindung I-17:

### 4,8-Di-tert-butyl-6-(2-(tert-butyl)-6-(3-(tert-butyl)-2-((5-(tert-butyl)benzo[d][1,3,2]dioxaphosphol-2-yl)oxy)-5-ethylbenzyl)-4-ethylphenoxy)-2,10-diethyl-12H-dibenzo[d,g][1,3,2]dioxaphosphocin

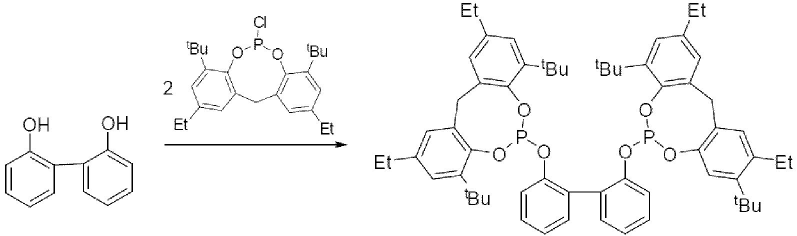

Zu einer Lösung von 2,2'-Biphenol (112 mg, 0,60 mmol) in THF (2 ml) wird bei 0°C zunächst Triethylamin (183 mg, 1,80 mmol) und dann langsam das 4,8-Di-*tert*-butyl-6-chloro-2,10-diethyl-12*H*-di-benzo[*d,g*][1,3,2]dioxaphosphocin (521 mg, 1,20 mmol) in THF (5 ml) gegeben. Es wird über Nacht bei Raumtemperatur weitergerührt und anschließend vom ausgefallenen Niederschlag abfiltriert. Das Filtrat wird eingeengt, der verbleibende Rückstand aus Acetonitril umkristallisiert und nach der Filtration und Trocknen das Bisphosphit praktisch rein erhalten.

Ausbeute: 420 mg weißer Feststoff (70%).

³¹P-NMR (CD₂Cl₂): □ +130,8 ppm.

¹H-NMR (CD₂Cl₂): □ 7,68 (2H, m, 2xHₐᵣ), 7,49 (2H,dd, *J* 7,6, 1,8 Hz, 2xHₐᵣ), 7,35 (2H, m, 2xHₐᵣ), 7,15 (2H, m, 2xHₐᵣ), 7,11 (4H, d, *J* 2,1 Hz, 4xHₐᵣ), 7,06 (4H, d, *J* 2,1 Hz, 4xHₐᵣ), 4,35 (2H, d, *J* 13,5 Hz, 2xHₐ-CH₂), 3,56 (2H, d, *J* 13,5 Hz, 2xH_{b}-CH₂), 2,61 (8H, q, *J* 7,6 Hz, 4xCH₂), 1,24 (36H, s, 4xC(CH₃)₃), 1,24 (12H, t, *J* 7,6 Hz, 4xCH₃).

¹³C-NMR (CD₂Cl₂): □ 150,2 (d, *J* 2,2 Hz, 2xCₐᵣ), 146,8 (4xCₐᵣ), 142,1 (d, *J* 3,5 Hz, 4xCₐᵣ), 140,5 (4xCₐᵣ), 135,2 (4xCₐᵣ), 133,3 (2xCₐᵣH), 130,0 (d, *J* 3,0 Hz, 2xCₐᵣ), 129,0 (2xCₐᵣH), 127,9 (4xCₐᵣH), 125,8 (4xCₐᵣH), 123,8 (2xCₐᵣH), 121,6 (d, *J* 11,0 Hz, 2xCₐᵣH), 36,3 (2xCH₂), 35,0 (4x^{t}C), 30,9 (d, *J* 3,0 Hz, 12xCH₃), 28,9 (4xCH₂), 16,0 (4xCH₃).

HRMS (ESI-TOF) [M+H]⁺: *m*/*z* berechnet: für C₆₂H₇₆O₆P₂ 979,5159, gefunden: 979,5182.

HRMS (ESI-TOF) [M+Na]⁺: *m*/*z* berechnet: für C₆₂H₇₆O₆P₂ 1001,5009, gefunden: 1001,5004.

| | | | | |
|---|---|---|---|---|
| Elementaranalyse: | berechnet: | C 76,05% | H 7,82% | P 6,33% |
| | gefunden: | C 76,16% | H 7,65% | P 6,16%. |

### Verbindung I-18:

### 3,8-Di-tert-butyl-6-(2-(2-((4,8-di-tert-butyl-2,10-diethyl-12H-dibenzo[d,g][1,3,2]dioxaphosphocin-6-yl)oxy)benzyl)phenoxy)-2,10-diethyl-12H-dibenzo[d,g][1,3,2]dioxaphosphocin

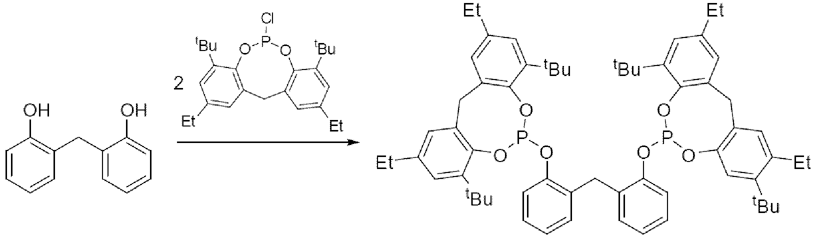

Zu einer Lösung von 2,2'-Methylendiphenol (147 mg, 0,73 mmol) in THF (2 ml) wird bei 0°C zunächst Triethylamin (222 mg, 2,20 mmol) und dann langsam das 4,8-Di-*tert*-butyl-6-chloro-2,10-diethyl-12*H*-di-benzo[*d,g*][1,3,2]dioxaphosphocin (634 mg, 1,20 mmol) in THF (6 ml) gegeben. Es wird über Nacht bei Raumtemperatur weitergerührt und anschließend vom ausgefallenen Niederschlag abfiltriert. Das Filtrat wird eingeengt, der verbleibende Rückstand aus Acetonitril umkristallisiert und nach der Kristallisation in der Kälte wird durch Filtration und Trocknen das Bisphosphit rein erhalten.

Ausbeute: 550 mg weißer Feststoff (75%).

³¹P-NMR (CD₂Cl₂): □ +132,1 ppm.

¹H-NMR (CD₂Cl₂): □ 7,73 (2H, m, 2xHₐᵣ), 7,27 (2H, dd,J 7,2, 1,7 Hz, 2xHₐᵣ), 7,23 (2H, dd,J 7,9, 1,8 Hz, 2xHₐᵣ), 7,19 (2H, d, *J* 2,2 Hz, 4xHₐᵣ), 7,10 (4H, d, *J* 2,2 Hz, 4Hₐᵣ), 7,09 (2H, ddd, *J* 7,5, 7,5, 1,2 Hz, 2xHₐᵣ), 4,42 (2H, dd, *J* 13,0, 2,9 Hz, 2xHₐ-CH₂), 4,38 (2H, s, CH₂), 3,57 (2H, d, *J* 13,0 Hz, 2xH_{b}-CH₂), 2,62 (8H, q, *J* 7,6 Hz, 4xCH₂), 1,34 (36H, s, 4xC(CH₃)₃), 1,25 (12H, q, *J* 7,6 Hz, 4xCH₃).

¹³C-NMR (CD₂Cl₂): □ 151,1 (d, *J* 5,3 Hz, 2xCₐᵣ), 146,3 (d, *J* 6,2 Hz, 4xCₐᵣ), 142,4 (d, 3,7 Hz, 4xCₐᵣ), 140,9 (4xCₐᵣ), 136,3 (4xCₐᵣ), 131,9 (d, *J* 2,5 Hz, 2xCₐᵣ), 131,6 (2xCₐᵣH), 127,9 (4xCₐᵣH), 127,5 (2xCₐᵣH), 126,1 (4xCₐᵣH), 124,3 (2xCₐᵣH), 121,0 (d, *J* 9,4 Hz, 2xCₐᵣH), 35,7 (2xCH₂), 35,1 (4x^{t}C), 30,9 (d, *J* 3,6 Hz, 12xCH₃), 30,8 (CH₂), 28,9 (4xCH₂), 16,0 (4xCH₃).

HRMS (ESI-TOF) [M+H]⁺: *m*/*z* berechnet: für C₆₃H₇₈O₆P₂ 993,5352, gefunden: 993,533682.

HRMS (ESI-TOF) [M+Na]⁺: *m*/*z* berechnet: für C₆₃H₇₈O₆P₂ 1015,5165, gefunden: 1015,5154.

| | | | | |
|---|---|---|---|---|
| Elementaranalyse: | berechnet: | C 76,18% | H 7,92% | P 6,24% |
| | gefunden: | C 76,11% | H 7,21% | P 6,25%. |

### Verbindung I-19:

### 3,8-Di-tert-butyl-6-((1-((2-((4,8-di-tert-butyl-2,10-diethyl-12H-dibenzo[d,g][1,3,2]dioxaphosphocin-6-yl)oxy)naphthalen-1-yl)methyl)naphthalen-2-yl)oxy)-2,10-diethyl-12H-dibenzo[d,g][1,3,2]dioxaphosphocine

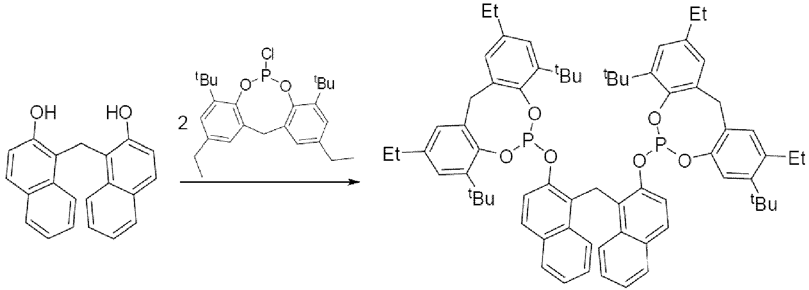

Zu einer Lösung von 1,1'-Methylenbis(naphthalen-2-ol) (150 mg, 0,5 mmol) in THF (3 ml) wird bei 0°C zunächst Triethylamin (152 mg, 1,50 mmol) und dann langsam das 4,8-Di-*tert*-butyl-6-chloro-2,10-diethyl-12*H*-dibenzo[*d,g*][1,3,2]dioxaphosphocin (434 mg, 1,00 mmol) in THF (4 ml) gegeben. Es wird über Nacht bei Raumtemperatur weitergerührt und anschließend vom ausgefallenen Niederschlag abfiltriert. Das Filtrat wird eingeengt und der verbleibende Rückstand in wenig Acetonitril in der Hitze suspendiert und nach dem Abkühlen abfiltriert.

Ausbeute: 320 mg weißer Feststoff (58%).

³¹P-NMR (CD₂Cl₂): □ +133,4 ppm.

¹H-NMR (CD₂Cl₂): □ 8,61 (2H, d, *J* 8,4 Hz, 2xHₐᵣ), 8,01 (2H, dd, *J* 8,9, 0,8 Hz, 2xHₐᵣ), 7,79 (2H, s, 2xHₐᵣ), 7,76 (2H, s, 2xHₐᵣ), 7,42 (2H, m, 2xHₐᵣ), 7,36 (2H, m, 2xHₐᵣ), 7,22 (2H, d, *J* 2,2 Hz, 4xHₐᵣ), 7,13 (2H, d, *J* 2,1 Hz, 4xHₐᵣ), 5,44 (2H, br s, CH₂), 4,53 (2H, dd, *J* 12,9, 2,9 Hz, 2xHₐ-CH₂), 3,57 (2H, d, *J* 12,9 Hz, 2xH_{b}-CH₂), 2,64 (8H, q, *J* 7,6 Hz, 4xCH₂), 1,38 (36H, s, 4xC(CH₃)₃), 1,27 (12H, t, *J* 7,6 Hz, 4xCH₃).

¹³C-NMR (CD₂Cl₂): □ 148,0 (d, *J* 5,7 Hz, 2xCₐᵣ), 146,1 (d, *J* 7,2 Hz, 4xCₐᵣ), 142,5 (d, *J* 3,6 Hz, 4xCₐᵣ), 141,1 (4xCₐᵣ), 136,6 (d, *J* 3,6 Hz, 4xCₐᵣ), 134,2 (2xCₐᵣ), 131,6 (2xCₐᵣ), 128,7 (2xCₐᵣH), 128,5 (2xCₐᵣH), 127,9 (4xCₐᵣH), 127,0 (d, *J* 2,6 Hz, 2xCₐᵣ), 126,6 (2xCₐᵣH), 126,2 (4xCₐᵣH), 125,6 (2xCₐᵣH), 124,9 (2xCₐᵣH), 121,9 (d, *J* 7,6 Hz, 2xCₐᵣH), 35,6 (2xCH₂), 35,1 (4x^{t}C), 31,0 (d, *J* 3,6 Hz, 12xCH₃), 28,9 (4xCH₂), 24,3 (CH₂) 16,0 (4xCH₃).

HRMS (ESI-TOF) [M+H]⁺: *m*/*z* berechnet: für C₇₁H₈₂O₆P₂ 1093,5665, gefunden: 1093,5673.

HRMS (ESI-TOF) [M+Na]⁺: *m*/*z* berechnet: für C₇₁H₈₂O₆P₂ 1115,5480, gefunden: 1115,5492.

| | | | | |
|---|---|---|---|---|
| Elementaranalyse: | berechnet: | C 78,00% | H 7,56% | P 5,67% |
| | gefunden: | C 77,69% | H 7,59% | P 5,44%. |

### Verbindung I-20:

### Bis(2-((2,4,8,10-tetra-tert-butyldibenzo[d,f][1,3,2]dioxaphosphepin-6-yl)oxy)naphthalen-1-yl)methan

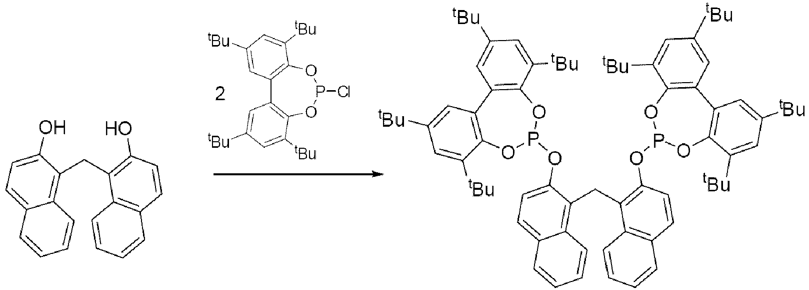

Zu einer auf 0°C abgekühlten Lösung von 1,1'-Methylenbis(naphthalen-2-ol) (150 mg, 0,50 mmol) und Triethylamin (152 mg, 1,50 mmol) in Toluol (4 ml) wird langsam eine Lösung des 2,4,8,10-Tetra-*tert*-butyl-6-chlorodibenzo[*d*,*f*][1,3,2]dioxaphosphepins (523 mg, 1,10 mmol) in Toluol (2 ml) zugetropft. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 530 mg weißer Feststoff (90%).

³¹P-NMR (CD₂Cl₂): □ +140,0 ppm.

¹H-NMR (CD₂Cl₂): □ 7,91 (2H, d, *J* 8,4 Hz, 2xHₐᵣ), 7,62 (2H, m, 2xHₐᵣ), 7,55 (2H, d, *J* 8,7 Hz, 2xHₐᵣ), 7,46 (4H, d, *J* 2,5 Hz, 4xHₐᵣ), 7,20 (4H, d, *J* 2,5 Hz, 4xHₐᵣ), 7,20 (2H, m, 2xHₐᵣ), 7,16 (2H, m, 2xHₐᵣ), 7,06 (2H, dd, *J* 8,5, 6,8, 1,4 Hz, 2xHₐᵣ), 4,97 (2H, br s, CH₂), 1,43 (36H, s, 4xC(CH₃)₃), 1,31 (36H, s, 4xC(CH₃)₃).

¹³C-NMR (CD₂Cl₂): □ 147,4 (4xCₐᵣ), 146,9 (2xCₐᵣ), 146,1 (d, *J* 6,4 Hz, 2xCₐᵣ), 140,7 (4xCₐᵣ), 133,7 (4xCₐᵣ), 133,1 (d, *J* 3,8 Hz, 4xCₐᵣ), 131,4 (2xCₐᵣ), 128,4 (4xCₐᵣH), 127,5 (d, *J* 2,8 Hz, 2xCₐᵣ), 127,1 (4xCₐᵣH), 126,4 (2xCₐᵣH), 125,4 (2xCₐᵣH), 124,8 (4xCₐᵣH), 124,8 (2xCₐᵣH), 121,7 (d, *J* 10,0 Hz, 2xCₐᵣH), 35,7 (4x^{t}C), 35,0 (4x^{t}C), 31,6 (12xCH₃), 31,3 (d, *J* 2,5 Hz, 12xCH₃), 24,0 (CH₂).

HRMS (ESI-TOF) [M+2O+H]⁺: *m*/*z* berechnet: für C₇₇H₉₄O₆P₂ 1209,6497, gefunden: 1209,6552.

HRMS (ESI-TOF) [M+2O+Na]⁺: *m*/*z* berechnet: für C₇₇H₉₄O₆P₂ 1231,6316, gefunden: 1231,6376.

### Verbindung I-21:

### Bis(2-((16H-dinaphtho[2,1-d:1',2'-g][1,3,2]dioxaphosphocin-8-yl)oxy)naphthalen-1-yl)methan

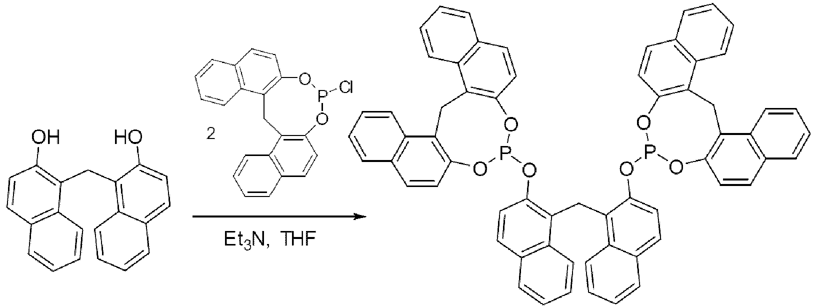

Zu einer Lösung von 8-Chloro-16*H*-dinaphtho[2,1-*d*:1',2'-*g*][1,3,2]dioxaphosphocin (113 mg, 0,76 mmol) in Tetrahydrofuran (4 ml) gibt man bei Raumtemperatur langsam eine Lösung von 1,1'-Methylenbis(naph-thalen-2-ol) (113 mg, 0,38 mmol) und Triethylamin (114 mg, 1,13 mmol) in Tetrahydrofuran (4 ml). Nach dem Rühren über Nacht wird vom Niederschlag abfiltriert und das Lösemittel unter Vakuum entfernt. Nach der Zugabe von Toluol wird von den unlöslichen Bestandteilen erneut abfiltriert und das Filtrat eingeengt. Der Rückstand wurde aus Acetonitril umkristallisiert und man erhält das Bisphosphit als weißen Feststoff.

Ausbeute: 210 mg weißer Feststoff (58%).

³¹P-NMR (CD₂Cl₂): □ +123,8 ppm.

¹H-NMR (CD₂Cl₂): □ 8,31 (2H, m, 2xHₐᵣ), 8,23 (4H, d, *J* 8,4 Hz, 4xHₐᵣ), 7,86-7,67 (10H, m, 10xHₐᵣ), 7,58-7,49 (8H, m, 8xHₐᵣ), 7,46-7,34 (8H, m, 8xHₐᵣ), 7,16 (4H,d, *J* 8,8 Hz, 4xHₐᵣ), 5,26 (2H, s, CH₂), 5,20 (2H, d, *J* 16,1 Hz, 2xHₐ-CH₂), 4,58 (2H, d, *J* 16,1 Hz, 2xH_{b}-CH₂).

HRMS (ESI-TOF) [M+H]⁺: *m*/*z* berechnet: für C₆₃H₄₂O₆P₂ 957.2535, gefunden: 957,2537.

HRMS (ESI-TOF) [M+Na]⁺: *m*/*z* berechnet: für C₆₃H₄₂O₆P₂ 979,2354, gefunden: 979,2349.

| | | | | |
|---|---|---|---|---|
| Elementaranalyse: | berechnet: | C 79,07% | H 4,42% | P 6,47% |
| | gefunden: | C 79,16% | H 4,11% | P 6,18%. |

### Verbindung I-22:

### rac-4,8-Di-tert-butyl-1,2,10,11-tetramethyl-6-((1-((2-((2,4,8,10-tetra-tert-butyldibenzo[d,f][1,3,2]dioxaphosphepin-6-yl)oxy)naphthalen-1-yl)methyl)naphthalen-2-yl)oxy)dibenzo[d,f][1,3,2]dioxaphosphepin

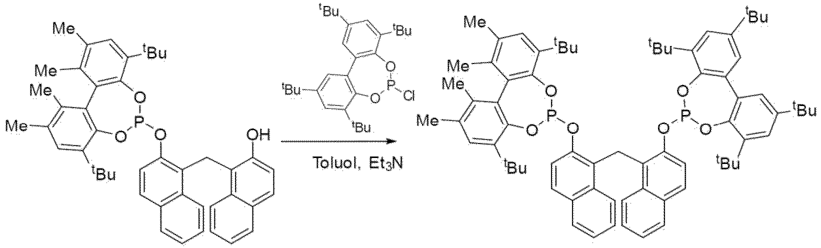

Eine Lösung von 1-((2-((*rac*-4,8-Di-*tert*-butyl-1,2,10,11-tetramethyldibenzo[*d*,*f*][1,3,2]dioxaphosphepin-6-yl)oxy)naphthalen-1-yl)methyl)naphthalen-2-ol (250 mg, 0,37 mmol) und Triethylamin (56 mg, 0,55 mmol) in Toluol (4 ml) wird auf 0°C abgekühlt und langsam mit einer Lösung des 2,4,8,10-Tetra-*tert*-butyl-6-chlo-rodibenzo[*d*,*f*][1 ,3,2]dioxaphosphepins (209 mg, 0,44 mmol) in Toluol (2 ml) versetzt. Nach 30 Minuten lässt man auf Raumtemperatur erwärmen und die Reaktionsmischung über Nacht weiterrühren. Anschließend wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 270 mg weißer Feststoff (66%).

Schmelzpunkt: 178-180°C.

³¹P-NMR (CD₂Cl₂): □ +140,5 ppm und +133,6 ppm.

¹H-NMR (CD₂Cl₂): □ 7,80 (1H, d, *J* 8,1 Hz, Hₐᵣ), 7,74 (1H, d, *J* 8,1 Hz, Hₐᵣ), 7,62 (1H, dm, *J* 8,1 Hz, Hₐᵣ), 7,62 (1H, s, Hₐᵣ), 7,59 (1H, s, Hₐᵣ), 7,55 (1H, d, *J* 8,9 Hz, Hₐᵣ), 7,47 (1H, d, *J* 2,6 Hz, Hₐᵣ), 7,46 (1H, d, *J* 2,5 Hz, Hₐᵣ), 7,35 (1H, dd, *J* 8,8, 0,9 Hz, Hₐᵣ), 7,22 (1H, d, *J* 2,5 Hz, Hₐᵣ), 7,19 (1H, d, *J* 2,5 Hz, Hₐᵣ), 7,19-7,10 (5H, m, 5xHₐᵣ), 6,98 (1H, m, Hₐᵣ), 6,97 (1H, m, Hₐᵣ), 4,87 (1H, d, *J* 16,0 Hz, Hₐ-CH₂), 4,71 (1H, d, *J* 16,0 Hz, H_{b}-CH₂), 2,27 (3H, s, CH₃), 1,97 (3H, s, CH₃), 1,81 (3H, s, CH₃), 1,59 (3H, s, CH₃), 1,52 (9H, s, C(CH₃)₃), 1,48 (9H, s, C(CH₃)₃), 1,45 (9H, s, C(CH₃)₃), 1,32 (9H, s, C(CH₃)₃), 1,31 (9H, s, C(CH₃)₃), 1,30 (9H, s, C(CH₃)₃).

¹³C-NMR (CD₂Cl₂): □ 147,5-130,9 (24xCₐᵣ), 128,5 (CₐᵣH), 128,3 (3xCₐᵣH), 128,3(2xCₐᵣH), 127,8 (d, *J* 2,9 Hz, Cₐᵣ), 127,1 (2xCₐᵣH), 126,7 (Cₐᵣ), 126,4 (CₐᵣH), 126,3 (CₐᵣH), 125,6 (CₐᵣH), 125,4 (CₐᵣH), 124,9 (CₐᵣH), 124,9 (CₐᵣH), 124,8 (CₐᵣH), 124,6 (CₐᵣH), 121,8 (d, *J* 10,1 Hz, CₐᵣH), 121,0 (d, *J* 10,5 Hz, CₐᵣH), 35,8 (2x^{t}C), 35,2 (^{t}C), 35,0 (^{t}C), 34,9 (^{t}C), 34,9 (^{t}C), 31,8 (3xCH₃), 31,6 (6xCH₃), 31,4 (d, 3xCH₃), 31,4 (6xCH₃), 23,8 (CH₂), 20,5 (CH₃), 20,2 (CH₃), 16,8 (CH₃), 16,6 (CH₃).

HRMS (ESI-TOF) [M+H]⁺: *m*/*z* berechnet: für C₇₃H₈₆O₆P₂ 1121,5973, gefunden: 1121,6003.

HRMS (ESI-TOF) [M+Na]⁺: *m*/*z* berechnet: für C₇₃H₈₆O₆P₂ 1143,5792, gefunden: 1143,5822.

### Verbindung I-23:

### Bis(2-((rac-4,8-di-tert-butyl-1,2,10,11-tetramethyldibenzo[d,f][1,3,2]dioxaphosphepin-6-yl)oxy)-phenyl)me-thanon

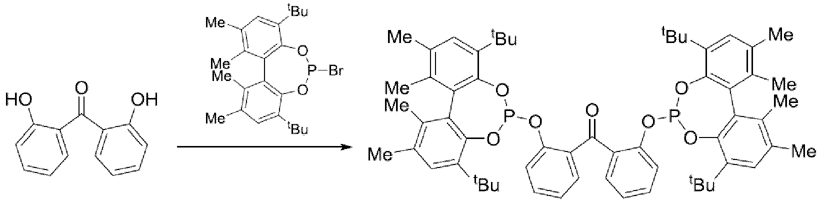

Zu einer Lösung des *rac*-6-Bromo-4,8-di-tert-butyl-1,2,10,11-tetramethyldibenzo[*d*,*f*][1,3,2]dioxa-phosphe-pins (880 mg, 1,90 mmol) in Toluol (6 ml) werden bei Raumtemperatur zunächst Triethylamin (288 mg, 2,85 mmol) und anschließend 2,2'-Dihydroxybenzophenon (204 mg, 0,95 mmol) gegeben. Nach dem Rühren über Nacht wird vom ausgefallenen Niederschlag abfiltriert und das Filtrat unter Vakuum eingeengt. Der verbleibende Rückstand wird zunächst durch Säulenchromatographie (Eluent: Cyclohexan/AcOEt 99/1) gereinigt. Um das Produkt vom Nebenprodukt (Monophosphit) zu befreien, wurde das isolierte Diastereomerengemisch aus der Säulenchromatographie mit wenig Acetonitril umkristallisiert (ca. 95% Reinheit im ³¹P-NMR).

Ausbeute: 200 mg weißer Feststoff (21%).

Schmelzpunkt: 220-222°C.

³¹P-NMR (CD₂Cl₂): □ +129,8 ppm (60%) und +129,5 ppm (40%), Gemisch aus (*R,R*)/(*S,S*)*-* und (*R*,*S*)-Verbindung.

¹H-NMR (CD₂Cl₂): Diastereomerengemisch, □ 7,20-7,08 (16H, m, 16xHₐᵣ), 6,87 (2H, m, 2xHₐᵣ), 6,82 (2H, m, 2xHₐᵣ), 6,47 (2H, m, 2xHₐᵣ) 6,34 (2H, m, 2xHₐᵣ), 2,33 (12H, s, 4xCH₃), 2,25 (12H, s, 4xCH₃), 1,83 (12H, s, 4xCH₃), 1,79 (12H, s, 4xCH₃), 1,31 (18H, s, 2xC(CH₃)₃), 1,30 (18H, s, 2xC(CH₃)₃), 1,29 (18H, s, 2xC(CH₃)₃), 1,28 (18H, s, 2xC(CH₃)₃).

¹³C-NMR (CD₂Cl₂): Diastereomerengemisch, □ 194,4 (C=O), 194,1 (C=O), 150,2 (d, *J* 2,7 Hz, 2xCₐᵣ), 150,1 (2xCₐᵣ), 145,4 (d, *J* 4,7 Hz, 2xCₐᵣ), 145,3 (d, *J* 5,4 Hz, 2xCₐᵣ), 145,0 (2xCₐᵣ), 144,8 (2xCₐᵣ), 138,8 (d, *J 2,7* Hz, 2xCₐᵣ), 138,8 (d, *J* 3,1 Hz, 2xCₐᵣ), 137,8 (2xCₐᵣ), 137,8 (2xCₐᵣ), 135,4 (2xCₐᵣ), 135,4 (2xCₐᵣ), 134,8 (2xCₐᵣ), 134,8 (2xCₐᵣ), 133,3 (2xCₐᵣ), 133,2 (2xCₐᵣ), 132,5 (d, *J* 2,6 Hz, 2xCₐᵣ), 132,5 (d, *J* 3,0 Hz, 2xCₐᵣ), 132,3 (4xCₐᵣ), 132,3 (2xCₐᵣH), 132,2 (4xCₐᵣ),132,2 (2xCₐᵣH), 131,2 (2xCₐᵣ), 131,2 (2xCₐᵣH), 131,1 (d, *J* 2,6 Hz, 2xCₐᵣ), 131,0 (2xCₐᵣH), 128,6 (4xCₐᵣH), 128,2 (4xCₐᵣH), 123,7 (2xCₐᵣH), 123,7 (2xCₐᵣH), 122,7 (d, *J* 8,5 Hz, 2xCₐᵣH), 122,1 (d, *J* 10,5 Hz, 2xCₐᵣH), 35,0 (2x^{t}C), 34,8 (^{t}C), 34,8 (^{t}C), 31,5 (12xCH₃), 31,3 (d, *J* 4,5 Hz, 6xCH₃), 31,2 (d, *J* 4,6 Hz, 6xCH₃), 20,5 (CH₃), 20,5 (CH₃), 20,5 (2xCH₃), 16,8 (2xCH₃), 16,6 (CH₃), 16,5 (CH₃).

HRMS (ESI-TOF) [M+H]⁺: *m*/*z* berechnet: für C₆₁H₇₂O₇P₂ 979,4832, gefunden: 979,4835.

HRMS (ESI-TOF) [M+Na]⁺: *m*/*z* berechnet: für C₆₁H₇₂O₇P₂ 1001,4645, gefunden: 1001,4656.

### Verbindung I-24:

### Bis(2-((2,4,8,10-tetra-tert-butyldibenzo[d,f][1,3,2]dioxaphosphepin-6-yl)oxy)phenyl)methanon

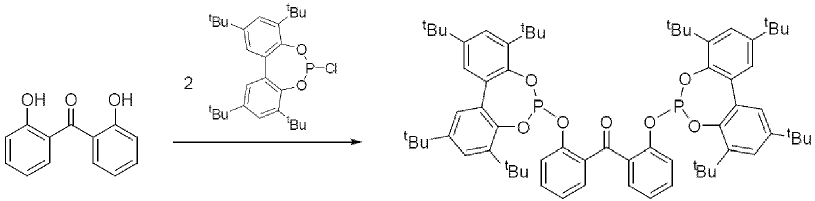

Zu einer Lösung von 2,2'-Dihydroxybenzophenon (107 mg, 0,50 mmol) und Triethylamin (111 mg, 1,10 mmol) in Tetrahydrofuran (4 ml) wird bei Raumtemperatur langsam eine Lösung des 2,4,8,10-Tetra-*tert*-butyl-6-chlorodibenzo[*d,f*][1,3,2]dioxaphosphepins (499 mg, 1,05 mmol) in THF (4 ml) getropft. Nach dem Rühren über Nacht wird vom ausgefallenen Niederschlag abfiltriert und das Reaktionsgemisch unter Vakuum eingeengt. Das erhaltene Rohprodukt wird durch Umkristallisation aus Acetonitril gereinigt.

Ausbeute: 280 mg weißer Feststoff (51%).

Schmelzpunkt: 175-177°C.

³¹P-NMR (CD₂Cl₂): □ ⁺136,2 ppm.

¹H-NMR (CD₂Cl₂): □ 7,44 (4H, d, *J* 2,4 Hz, 4xHₐᵣ), 7,27 (2H, dd, *J* 7,7, 1,7 Hz, 2xHₐᵣ), 7,16 (4H, d, *J* 2,5 Hz, 4xHₐᵣ), 7,11 (2H, ddd, *J* 8,2, 7,4, 1,8 Hz, 2xHₐᵣ), 6,91 (2H, dt, *J* 7,6, 0,9 Hz, 2xHₐᵣ), 6,33 (2H, m, 2xHₐᵣ), 1,38 (36H, s, 4xC(CH₃)₃), 1,35 (36H, s, 4xC(CH₃)₃).

¹³C-NMR (CD₂Cl₂): □ 194,0 (C=O), 150,2 (d, *J* 4,7 Hz, 4xCₐᵣ), 147,4 (4xCₐᵣ), 146,0 (d, *J* 5,0 Hz, 2xCₐᵣ), 140,8 (4xCₐᵣ), 133,2 (d, *J* 3,8 Hz, 4xCₐᵣ), 132,6 (2xCₐᵣH), 132,2 (d, *J* 3,1 Hz, 2xCₐᵣ), 131,3 (2xCₐᵣH), 126,9 (4xCₐᵣH), 124,8 (4xCₐᵣH), 123,8 (2xCₐᵣH), 122,8 (d, *J* 7,3 Hz, 2xCₐᵣH), 35,6 (4x^{t}C), 35,0 (4x^{t}C), 31,7 (12xCH₃), 31,2 (d, *J* 2,4 Hz, 12xCH₃).

HRMS (ESI-TOF) [M+H]⁺: *m*/*z* berechnet: für C₆₉H₈₈O₇P₂ 1091,6084, gefunden: 1091,6088.

HRMS (ESI-TOF) [M+Na]⁺: *m*/*z* berechnet: für C₆₉H₈₈O₇P₂ 1113,5898, gefunden: 1113,5907.

### Verbindung I-25:

### Bis(2-((4,8-di-tert-butyl-2,10-dimethoxydibenzo[d,f][1,3,2]dioxaphosphepin-6-yl)oxy)phen-yl)methanone

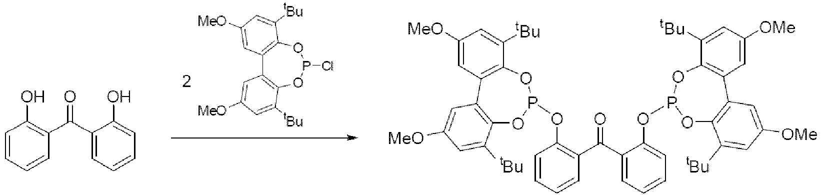

Zu einer Lösung von 2,2'-Dihydroxybenzophenon (130 mg, 0,61 mmol) und Triethylamin (184 mg, 1,82 mmol) in Tetrahydrofuran (3 ml) wird bei Raumtemperatur langsam eine Lösung des 4,8-Di-*tert-*butyl-6-chloro-2,10-dimethoxydibenzo[*d,f*][1,3,2]dioxaphosphepins (516 mg, 1,82 mmol) in THF (4 ml) getropft. Nach dem Rühren über Nacht wird vom ausgefallenen Niederschlag abfiltriert und das Reaktionsgemisch unter Vakuum eingeengt. Aus dem verbleibenden Rückstand wird durch Säulenchromatographie (Eluent: Dichlormethan) das Bisphosphit erhalten.

Ausbeute: 440 mg weißer Feststoff (75%).

³¹P-NMR(CD₂CI₂): □ +137,7 ppm.

¹H-NMR (CD₂Cl₂): □ 7,24 (2H, dd, *J* 7,7, 1,8 Hz, 2xHₐᵣ), 7,20 (2H, ddd, *J* 8,1, 7,5, 1,7 Hz, 2xHₐᵣ), 6,95 (4H, d, *J* 3,1 Hz, 4xHₐᵣ), 6,90 (2H, dt, *J* 7,6, 0,8 Hz, 2xHₐᵣ), 6,70 (4H, d, *J* 3,1 Hz, 4xHₐᵣ), 6,64 (2H, m, 2xHₐᵣ), 3,83 (12H, s, 4xOCH₃) 1,30 (36H, s, 4xC(CH₃)₃).

¹³C-NMR (CD₂Cl₂): □ 194,2 (C=O), 156,3 (4xCₐᵣ-OMe), 149,9 (2xCₐᵣ), 143,1 (4xCₐᵣ), 141,7 (d, *J* 5,6 Hz, 2xCₐᵣ), 134,0 (d, *J* 3,2 Hz, 4xCₐᵣ), 132,7 (d, *J* 2,5 Hz, 2xCₐᵣ), 132,6 (2xCₐᵣH), 131,2 (2xCₐᵣH), 124,0 (2xCₐᵣH), 122,3 (d, *J* 9,1 Hz, 2xCₐᵣH), 114,7 (4xCₐᵣH), 113,2 (4xCₐᵣH), 56,0 (4xOCH₃), 35,6 (4x^{t}C), 31,0 (d, *J* 1,8 Hz, 12xCH₃).

HRMS (ESI-TOF) [M+H]⁺: *m*/*z* berechnet: für C₅₇H₆₄O₁₁P₂ 987,4002, gefunden: 987,4010.

HRMS (ESI-TOF) [M+Na]⁺: *m*/*z* berechnet: für C₅₇H₆₄O₁₁P₂ 1009,3816, gefunden: 1009,3824.

| | | | | |
|---|---|---|---|---|
| Elementaranalyse: | berechnet: | C 69,36% | H 6,54% | P 6,28% |
| | gefunden: | C 69,26% | H 6,30% | P 6,24%. |

### Verbindung I-26:

### (2-((3,8-Di-tert-butyl-2,10-diethyl-12H-dibenzo[d,g][1,3,2]dioxaphosphocin-6-yl)oxy)phenyl)(2-((4,8-di-tert-butyl-2,10-diethyl-12H-dibenzo[d,g][1,3,2]dioxaphosphocin-6-yl)oxy)phenyl)methanon

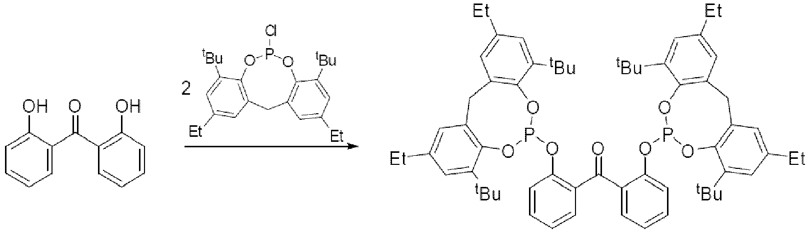

Zu einer Lösung von 2,2'-Dihydroxybenzophenon (138 mg, 0,64 mmol) in THF (2 ml) wird bei 0°C zunächst Triethylamin (195 mg, 1,93 mmol) und dann langsam das 4,8-Di-*tert-*butyl-6-chloro-2,10-diethyl-12*H*-dibenzo[*d,g*][1,3,2]dioxaphosphocin (558 mg, 1,29 mmol) in THF (6 ml) gegeben. Es wird über Nacht bei Raumtemperatur weitergerührt und anschließend vom ausgefallenen Niederschlag abfiltriert. Das Filtrat wird eingeengt und der verbleibende Rückstand aus Acetonitril umkristallisiert. Nach der Kristallisation in der Kälte wird durch Filtration und Trocknen das Bisphosphit rein erhalten.

Ausbeute: 560 mg weißer Feststoff (86%).

³¹P-NMR (CD₂Cl₂): □ ⁺131,1 ppm.

¹H-NMR (CD₂Cl₂): □ 7,71 (2H, dd, *J* 7,7, 1,7 Hz, 2xHₐᵣ), 7,57 (2H, m, 2xHₐᵣ), 7,46 (2H, ddd, *J* 8,3, 7,4, 1,8 Hz, 2xHₐᵣ), 7,22 (2H, ddd, *J* 7,5, 7,5, 0,9 Hz, 2xHₐᵣ), 7,13 (4H, d, *J* 2,1 Hz, 4xHₐᵣ), 7,06 (4H, d, *J* 2,1 Hz, 4xHₐᵣ), 4,27 (2H, dd, *J* 12,9, 2,9 Hz, 2xHₐ-CH₂), 3,43 (2H, d, *J* 13,0 Hz, 2xH_{b}-CH₂), 2,60 (8H, q, *J* 7,6 Hz, 4xCH₂), 1,23 (36H, s, 4xC(CH₃)₃), 1,23 (12H, q, *J* 7,6 Hz, 4xCH₃).

¹³C-NMR (CD₂Cl₂): □ 194,3 (C=O), 150,8 (d, *J* 6,0 Hz, 2xCₐᵣ), 145,8 (d, *J* 6,5 Hz, 4xCₐᵣ), 142,5 (d, 5,0 Hz, 4xCₐᵣ), 140,9 (4xCₐᵣ), 136,4 (d, *J* 6,2 Hz, 4xCₐᵣ), 134,1 (d, *J* 2,3 Hz, 2xCₐᵣ), 131,6 (2xCₐᵣH), 131,0 (2xCₐᵣH), 127,8 (4xCₐᵣH), 126,0 (4xCₐᵣH), 124,3 (2xCₐᵣH), 122,3 (d, *J* 7,4 Hz, 2xCₐᵣH), 35,4 (2xCH₂), 35,0 (4x^{t}C), 30,9 (d, *J* 4,0 Hz, 12xCH₃), 28,9 (4xCH₂), 16,0 (4xCH₃).

HRMS (ESI-TOF) [M+Na]⁺: *m*/*z* berechnet: für C₆₃H₇₆O₇P₂ 1029,4948, gefunden: 1029,4967.

| | | | | |
|---|---|---|---|---|
| Elementaranalyse: | berechnet: | C 75,13% | H 7,61% | P 6,15% |
| | gefunden: | C 74,63% | H 7,23% | P 6,08%. |

### Verbindung I-27:

### (4S,5S)-trans-Bis(diphenylphosphinoxymethyl)-2,2-dimethyl-1,3-dioxolan

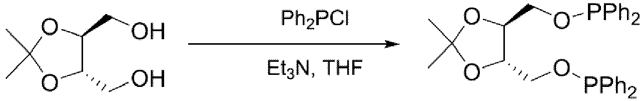

Zu einer Lösung von 307 mg 2,3-O-Isopropylidene-L-threitol (1,89 mmol) und Triethylamin (575 mg, 5,67 mmol) in THF (10 ml) tropft man unter Rühren bei 0°C (Eisbad) eine Lösung von Chlordiphenylphosphin (876 mg, 3,97 mmol) in THF (10 ml) hinzu. Nach etwa 20 Stunden wird vom Niederschlag abfiltriert und das Lösemittel im Vakuum entfernt. Versuche zur Kristallisation scheiterten, so dass der viskose Rückstand mittel Flash-Chromatographie (Eluent AcOEt) gereinigt und das Produkt als viskoser Sirup isoliert.

Ausbeute: 573 mg zäher Sirup (57%).

³¹P-NMR (CD₂Cl₂): □ ⁺116,0 ppm.

¹H-NMR ( CD₂Cl₂): □ 7,58-7,48 (8H, m, Hₐᵣ), 7,42-7,34 (12H, m, Hₐᵣ), 4,16 (2H, m, CH-O), 4,00 (4H, m, CH₂O), 1,36 (6H, s, 2xCH₃).

¹³C-NMR ( CD₂Cl₂): □ 144,2 (m, 2xCₐᵣ-P), 130,7 (d, *J* 22,0 Hz, 4xCHₐᵣ), 129,8 (2xCHₐᵣ), 128,8 (d, *J* 6,9 Hz, 4xCHₐᵣ), 110,0 (C), 78,1 (d, *J* 8,3 Hz, 2xCHO), 70,4 (d, *J* 18,9 Hz, 2xCH₂O), 27,2 (2xCH₃).

HRMS (ESI-TOF) [M+H]⁺: *m*/*z* berechnet: für C₃₁H₃₂O₄P₂ 531,1854, gefunden: 531,1851.

### Verbindung I-28:

### 2,2'-((((4S,5S)-2,2-dimethyl-1,3-dioxolane-4,5-diyl)bis(methylene))bis(oxy))dinaphtho[1,8-de][1,3,2]di-ox-aphosphinin

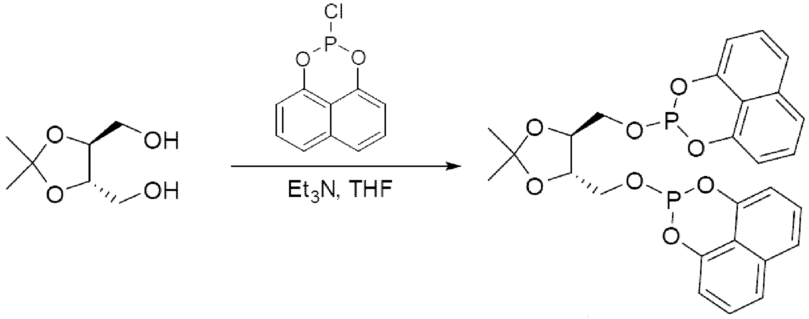

**Zu einer** Lösung von 2,3-O-Isopropylidene-L-threitol (142 mg, 0,88 mmol) und Triethylamin (266 mg, 2,63 mmol) in THF (2 ml) tropft man unter Rühren bei 0°C (Eisbad) eine Lösung des 2-Chlornaphtho[1,8-de][1,3,2]dioxaphosphinins (414 mg, 1,84 mmol) in THF (6 ml) hinzu. Nach dem Rühren über Nacht wird vom Niederschlag abfiltriert, das Lösemittel im Vakuum entfernt und der verbleibende Rückstand getrocknet (Vakuum, 60°C). Das Produkt konnte ohne weitere Reinigung weiterverwendet werden.

Ausbeute: 368 mg mobiler Sirup (77%).

³¹P-NMR (CD₂Cl₂): □ +112,4 ppm.

¹H-NMR (CD₂Cl₂): □ 7,49 (2H, m, 2xHₐᵣ), 7,47 (2H, m, 2xHₐᵣ), 7,38 (1H, d, *J* 7,5 Hz, 1xHₐᵣ), 7,35 (2H, d, *J* 7,5 Hz, 2xHₐᵣ), 7,33 (1H, d, *J* 7,5 Hz, 1xHₐᵣ), 6,90 (2H, dt, *J* 7,5, 0,9 Hz, 2xHₐᵣ), 6,86 (2H, dt, *J* 7,5, 0,9 Hz, 2xHₐᵣ), 3,78-2,60 (4H, m, 2xCH₂O), 3,54 (2H, m, 2xCH-O), 1,18 (6H, s, 2xCH₃).

¹³C-NMR (CD₂Cl₂): □ 144,4 (m, 4xCₐᵣ-O), 135,3 (d, *J* 1,9 Hz, 2xCₐᵣ), 127,6 (4xCₐᵣH), 122,5 (4xCₐᵣH), 117,0 (d, *J* 14,0 Hz, 2xCₐᵣ), 112,3 (d, *J* 2,0 Hz, 4xCₐᵣH), 110,2 (C), 76,6 (d, *J* 4,5 Hz, CH-O), 63,7 (d, *J* 13,7 Hz, CH₂-O), 26,7 (2xCH₃).

HRMS (ESI-TOF) [M+H]⁺: *m*/*z* berechnet: für C₂₇H₂₄O₈P₂ 539,1025, gefunden: 539,1033.

| | | | | |
|---|---|---|---|---|
| Elementaranalyse: | berechnet: | C 60,23% | H 4,49% | P 11,51% |
| | gefunden: | C 60,08% | H 4,35% | P 11,61%. |

### Verbindung I-29:

### Tetraethyl2,2'-((((4S,5S)-2,2-dimethyl-1,3-dioxolane-4,5-diyl)bis(methylene))bis(oxy))(4R,4'R,5R,5'R)-bis(1,3,2-dioxaphospholane-4,5-dicarboxylat)

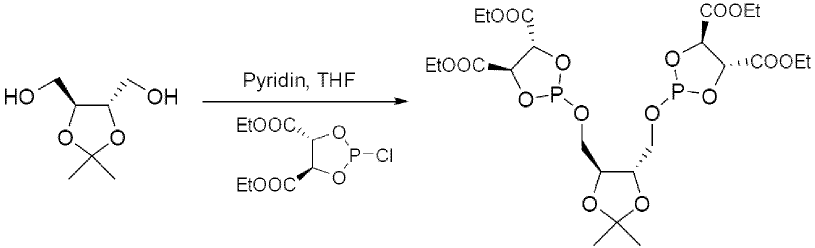

Eine Lösung von (+)-2,3-O-Isopropyliden-L-threitol (162 mg, 1.00 mmol) und Pyridin (166 mg, 2.10 mmol) in THF (5 ml) wird bei 0°C (Eisbad) zu der in THF (4 ml) gelösten Phosphinchlorid-Verbindung (568 mg, 2.10 mmol) langsam unter Rühren getropft. Nach 30 Minuten lässt man auf Raumtemperatur erwärmen und rührt über Nacht weiter. Zur Aufarbeitung wird vom ausgefallenen Niederschlag abfiltriert und dieser einmal mit THF (3 ml) nachgewaschen. Das Lösemittel wird unter Vakuum entfernt und der Rückstand weist eine genügende Reinheit (>95%) auf.

Ausbeute: 160 mg farbloser Sirup (25%).

³¹P-NMR (CDCl₃): □ +144,4 ppm.

¹H-NMR (CDCl₃): □ 5,09 (2H, d, *J* 6,2 Hz, 2xCH-O), 4,73 (2H, dd, *J* 8,6, 6,2 Hz, 2xCH-O), 4,33-4,16 (8H, m, 4xCH₂O), 3,96-3,88 (6H, m, 2xCH-O, 2xCH₂O), 1,37 (6H, s, 2xCH₃), 1,28 (6H, t, *J* 7,1 Hz, 2xCH₃), 1,28 (6H, t, *J* 7,1 Hz, 2xCH₃).

¹³C-NMR (CDCl₃): □ 168,3 (C=O), 168,3 (d, *J* 5,0 Hz, C=O), 110,0 (^{t}CO₂), 76,8 (d, *J* 9,1 Hz, 2xCH-O), 76,1 (d, *J* 4,5 Hz, 2xCH-O), 75,8 (d, *J* 8,9 Hz, 2xCH-O), 62,8 (d, *J* 12,8 Hz, 2xCH₂O), 62,2 (4xCH₂O), 26,8 (2xCH₃), 14,0 (4xCH₃).

HRMS (ESI-TOF): [M+Na]⁺: *m*/*z* berechnet: für C₂₃H₃₆O₁₆P₂ 653,1376, gefunden: 653,1386.

### Verbindung I-30:

### 6,6'-((Oxybis(2,1-phenylen))bis(oxy))bis(2,4,8,10-tetra-tert-butyldibenzo[d,f][1,3,2]dioxaphosphepin)

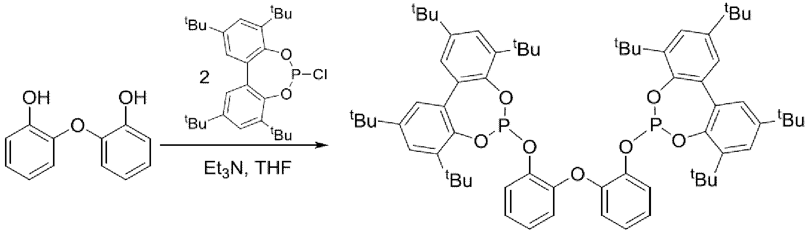

**Zu einer** Lösung von 2,2'-Oxydiphenol (93,5 mg, 0,46 mmol) und Triethylamin (141 mg, 1,39 mmol) in THF (2 ml) tropft man unter Rühren bei 0°C (Eisbad) eine Lösung von 2,4,8,10-Tetra-*tert*-butyl-6-chlorodi-benzo[*d,f*][1,3,2]dioxaphosphepin (439 mg, 0,92 mmol) in THF (5 ml) hinzu. man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand in Acetonitril (5 ml) aufgenommen und bei Siedehitze gelöst. Der beim Abkühlen in der Kälte ausgefallene Niederschlag wird abgetrennt und unter Vakuum bei 60°C getrocknet.

Ausbeute: 305 mg weißer Feststoff (61%).

³¹P-NMR (CD₂Cl₂): □ +136,9 ppm.

¹H-NMR (CD₂Cl₂): □ 7,47 (4H, d, *J* 2,5 Hz, 4xHₐᵣ), 7,23 (4H, d, *J* 2,5 Hz, 4xHₐᵣ), 7,08-6,96 (6H, m, 6xHₐᵣ), 6,90 (2H, m, 2xHₐᵣ), 1,39 (36H, s, 4xC(CH₃)₃), 1,38 (36H, s, 4xC(CH₃)₃).

¹³C-NMR (CD₂Cl₂): □ 148,6 (2xCₐᵣ), 147,4 (4xCₐᵣ), 145,9 (2xCₐᵣ), 143,2 (4xCₐᵣ), 141,0 (4xCₐᵣ), 133,3 (4xCₐᵣ), 126,9 (4xCₐᵣH), 125,3 (2xCₐᵣH), 125,0 (4xCₐᵣH), 124,3 (2xCₐᵣH), 123,8 (m, 2xCₐᵣH), 120,4 (2xCₐᵣH), 35,8 (4x^{t}C), 35,1 (4x^{t}C), 31,7 (12xCH₃), 31,4 (12xCH₃).

HRMS (ESI-TOF) [M+H]⁺: m/z berechnet: für C₆₈H₈₈O₇P₂ 1079,6084, gefunden: 1079,6101.

HRMS (ESI-TOF) [M+Na]⁺: m/z berechnet: für C₆₈H₈₈O₇P₂ 1101,5898, gefunden: 1101,5920.

| | | | | |
|---|---|---|---|---|
| Elementaranalyse: | berechnet: | C 75,67% | H 8,22% | P 5,74% |
| | gefunden: | C 74,82% | H 8,30% | P 5,31%. |

### Verbindung I-31:

### 6,6'-((Oxybis(2,1-phenylen))bis(oxy))bis(4,8-di-tert-butyl-2,10-dimethoxydibenzo[d,f][1,3,2]di-oxaphosphe-pin)

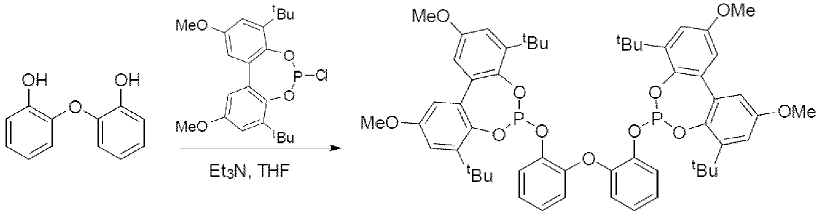

Zu einer Lösung von 2,2'-Oxydiphenol (116 mg, 0,58 mmol) und Triethylamin (175 mg, 1,73 mmol) in THF (2 ml) tropft man unter Rühren bei 0°C (Eisbad) eine Lösung von 4,8-Di-*tert*-butyl-6-chloro-2,10-di-methoxydibenzo[*d,f*][1,3,2]dioxaphosphepin (439 mg, 0,92 mmol) in THF (5 ml) hinzu. man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand in Heptan (4 ml) aufgenommen und bei Siedehitze gelöst. Der beim Abkühlen in der Kälte ausgefallene Niederschlag wird abgetrennt und unter Vakuum bei 60°C getrocknet.

Ausbeute: 250 mg weißer Feststoff (44%).

³¹P-NMR (CD₂Cl₂): □ +137,7 ppm.

¹H-NMR (CD₂Cl₂): □ 7,11-7,01 (6H, m, 6xHₐᵣ), 6,98 (4H, d, *J* 3,1 Hz, 4xHₐᵣ), 6,87 (2H, m, 2xHₐᵣ), 6,75 (4H, d, *J* 3,1 Hz, 4xHₐᵣ), 3,81 (12H, s, 4xOCH₃), 1,36 (36H, s, 4xC(CH₃)₃).

¹³C-NMR (CD₂Cl₂): □ 156,3 (4xCₐᵣ-OMe), 148,6 (2xCₐᵣ), 143,3 (4xCₐᵣ), 143,1 (4xCₐᵣ), 141,7 (m, 2xCₐᵣ), 134,1 (4xCₐᵣ), 125,3 (2xCₐᵣH), 124,3 (2xCₐᵣH), 123,6 (m, *J* 6,6 Hz, 2xCₐᵣH), 120,4 (2xCₐᵣH), 114,8 (4xCₐᵣH), 113,3 (4xCₐᵣH), 56,0 (4xOCH₃), 35,7 (4x^{t}C), 31,1 (12xCH₃).

HRMS (ESI-TOF) [M+H]⁺: m/z berechnet: für C₅₆H₆₄O₁₁P₂ 975,4002, gefunden: 975,3986.

HRMS (ESI-TOF) [M+Na]⁺: m/z berechnet: für C₅₆H₆₄O₁₁P₂ 997,3816, gefunden: 997,3822.

| | | | | |
|---|---|---|---|---|
| Elementaranalyse: | berechnet: | C 68,98% | H 6,62% | P 6,35% |
| | gefunden: | C 68,80% | H 6,76% | P 6,19%. |

### Verbindung I-32:

### 2-(2-((rac-4,8-Di-tert-butyl-1,2,10,11-tetramethyldibenzo[d,f][1,3,2]dioxaphosphepin-6-yl)oxy)-phen-oxy)phenol

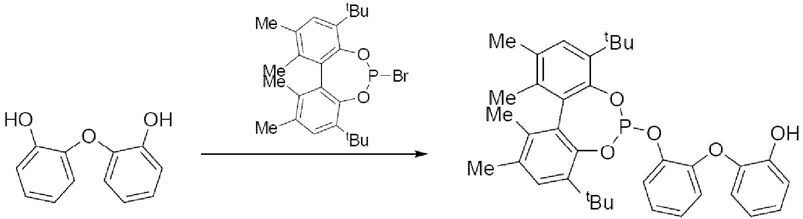

Das *rac*-6-Bromo-4,8-di-*tert*-butyl-1,2,10,11-tetramethyldibenzo[*d*,*f*][1,3,2]dioxa-phosphepin (760 mg, 1,64 mmol) in Toluol (4 ml) wird bei Raumtemperatur langsam zu einer Lösung von 1,5 Äquivalenten 2,2'-Dihydroxydiphenylether (497 mg, 2,46 mmol) und Triethylamin (498 mg, 4,92 mmol) in Toluol (10 ml) getropft. Man lässt über Nacht bei Raumtemperatur weiterrühren und filtriert anschließend vom ausgefallenen Niederschlag ab. Das Rohprodukt enthält trotz des Überschusses an 2,2'-Dihydroxydiphenylether ca. 39 mol-% der di-substituierten Verbindung. Die gewünschte mono-Verbindung wird durch Säulenchromatographie (Eluent: Cyclohexan/AcOEt 99/1 bis 95/5) isoliert.

Ausbeute: 400 mg weißer Feststoff (36%).

Schmelzpunkt: 67-69°C.

³¹P-NMR (CD₂Cl₂): □ +132,4 ppm.

¹H-NMR (CD₂Cl₂): □ 7,21 (1H, s, Hₐᵣ), 7,21 (1H, s, Hₐᵣ), 7,09-6,78 (8H, m, 8xHₐᵣ), 5,66 (br s, -OH), 2,28 (3H, s, CH₃), 2,25 (3H, s, CH₃), 1,84 (6H, s, 2xCH₃), 1,43 (9H, s, C(CH₃)₃), 1,36 (9H, s, C(CH₃)₃).

¹³C-NMR (CD₂Cl₂): □ 148,4 (d, *J* 3,0 Hz, Cₐᵣ), 147,9 (Cₐᵣ), 144,9 (d, *J* 6,8 Hz, Cₐᵣ), 144,6 (Cₐᵣ), 143,7 (Cₐᵣ), 142,7 (d, *J* 1,9 Hz, Cₐᵣ), 138,7 (d, *J* 3,0 Hz, Cₐᵣ), 137,9 (Cₐᵣ), 135,7 (Cₐᵣ), 134,9 (Cₐᵣ), 133,7 (Cₐᵣ), 132,8 (Cₐᵣ), 132,5 (d,J 5,4 Hz, Cₐᵣ), 130,8 (d,J 3,3 Hz, Cₐᵣ), 128,8 (CₐᵣH), 128,4 (CₐᵣH), 125,3 (CₐᵣH), 125,3 (CₐᵣH), 124,5 (CₐᵣH), 123,4 (d,*J* 6,7 Hz, CₐᵣH), 120,8 (CₐᵣH), 119,6 (CₐᵣH), 119,0 (CₐᵣH), 116,7 (CₐᵣH), 35,2 (^{t}C), 34,9 (^{t}C), 31,7 (3xCH₃), 31,3 (d, *J* 5,1 Hz, 3xCH₃), 20,5 (CH₃), 20,5 (CH₃), 16,8 (CH₃), 16,7 (CH₃).

HRMS (ESI-TOF) [M+H]⁺: m/z berechnet: für C₃₆H₄₁O₅P 585,2770, gefunden: 585,2776.

HRMS (ESI-TOF) [M+Na]⁺: m/z berechnet: für C₃₆H₄₁O₅P 607,2584, gefunden: 607,2595.

### Verbindung I-33:

### rac-4,8-Di-tert-butyl-1,2,10,11-tetramethyl-6-(2-(2-((2,4,8,10-tetra-tert-butyldibenzo[d,f][1,3,2]dioxaphos-phepin-6-yl)oxy)phenoxy)phenoxy)dibenzo[d,f][1,3,2]dioxaphosphepine

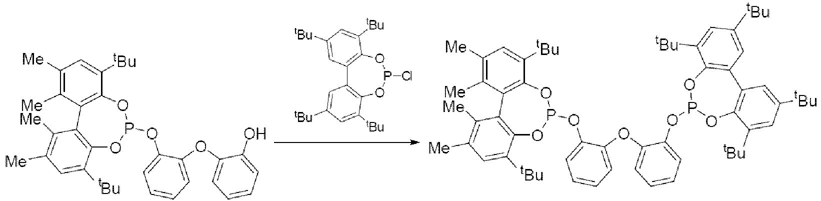

Eine Lösung des 2-(2-((*rac*-*4,8*-Di-*tert-*butyl*-*1,2,10,11-tetramethyldibenzo[*d,f*][1,3,2]dioxaphosphepin-6-yl)oxy)-phenoxy)phenols (175 mg, 0,30 mmol) in Toluol (3 ml) wird zunächst mit Triethylamin (45 mg, 0,45 mmol) und anschließend bei Raumtemperatur mit 2,4,8,10-Tetra-*tert*-butyl-6-chlorodi-benzo[*d,t*][1,3,2]dioxaphosphepine (157 mg, 0,33 mmol) versetzt. Nach dem Rühren über Nacht wird vom ausgefallenen Niederschlag abfiltriert und das Filtrat unter Vakuum eingeengt. Nach der Reinigung mit Säulenchromatographie (Eluent: Cyclohexan/AcOEt 99/1) wird das Produkt in sehr guter Reinheit erhalten.

Ausbeute: 160 mg weißer Feststoff (52%).

Schmelzpunkt: 112-115°C.

³¹P-NMR (CD₂Cl₂): □ +137,1 ppm (d, *J* 13,1 Hz) und +131,2 ppm (d, *J* 13,1 Hz).

¹H-NMR (CD₂Cl₂): □ 7,45 (2H, m, 2xHₐᵣ), 7,21 (2H, d, *J* 2,4 Hz, 2xHₐᵣ), 7,20 (1H, s, Hₐᵣ), 7,16 (1H, s, Hₐᵣ), 7,06-6,90 (6H, m, 6xHₐᵣ), 6,85 (1H, m, Hₐᵣ), 6,82 (1H, m, Hₐᵣ), 2,27 (3H, s, CH₃), 2,26 (3H, s, CH₃), 1,84 (3H, s, CH₃), 1,82 (3H, s, CH₃), 1,37 (9H, s, C(CH₃)₃), 1,36 (18H, s, 2xC(CH₃)₃), 1,36 (9H, s, C(CH₃)₃), 1,34 (9H, s, C(CH₃)₃), 1,33 (9H, s, C(CH₃)₃).

¹³C-NMR (CD₂Cl₂): □ 148,7 (d, *J* 2,9 Hz, Cₐᵣ), 148,6 (d, *J* 2,8 Hz, Cₐᵣ), 147,4 (2xCₐᵣ), 145,8 (d, *J* 6,4 Hz, 2xCₐᵣ), 145,2 (d, *J* 7,0 Hz, Cₐᵣ), 144,9 (Cₐᵣ), 143,2 (Cₐᵣ), 143,0 (Cₐᵣ), 140,9 (2xCₐᵣ), 138,7 (d, *J* 2,7 Hz, Cₐᵣ), 138,1 (Cₐᵣ), 135,5 (Cₐᵣ), 134,7 (Cₐᵣ), 133,3 (Cₐᵣ), 133,2 (m, 2xCₐᵣ), 132,5 (d, *J* 2,5 Hz, Cₐᵣ), 132,4 (Cₐᵣ), 131,1 (d, *J* 2,8 Hz, Cₐᵣ), 128,8 (CₐᵣH), 128,2 (CₐᵣH), 126,9 (CₐᵣH), 126,8 (CₐᵣH), 125,3 (CₐᵣH), 125,1 (CₐᵣH), 124,9 (2xCₐᵣH), 124,2 (CₐᵣH), 124,2 (CₐᵣH), 123,8 (d, *J* 5,7 Hz, CₐᵣH), 123,7 (d, *J* 5,3 Hz, CₐᵣH), 120,4 (CₐᵣH), 120,3 (CₐᵣH), 35,7 (^{t}C), 35,7 (^{t}C), 35,2 (^{t}C), 35,0 (2x^{t}C), 34,9 (^{t}C), 31,7 (3xCH₃), 31,7 (6xCH₃), 31,4 (d, *J* 4,9 Hz, 3xCH₃), 31,3 (3xCH₃), 31,3 (d, *J* 5,6 Hz, 3xCH₃), 20,5 (CH₃), 20,5 (CH₃), 16,9 (CH₃), 16,6 (CH₃).

HRMS (ESI-TOF) [M+H]⁺: m/z berechnet: für C₆₄H₈₀O₇P₂ 1023,5457, gefunden: 1023,5480.

HRMS (ESI-TOF) [M+Na]⁺: m/z berechnet: für C₆₄H₈₀O₇P₂ 1045,5271, gefunden: 1045,5299.

### Verbindung I-34:

### 3,8-Di-tert-butyl-6-(2-(2-((4,8-di-tert-butyl-2,10-diethyl-12H-dibenzo[d,g][1,3,2]dioxaphosphocin-6-yl)oxy)phenoxy)phenoxy)-2,10-diethyl-12H-dibenzo[d,g][1,3,2]dioxaphosphocin

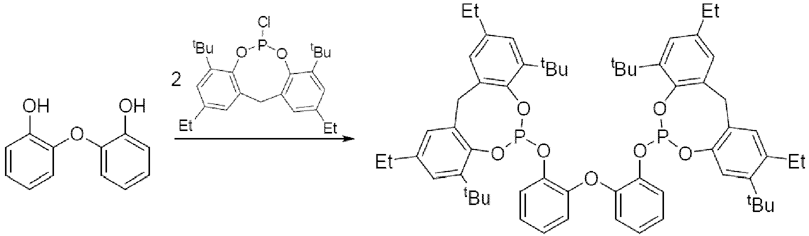

Zu einer Lösung von 2,2'-Dihydroxyphenylether (104 mg, 0,51 mmol) in THF (3 ml) wird bei 0°C zunächst Triethylamin (156 mg, 1,54 mmol) und dann langsam das 4,8-Di-*tert*-butyl-6-chloro-2,10-diethyl-12*H*-di-benzo[d,g][1,3,2]dioxaphosphocin (445 mg, 1,03 mmol) in THF (4 ml) gegeben. Es wird über Nacht bei Raumtemperatur weitergerührt und anschließend vom ausgefallenen Niederschlag abfiltriert. Das Filtrat wird eingeengt und der verbleibende Rückstand aus wenig Acetonitril umkristallisiert und nach der Filtration und Trocknen das Bisphosphit erhalten.

Ausbeute: 290 mg weißer Feststoff (57%).

³¹P-NMR (CD₂Cl₂): □ +132,6 ppm.

¹H-NMR (CD₂Cl₂): □ 7,78 (2H, m, 2xHₐᵣ), 7,17 (2H, d, *J* 2,1 Hz, 4xHₐᵣ), 7,15-7,09 (4H, m, 4xHₐᵣ), 7,08 (4H, d, *J* 2,0 Hz, 4xHₐᵣ), 7,01 (2H, m, 2xHₐᵣ), 4,40 (2H, dd, *J* 13,1, 2,7 Hz, 2xHₐ-CH₂), 3,54 (2H, d, *J* 12,8 Hz, 2xH_{b}-CH₂), 2,61 (8H, q, *J* 7,6 Hz, 4xCH₂), 1,30 (36H, s, 4xC(CH₃)₃), 1,24 (12H, t, *J* 7,6 Hz, 2xCH₃).

¹³C-NMR (CD₂Cl₂): □ 148,3 (d, *J* 3,4 Hz, 2xCₐᵣ), 146,2 (d, *J* 6,3 Hz, 4xCₐᵣ), 143,6 (d, *J* 5,3 Hz, 2xCₐᵣ), 142,6 (d, *J* 3,7 Hz, 4xCₐᵣ), 140,8 (4xCₐᵣ), 136,3 (d, *J* 3,2 Hz, 4xCₐᵣ), 127,6 (4xCₐᵣH), 126,0 (4xCₐᵣH), 125,1 (2xCₐᵣH), 124,2 (2xCₐᵣH), 123,7 (d, *J* 6,3 Hz, 2xCₐᵣH), 120,3 (2xCₐᵣH), 35,7 (2xCH₂), 35,1 (4x^{t}C), 30,9 (d, *J* 3,6 Hz, 12xCH₃), 28,9 (4xCH₂), 16,0 (4xCH₃).

HRMS (ESI-TOF) [M+H]⁺: m/z berechnet: für C₆₂H₇₆O₇P₂ 995,5145, gefunden: 995,5148.

HRMS (ESI-TOF) [M+Na]⁺: m/z berechnet: für C₆₂H₇₆O₇P₂ 1017,4958, gefunden: 1017,4959.

| | | | | |
|---|---|---|---|---|
| Elementaranalyse: | berechnet: | C 74,83% | H 7,70% | P 6,22% |
| | gefunden: | C 74,92% | H 7,65% | P 6,35%. |

### Verbindung I-35:

### 6,6'-((Oxybis(2,1-phenylen))bis(oxy))bis(4,8-di-tert-butyl-1,2,10,11-tetramethyldibenzo[d,f]-[1,3,2]dio-xaphosphepin)

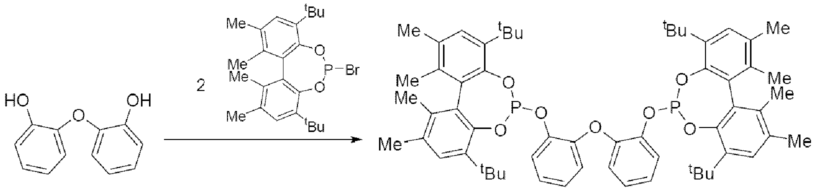

Zu einer Lösung des *rac*-6-Bromo-4,8-di-*tert*-butyl-1,2,10,11-tetramethyldibenzo[*d,f*][1,3,2]dioxaphosphepins (835 mg, 1,80 mmol) in Toluol (7 ml) werden bei Raumtemperatur zunächst Triethylamin (273 mg, 2,70 mmol) und anschließend 2,2'-Oxodiphenol (182 mg, 0,90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 540 mg weißer Feststoff (62%).

Schmelzpunkt: 115-118°C.

³¹P-NMR (CD₂Cl₂): □ +131,5 ppm (62%) und +131,3 ppm (38%), Gemisch aus (*R,R*)/(*S,S*)*-* und (*R*,*S*)-Verbindung.

¹H-NMR (CD₂Cl₂): Diastereomerengemisch, □ 7,20-7,16 (4H, 3xs, 4xHₐᵣ), 7,03-6,89 (6H, m, 6xHₐᵣ), 6,83-6,77 (2H, m, 2xHₐᵣ), 2,29-2,24 (12H, 3xs, 4xCH₃), 1,86-1,81 (12H, 4xs, 4xCH₃), 1,35-1,32 (36H, 4xs, 4xC(CH₃)₃).

¹³C-NMR (CD₂Cl₂): Signale von 146-129 ppm nicht eindeutig zuordbar, 128,8+128,7 (4xCₐᵣH), 128,2 (4xCₐᵣH), 125,2+125,1 (4xCₐᵣH), 124,1 (4xCₐᵣH), 123,7 (4xCₐᵣH), 120,5+120,4 ppm (4xCₐᵣH), 35,2 (2x^{t}C), 35,1 (2x^{t}C), 34,9 (4x^{t}C), 31,8 (6xCH₃), 31,7 (6xCH₃), 31,4 (6xCH₃), 31,4 (6xCH₃), 20,5 (6xCH₃), 20,5 (2xCH₃), 16,9 (2xCH₃), 16,9 (2xCH₃), 16,6 (4xCH₃).

HRMS (ESI-TOF) [M+H]⁺: m/z berechnet: für C₆₀H₇₂O₇P₂ 967,4832, gefunden: 967,4845.

HRMS (ESI-TOF) [M+Na]⁺: m/z berechnet: für C₆₀H₇₂O₇P₂ 989,4645, gefunden: 989,4665.

### Verbindung I-36:

### 4,6-Bis((2,4,8,10-Tetra-tert-butyldibenzo[d,f][1,3,2]dioxaphosphepin-6-yl)oxy)dibenzo[b,d]furan

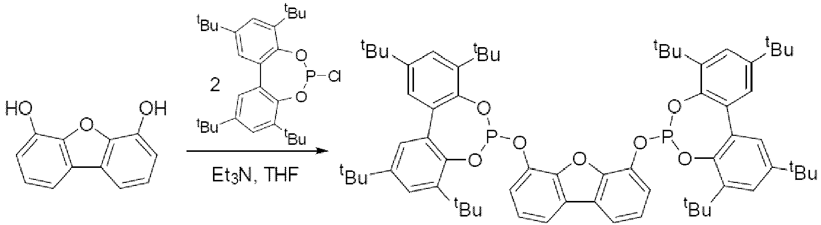

Die Synthese des Dibenzo[*b,d*]furan-4,6-diols kann zum einen nach der Literatur (Y. Sato, R. Yamasaki, S. Saito Angew. Chem. Int. Ed. 2009, 48, 504-507) erfolgen und zum anderen (wie hier der Fall) durch Analogie zur Herstellung des 2,7-Dimethyl-Xanthen-4,5-diols als "Eintopf"-Variante nach C. B. Dielemann, P. C. J. Kamer, J. N. H. Reek, P. W. N. m. van Leeuwen Helv. Chim. Acta 2001, 84, 3269-3280.

Zu einer Lösung von Dibenzo[*b,d*]furan-4,6-diol (111 mg, 0,56 mmol) und Triethylamin (169 mg, 1,67 mmol) in THF (2 ml) tropft man unter Rühren bei 0°C (Eisbad) eine Lösung von 2,4,8,10-Tetra-tert-butyl-6-chlorodibenzo[*d,f*][1,3,2]dioxaphosphepin (532 mg, 1,12 mmol) in THF (6 ml) hinzu. Man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand in Acetonitril (7 ml) aufgenommen und bei Siedehitze gelöst. Der beim Abkühlen rasch ausgefallene Niederschlag wird abgetrennt und unter Vakuum bei 60°C getrocknet.

Ausbeute: 330 mg weißer Feststoff (54%).

³¹P-NMR (CD₂Cl₂): □ +136,7 ppm.

¹H-NMR (CD₂Cl₂): □ 7,66 (2H, dd, *J* 7,7, 0,7 Hz, 2xHₐᵣ), 7,51 (4H, d, *J* 2,5 Hz, 4xHₐᵣ), 7,24 (4H, d, *J* 2,5 Hz, 4xHₐᵣ), 7,13 (2H, t, *J* 7,9 Hz, 2xHₐᵣ), 6,86 (2H, m,2xHₐᵣ), 1,47 (36H, s, 12xCH₃) 1,40 (36H, s, 4xC(CH₃)₃).

¹³C-NMR (CD₂Cl₂): □ 147,6 (4xCₐᵣ), 145,8 (d, *J* 5,8 Hz, 2xCₐᵣ), 140,9 (4xCₐᵣ), 137,8 (2xCₐᵣ), 133,2 (d, *J* 3,7 Hz, 4xCₐᵣ), 127,0 (4xCₐᵣH), 126,9 (2xCₐᵣ), 125,0 (4xCₐᵣH), 123,8 (2xCₐᵣH), 120,1 (d, *J* 7,5 Hz, 2xCₐᵣH), 116,6 (2xCₐᵣH), 35,8 (4x^{t}C), 35,1 (4x^{t}C), 31,7 (12xCH₃), 31,4 (12xCH₃).

HRMS (ESI-TOF) [M+H]⁺: m/z berechnet: für C₆₈H₈₆O₁₁P₂ 1077,5927, gefunden: 1077,5924.

HRMS (ESI-TOF) [M+Na]⁺: m/z berechnet: für C₆₈H₈₆O₁₁P₂ 1099,5741, gefunden: 1099,5741.

| | | | | |
|---|---|---|---|---|
| Elementaranalyse: | berechnet: | C 75,81% | H 8,05% | P 5,75% |
| | gefunden: | C 75,19% | H 8,08% | P 5,52%. |

### Verbindung I-37:

### 4,6-Bis((4,8-di-tert-butyl-2,10-dimethoxydibenzo[d,f][1,3,2]dioxaphosphepin-6-yl)oxy)di-benzo[b,d]furan

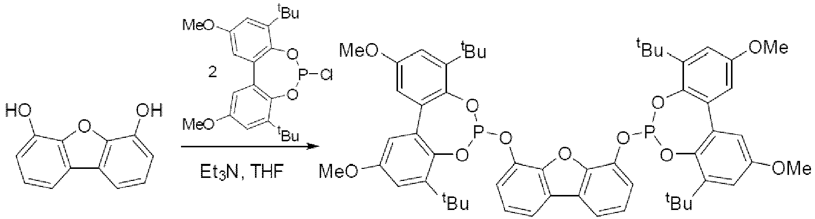

Zu einer Lösung von Dibenzo[b*,d*]furan-4,6-diol (124 mg, 0,62 mmol) und Triethylamin (188 mg, 1,86 mmol) in THF (2 ml) tropft man unter Rühren bei 0°C (Eisbad) eine Lösung von 4,8-Di-*tert*-butyl-6-chloro-2,10-dimethoxydibenzo[*d,f*][1,3,2]dioxaphosphepin (524 mg, 1,24 mmol) in THF (5 ml) hinzu. Man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand durch Säulenchromatographie (Eluent: Heptan/CH₂Cl₂ 1/4) vorgereinigt und anschließend das isolierte Produkt mit heißem Heptan (4 ml) verrührt und nach dem Abkühlen der verbleibende Niederschlag abgetrennt. Dieser wird unter Vakuum bei 60°C getrocknet.

Ausbeute: 280 mg weißer Feststoff (46%).

³¹P-NMR (CD₂Cl₂): □ +137,1 ppm.

¹H-NMR (CD₂Cl₂): □ 7,68 (2H, dd, *J* 7,6, 1,0 Hz, 2xHₐᵣ), 7,20 (2H, t, *J* 7,9 Hz, 2xHₐᵣ), 7,04 (2H, m,2xHₐᵣ), 7,01 (4H, d, *J* 3,2 Hz, 4xHₐᵣ), 6,77 (2H, d, *J* 3,1 Hz, 4xHₐᵣ), 3,82 (12H, s,4xOCH₃) 1,42 (36H, s, 4xC(CH₃)₃).

¹³C-NMR (CD₂Cl₂): □ 156,5 (4xCₐᵣ), 147,5 (d, *J* 2,8 Hz, 2xCₐᵣ), 143,3 (4xCₐᵣ), 141,6 (d, *J* 6,0 Hz, 2xCₐᵣ), 137,9 (4xCₐᵣ), 134,1 (d, *J* 3,9 Hz, 4xCₐᵣ-O), 127,0 (2xCₐᵣ), 123,9 (2xCₐᵣH), 119,8 (d, *J* 6,6 Hz, 2xCₐᵣH), 116,6 (2xCₐᵣH), 114,8 (4xCₐᵣH), 113,3 (4xCₐᵣH), 56,0 (4xOCH₃), 35,8 (4x^{t}C), 31,2 (12xCH₃).

| | | | | |
|---|---|---|---|---|
| Elementaranalyse: | berechnet: | C 69,12% | H 6,42% | P 6,37% |
| | gefunden: | C 69,04% | H 6,64% | P 6,24%. |

### Verbindung I-38:

### 4,6-Bis(((4R,5R)-4,5-diphenyl-1,3,2-dioxaphospholan-2-yl)oxy)dibenzo[b,d]furan

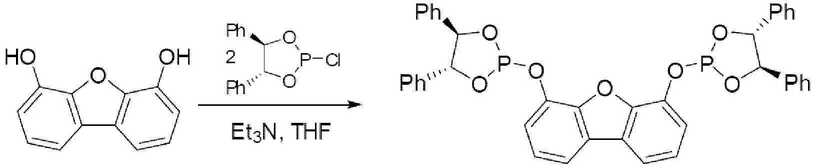

**Zu einer** Lösung von Dibenzo[*b,d*]furan-4,6-diol (214 mg, 1,07 mmol) und Triethylamin (325 mg, 3,21 mmol) in THF (2 ml) tropft man unter Rühren bei 0°C (Eisbad) eine Lösung von (4*R*,5*R*)-2-chloro-4,5-diphenyl-1,3,2-dioxaphospholan (597 mg, 2,14 mmol) in THF (6 ml) hinzu. man lässt über Nacht bei Raumtemperatur rühren und entfernt anschließend den ausgefallenen Niederschlag durch Filtration unter anaeroben Bedingungen. Nach Einengen unter Vakuum wird der verbleibende Rückstand durch Säulenchromatographie (Eluent: CH₂Cl₂) gereinigt und nach dem Entfernen des Lösemittels unter Vakuum der erhaltenen Feststoff unter Vakuum bei 60°C getrocknet.

Ausbeute: 325 mg weißer Feststoff (44%).

³¹P-NMR (CD₂Cl₂): □ +135,5 ppm.

¹H-NMR (CD₂Cl₂): □ 7,79 (2H, ddd, *J* 6,7, 2,1, 0,4 Hz, 2xHₐᵣ), 7,43-7,20 (24H, m, 24xHₐᵣ), 5,48 (2H, d, *J* 9,5 Hz, 2xCH-O), 4,99 (2H, dd, *J* 9,5, 2,8 Hz, 2xCH-O).

¹³C-NMR (CD₂Cl₂): □ 147,8 (d, *J* 2,7 Hz, 2xCₐᵣ), 137,9 (d, *J* 6,1 Hz, 2xCₐᵣ), 136,7 (4xCₐᵣ), 136,2 (d, *J* 6,1 Hz, 2xCₐᵣ), 129,7 (2xCₐᵣH), 129,1 (4xCₐᵣH), 129,0 (2xCₐᵣH), 129,0 (4xCₐᵣH), 127,9 (4xCₐᵣH), 127,4 (4xCₐᵣH), 127,2 (2xCₐᵣ), 124,2 (2xCₐᵣH), 119,4 (d, *J* 10,0 Hz, 2xCₐᵣH), 116,6 (2xCₐᵣH), 87,8 (d, *J* 7,9 Hz, 2xCH-O), 84,4 (d, *J* 6,8 Hz, 2xCH-O).

HRMS (ESI-TOF) [M+H]⁺: m/z berechnet: für C₄₀H₃₀O₇P₂ 685,1545, gefunden: 685,1552.

HRMS (ESI-TOF) [M+Na]⁺: m/z berechnet: für C₄₀H₃₀O₇P₂ 707,1359, gefunden: 707,1371.

| | | | | |
|---|---|---|---|---|
| Elementaranalyse: | berechnet: | C 70,18% | H 4,42% | P 9,05% |
| | gefunden: | C 70,17% | H 4,58% | P 9,40%. |

### Verbindung I-39:

### 4,6-Bis((rac-4,8-di-tert-butyl-1,2,10,11-tetramethyldibenzo[d,f][1,3,2]dioxaphosphepin-6-yl)oxy)di-benzo[b,d]furan

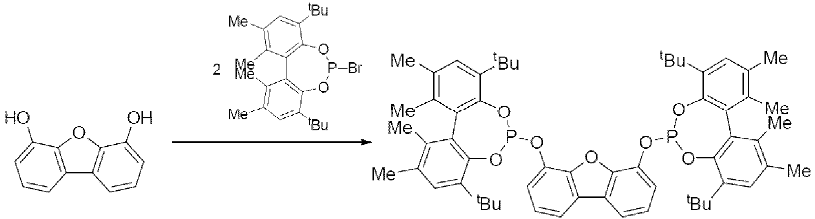

Entsprechend der Literatur (C. J. Cobley et al. J. Org. Chem. 2004, 69, 4031-4040) wurde das *rac*-6-Bromo-4,8-di-*tert*-butyl-1,2,10,11-tetramethyldibenzo[*d,f*][1,3,2]dioxaphosphepin hergestellt und als Rohprodukt direkt für den nächsten Reaktionsschritt verwendet (³¹P-NMR (CD₂Cl₂) □_{P} +179,6 ppm).

Zu einer Lösung des *rac*-6-Bromo-4,8-di-*tert*-butyl-1,2,10,11-tetramethyldibenzo[*d,f*][1,3,2]dioxa-phosphe-pins (835 mg, 1,80 mmol) in Toluol (7 ml) werden bei Raumtemperatur zunächst Triethylamin (273 mg, 2,70 mmol) und anschließend Dibenzo[b,d]furan-4,6-diol (180 mg, 0,90 mmol) gegeben. Nach dem Rühren über Nacht wird vom ausgefallenen Niederschlag abfiltriert und das Filtrat unter Vakuum eingeengt. Der verbleibende Rückstand wird durch Säulenchromatographie (Eluent: Cyclohexan/AcOEt 99/1) gereinigt.

Ausbeute: 780 mg weißer Feststoff (90%).

Schmelzpunkt: 135-140°C.

³¹P-NMR (CD₂Cl₂): □ +130,5 ppm (49%) und +130,3 ppm (51%), Gemisch aus (*R,R*)/(*S,S*)*-* und (*R*,*S*)-Verbindung.

¹H-NMR (CD₂Cl₂): ca. 1/1-Diastereomerengemisch; 7,64 (2H, dd, *J* 7,8, 0,9 Hz, 2xHₐᵣ), 7,25+7,24 (2H, s, 2xHₐᵣ), 7,18+7,18 (2H, s, 2xHₐᵣ), 7,13 (2H, dt, *J* 7,9, 2,9 Hz, 2xHₐᵣ), 6,85 (2H, m, 2xHₐᵣ), 2,30 +2,29 + 2,28 (12H, 3xs, 4xCH₃), 1,85+1,84+1,84 (12H, 3xs, 4xCH₃), 1,46+1,46+1,34+1,32 (36H, 4xs, 4xC(CH₃)₃).

¹³C-NMR (CD₂Cl₂): ca. 1/1-Diastereomerengemisch; 147,5+147,4 (4xCₐᵣ), 145,2+145,1 (4xCₐᵣ), 144,9 (4xCₐᵣ), 137,8-131,0 (restliche Cₐᵣ-Signale), 128,9+128,8 (4xCₐᵣH), 128,3 (4xCHₐᵣ), 126,9+126,9 (2xCₐᵣ), 123,7+123,6 (4xCHₐᵣ), 119,9+119,8 (4xCHₐᵣ), 116,4+116,3 (4xCHₐᵣ), 35,2 (2x^{t}C), 35,2 (2x^{t}C), 34,9 (2x^{t}C), 34,9 (2x^{t}C), 31,8 (12xCH₃), 31,3 (6xCH₃), 31,3 (6xCH₃), 20,5 (8xCH₃), 16,9 (2xCH₃), 16,8 (2xCH₃), 16,6 (4xCH₃).

HRMS (ESI-TOF) [M+H]⁺: m/z berechnet: für C₆₀H₇₀O₇P₂ 965,4675, gefunden: 965,4683.

HRMS (ESI-TOF) [M+Na]⁺: m/z berechnet: für C₆₀H₇₀O₇P₂ 987,4489, gefunden: 987,4501.

### Verbindung I-40:

### 6,6'-((Cyclohexan-1,1-diylbis(2-methyl-4,1-phenylen))bis(oxy))bis(4,8-di-tert-butyl-2,10-dimethoxy-di-benzo[d,f][1,3,2]dioxaphosphepin)

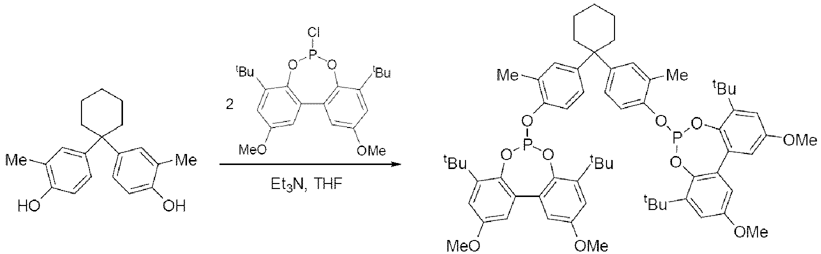

**Zu einer** Lösung von 4,4'-(Cyclohexan-1,1-diyl)bis(2-methylphenol) (257 mg, 0,87 mmol) und Triethylamin (263 mg, 2,60 mmol) in THF (3 ml) wird unter Rühren und bei 0°C langsam das 4,8-Di-*tert-*butyl-6-chloro-2,10-dimethoxydibenzo[*d,f*][1,3,2]dioxaphosphepin (770 mg, 1,82 mmol) in THF (6 ml) zugetropft. Zur Vervollständigung der Reaktion lässt man über Nacht bei Raumtemperatur weiterrühren und filtriert im nächsten Schritt vom ausgefallenen Hydrochlorid ab. Nach dem Entfernen des Lösemittels fällt das Produkt in praktisch reiner Form an und wurde nicht weiter gereinigt.

Ausbeute: 780 mg weißer Feststoff (84%).

³¹P-NMR (CD₂Cl₂): □ +138,8 ppm.

¹H-NMR (CD₂Cl₂): □ 7,11 (2H, m, 2xHₐᵣ), 7,03 (2H, m, 2xHₐᵣ), 7,02 (2H, d, *J* 3,3 Hz, 2xHₐᵣ), 6,97 (2H, m, 2xHₐᵣ), 6,78 (2H, d, *J* 3,3 Hz, 2xHₐᵣ), 3,83 (12H, s, 4xOCH₃), 2,23 (4H, m, 2xCH₂), 2,20 (6H, s, 2xCH₃), 1,59-1,46 (6H, m, 3xCH₂), 1,42 (36H, s, 12xCH₃).

¹³C-NMR (CD₂Cl₂): □ 156,3 (4xCₐᵣ-OMe), 148,1 (d, *J* 3,4 Hz, 2xCₐᵣ), 144,5 (2xCₐᵣ), 143,1 (d, *J* 1,3 Hz, 4xCₐᵣ), 141,8 (d, *J* 6,2 Hz, 4xCₐᵣ), 134,1 (d, *J* 3,9 Hz, 4xCₐᵣ), 130,4 (2xCₐᵣH), 129,8 (d, *J* 2,3 Hz, 2xCₐᵣ), 125,8 (2xCₐᵣH), 120,7 (d, *J* 10,7 Hz, 2xCₐᵣH), 114,8 (4xCₐᵣH, 113,3 (4xCₐᵣH), 56,0 (4xOCH₃), 45,6 (^{t}C), 37,6 (2xCH₂), 35,7 (4x^{t}C), 31,2 (6xCH₃), 31,1 (6xCH₃), 26,8 (CH₂), 23,3 (2xCH₂), 17,3 (2xCH₃).

HRMS (ESI-TOF) [M+H]⁺: *m*/*z* berechnet: für C₆₄H₇₈O₁₀P₂ 1069,5149, gefunden 1069,5156.

HRMS (ESI-TOF) [M+Na]⁺: *m*/*z* berechnet: für C₆₄H₇₈O₁₀P₂ 1091,4963, gefunden 1091,4977.

| | | | | |
|---|---|---|---|---|
| Elementaranalyse: | berechnet: | C 71,89% | H 7,35% | P 5,79% |
| | gefunden: | C 71,89% | H 7,47% | P 5,81%. |

### Verbindung I-41:

### 2,2'-((Cyclohexan-1,1-diylbis(2-methyl-4,1-phenylen))bis(oxy))bis(4,5-diphenyl-1,3,2-dioxaphosphol)

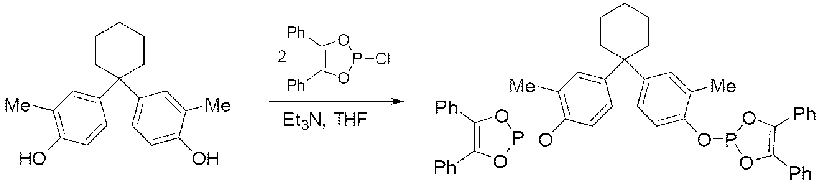

**Zu einer** Lösung von 4,4'-(Cyclohexan-1,1-diyl)bis(2-methylphenol) (350 mg, 1,18 mmol) und Triethylamin (358 mg, 3,54 mmol) in **THF** (3 ml) wird unter Rühren und bei 0°C langsam das 2-Chlor-4,5-diphenyl-1,3,2-dioxaphosphol (686 mg, 2,48 mmol) in **THF** (4 ml) zugetropft. Zur Vervollständigung der Reaktion lässt man über Nacht bei Raumtemperatur weiterrühren und filtriert im nächsten Schritt vom ausgefallenen Hydrochlorid ab. Nach dem Entfernen des Lösemittels wird der verbleibende Rückstand aus Acetonitril kristallisiert. Der zunächst anfallende viskose Rückstand kristallisiert beim Trocknen durch.

Ausbeute: 650 mg weißer Feststoff (75%).

³¹P-NMR (CD₂Cl₂): □ +116,9 ppm.

¹H-NMR (CD₂Cl₂): □ 7,30-7,14 (20H, m, 20xHₐᵣ), 6,95 (2H, br d, *J* 2,2 Hz, 2xHₐᵣ), 6,74 (2H, d, *J* 8,4 Hz, 2xHₐᵣ), 6,67 (2H, dd, *J* 8,4, 2,3 Hz, 2xHₐᵣ), 2,15 (6H, s, 2xCH₃), 2,09-1,99 (4H, m, 2xCH₂), 1,40-1,27 (6H, m, 3xCH₂).

¹³C-NMR (CD₂Cl₂): □ 147,6 (d, *J* 5,7 Hz, 2xCₐᵣ), 145,3 (2xCₐᵣ), 135,3 (d, *J* 7,7 Hz, 4xO-C=), 129,8 (2xCₐᵣH), 129,6 (d, *J* 1,4 Hz, 4xCₐᵣ), 128,9 (d, *J* 1,5 Hz, 2xCₐᵣ), 128,8 (4xCₐᵣH), 128,7 (8xCₐᵣH), 127,6 (8xCₐᵣH), 126,0 (2xCₐᵣH), 121,2 (d, *J* 4,7 Hz, 2xCₐᵣH), 45,3 (^{t}C), 37,4 (2xCH₂), 26,6 (CH₂), 23,1 (2xCH₂), 17,3 (2xCH₃).

HRMS (ESI-TOF) [M+H]⁺: *m*/*z* berechnet: für C₄₈H₄₂O₆P₂ 777,2535, gefunden: 777,2548.

HRMS (ESI-TOF) [M+Na]⁺: *m*/*z* berechnet: für C₄₈H₄₂O₆P₂ 799,2349, gefunden: 799,2371.

| | | | | |
|---|---|---|---|---|
| Elementaranalyse: | berechnet: | C 74,22% | H 5,45% | P 7,97% |
| | gefunden: | C 74,18% | H 5,50% | P 8,03%. |

### Verbindung I-42:

### 2,2'-((Cyclohexan-1,1-diylbis(2-methyl-4,1-phenylen))bis(oxy))bis(5,5-dimethyl-1,3,2-dioxaphosphinan)

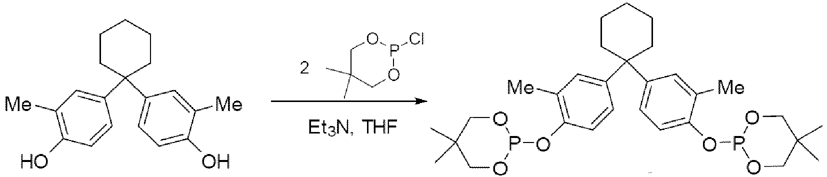

**Zu einer** Lösung von 4,4'-(Cyclohexan-1,1-diyl)bis(2-methylphenol) (389 mg, 1,31 mmol) und Triethylamin (398 mg, 3,93 mmol) in THF (3 ml) wird unter Rühren und bei 0°C das 2-Chlor-5,5-dimethyl-1,3,2-dio-xaphosphinan (464 mg, 2,75 mmol) in THF (3 ml) langsam zugetropft. Zur Vervollständigung der Reaktion lässt man über Nacht bei Raumtemperatur weiterrühren und filtriert im nächsten Schritt vom ausgefallenen Hydrochlorid ab. Nach dem Entfernen des Lösemittels und Trocknen des festen Rückstandes wird das Bisphosphit praktisch rein erhalten und bedarf keiner weiteren Reinigung.

Ausbeute: 675 mg weißer Feststoff (92%).

³¹P-NMR (CD₂Cl₂): □ +115,2 ppm.

¹H-NMR (CD₂Cl₂): □ 7,15 (2H, m, 2xHₐᵣ), 7,07 (2H, m, 2xHₐᵣ), 6,98 (2H, m, 2xHₐᵣ), 4,35 (4H, m, 4xHₐ-CH₂O), 3,45 (4H, m, 4xH_{b}-CH₂O), 2,27-2,21 (4H, m, 2xCH₂), 2,27 (6H, s, 2xCH₃), 1,63-1,44 (6H, m, 3xCH₂), 1,31 (6H, s, 2xCH₃), 0,79 (6H, s, 2xCH₃).

³¹C-NMR (CD₂Cl₂): □ 149,1 (d, *J* 6,6 Hz, 2xCₐᵣ), 144,0 (2xCₐᵣ), 130,1 (2xCₐᵣH), 129,3 (d, *J* 2,3 Hz, 2xCₐᵣ), 125,8 (2xCₐᵣH), 118,9 (d, *J* 12,9 Hz, 2xCₐᵣH), 69,7 (d, *J* 2,4 Hz, 4xCH₂O), 45,4 (^{t}C), 37,5 (2xCH₂), 33,1 (d, *J* 5,1 Hz, 2x^{t}C), 26,8 (CH₂), 23,4 (2xCH₂), 22,9 (2xCH₃), 22,6 (2xCH₃), 17,3 (2xCH₃).

HRMS (ESI-TOF) [M+H]⁺: *m*/*z* berechnet: für C₃₀H₄₂O₆P₂ 561,2535, gefunden: 561,2539.

HRMS (ESI-TOF) [M+Na]⁺: *m*/*z* berechnet: für C₃₀H₄₂O₆P₂ 583,2349, gefunden: 583,2360.

| | | | | |
|---|---|---|---|---|
| Elementaranalyse: | berechnet: | C 64,27% | H 7,55% | P 11,05% |
| | gefunden: | C 64,28% | H 7,52% | P 11,17%. |

### Verbindung I-43:

### (4R,4'R,5R,5'R)-2,2'-((Cyclohexan-1,1-diylbis(2-methyl-4,1-phenylen))bis(oxy))bis(4,5-diphenyl-1,3,2-dio-xaphospholan)

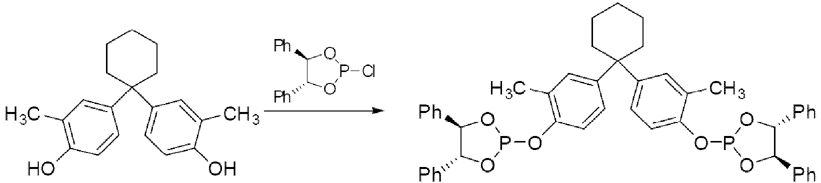

Zu einer Lösung von 4,4'-(Cyclohexan-1,1-diyl)bis(2-methylphenol) (221 mg, 0,75 mmol) und Triethylamin (226 mg, 2,24 mmol) in THF (3 ml) wird unter Rühren und bei 0°C das 2-Chlor-5,5-dimethyl-1,3,2-dio-xaphosphinan (437 mg, 1,56 mmol) in THF (3 ml) langsam zugetropft. Zur Vervollständigung der Reaktion lässt man über Nacht bei Raumtemperatur weiterrühren und filtriert im anschließend vom ausgefallenen Hydrochlorid ab. Nach dem Entfernen des Lösemittels und Trocknen des festen Rückstandes wird das Bisphosphit praktisch rein erhalten und nicht weiter gereinigt.

Ausbeute: 475 mg weißer Feststoff (81%).

³¹P-NMR (CD₂Cl₂): □ +134,1 ppm.

¹H-NMR (CD₂Cl₂): □ 7,42-7,29 (6H, m, 16xHₐᵣ), 7,29-7,22 (6H, m, 6xHₐᵣ), 7,15 (2H, dd, *J* 8,5, 2,3 Hz, 2xHₐᵣ), 7,09 (2H, d, *J* 8,4 Hz, 2xHₐᵣ), 5,26 (2H, d, *J* 9,4 Hz, 2xCH-O), 4,95 (2H, dd, *J* 9,5, 2,7 Hz, 2xCH-O), 2,34 (6H, s, 2xCH₃), 2,34-2,26 (4H, m, 2xCH₂), 1,68-1,48 (6H, m, 3xCH₂).

³¹C-NMR (CD₂Cl₂): □ 148,4 (d, *J* 4,3 Hz, 2xCₐᵣ), 145,0 (2xCₐᵣ), 136,9 (d, *J* 7,7 Hz, 2xCₐᵣ), 136,4 (d, *J* 5.7 Hz, 2xCₐᵣ), 130,3 (2xCₐᵣH), 129,9 (d, *J* 2,3 Hz, 2xCₐᵣ), 129,2 (2xCₐᵣH), 129,1 (4xCₐᵣH), 129,1 (2xCₐᵣH), 129,0 (4xCₐᵣH), 127,9 (4xCₐᵣH), 127,4 (4xCₐᵣH), 125,9 (2xCₐᵣH), 120,5 (d, *J* 10,9 Hz, 2xCₐᵣH), 87,5 (d, *J* 8,0 Hz, 2xCH-O), 84,0 (d, *J* 6,7 Hz, 2xCH-O), 45,6 (^{t}C), 37,7 (2xCH₂), 26,8 (CH₂), 23,4 (2xCH₂), 17,3 (2xCH₃).

HRMS (ESI-TOF) [M+H]⁺: *m*/*z* berechnet: für C₄₈H₄₆O₆P₂ 781,2848, gefunden: 781,2845.

HRMS (ESI-TOF) [M+Na]⁺: *m*/*z* berechnet: für C₄₈H₄₆O₆P₂ 803,2662, gefunden: 803,2665.

| | | | | |
|---|---|---|---|---|
| Elementaranalyse: | berechnet: | C 73,83% | H 5,94% | P 7,93% |
| | gefunden: | C 73,67% H | 5,82% | P 7,73%. |

### Verbindung I-44:

### 4,4'-((Cyclohexan-1,1-diylbis(2-methyl-4,1-phenylen))bis(oxy))didinaphtho[2,1-d:1',2'-f][1,3,2]dioxaphos-phepin

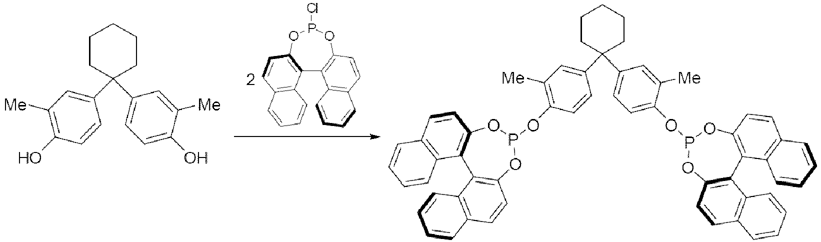

Zu einer Lösung von 4,4'-(Cyclohexan-1,1-diyl)bis(2-methylphenol) (215 mg, 0,73 mmol) und Triethylamin (220 mg, 2,18 mmol) in THF (3 ml) wird unter Rühren und bei 0°C das (*R*)-2-Chlor-5,5-dimethyl-1,3,2-dio-xaphosphinan (534 mg, 1,52 mmol) in THF (5 ml) langsam zugetropft. Zur Vervollständigung der Reaktion lässt man über Nacht bei Raumtemperatur weiterrühren und filtriert im anschließend vom ausgefallenen Hydrochlorid ab. Nach dem Entfernen des Lösemittels und Trocknen des festen Rückstandes wird das Bisphosphit praktisch rein erhalten.

Ausbeute: 530 mg weißer Feststoff (79%).

³¹P-NMR (CD₂Cl₂): □ +144,6 ppm.

¹H-NMR (CD₂Cl₂): □ 8,06 (2H, br d, *J* 8,8 Hz, 2xHₐᵣ), 8,02-7,94 (6H, m, 6xHₐᵣ), 7,64 (2H, dd, *J* 8,8, 0,8 Hz, 2xHₐᵣ), 7,54-7,39 (10H, m, 10xHₐᵣ), 7,35-7,27 (4H, m, 4xHₐᵣ), 7,26-7,20 (4H, m, 4xHₐᵣ), 7,14 (2H, dd, *J* 8,5, 2,3 Hz, 2xHₐᵣ), 2,36-2,24 (4H, m, 2xCH₂), 2,34 (6H, s, 2xCH₃), 1,71-1,50 (6H, s, 3xCH₂).

³¹C-NMR (CD₂Cl₂): □ 148,3 (d, *J* 7,5 Hz, 2xCₐᵣ), 148,2 (d, *J* 5,3 Hz, 2xCₐᵣ), 147,5 (d, *J* 2,3 Hz, 2xCₐᵣ), 145,2 (2xCₐᵣ), 133,2 (d, *J* 1,2 Hz, 2xCₐᵣ), 132,9 (d, *J* 1,2 Hz, 2xCₐᵣ), 132,2 (2xCₐᵣ), 131,7 (2xCₐᵣ), 131,0 (2xCₐᵣH), 130,4 (2xCₐᵣH), 130,3 (2xCₐᵣH), 129,6 (d, *J* 2,7 Hz, 2xCₐᵣ), 128,9 (2xCₐᵣH), 128,8 (2xCₐᵣH), 127,2 (2xCₐᵣH), 127,1 (2xCₐᵣH), 126,8 (2xCₐᵣH), 126,7 (2xCₐᵣH), 126,0 (2xCₐᵣH), 125,7 (2xCₐᵣH), 125,5 (2xCₐᵣH), 124,7 (d, *J* 5,3 Hz, 2xCₐᵣ), 123,2 (d, *J* 2,7 Hz, 2xCₐᵣ), 122,1 (2xCₐᵣH), 122,1 (2xCₐᵣH), 120,0 (d, *J* 10,7 Hz, 2xCₐᵣH), 45,6 (^{t}C), 37,5 (2xCH₂), 26,8 (CH₂), 23,3 (2xCH₂), 17,3 (2xCH₃).

HRMS (ESI-TOF) [M+H]⁺: *m*/*z* berechnet: für C₄₈H₄₆O₆P₂ 781,2848, gefunden: 781,2845.

HRMS (ESI-TOF) [M+Na]⁺: *m*/*z* berechnet: für C₄₈H₄₆O₆P₂ 803,2662, gefunden: 803,2665.

| | | | | |
|---|---|---|---|---|
| Elementaranalyse: | berechnet: | C 73,83% | H 5,94% | P 7,93% |
| | gefunden: | C 73,67% | H 5,82% | P 7,73%. |

### 2) Einsatz erfindungsgemäßer Verbindungen und Substanzen in Hydroformylierungsreaktionen

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung des Einflusses von Feuchtigkeit und Sauerstoff wurde das als Solvens benutzte Toluol in einem Pure Solv. MD-7 System gereinigt und unter Argon aufbewahrt. Cis/trans-2-Penten (Aldrich) wurde über Natrium am Rückfluss erhitzt und unter Argon destilliert. Im Autoklav wurden unter Argonatmosphäre Lösungen der Katalysatorvorstufe (Metallprecursor) und der erfindungsgemäßen Verbindungen als Liganden jeweils in Toluol gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischung wurden entnommen, mit 10 ml Pentan verdünnt und mittels Gaschromatographie analysiert: HP 5890 Series II plus, PONA, 50 m x 0,2 mm x 0,5 µm.

Die in den nachstehenden Tabellen dargestellten Ergebnisse der Hydroformylierung wurden falls nicht anders angegeben unter folgenden Bedingungen / Parametern erhalten:
- eingesetztes Olefin: 2-Penten (2,41 M),
- Temperatur: 120°C,
- Reaktionszeit: 4 h,
- Druck: 20 bar,
- Zusammensetzung des Synthesegases: CO/H₂ (1:1),
- Lösungsmittel: Toluol,
- eingesetzte Menge an Rhodium in Form von [(acac)Rh(COD)] bezogen auf die eingesetzte Menge an 2-Penten: µmol/mol,
- molares Verhältnis von Rhodiumatomen zu eingesetzter Verbindung 1:2.

### 2.1) Hydroformylierung mit erfindungsgemäßen Verbindungen 1-1 bis 1-22 - Phosphite mit einem Methylen-verbrücktem Rückgrat, insbesondere mit einem Diphenylmethan-verbrücktem Rückgrat:

**Tabelle 1. Ergebnisse erfindungsgemäßer Verbindungen I-1 bis I-22 in der Hydroformylierung von 2-Penten**

| Verbindung | Umsatz (%) | n-Selektivität (%) |
|---|---|---|
| I-1 | 94 | 35 |
| I-2 | 99 | 43 |
| I-3 | 98 | 49 |
| I-4 | 98 | 41 |
| I-6 | 87 | 42 |
| I-6^{a} | 85 | 32 |
| I-7 | 1 | - |
| I-9 | 19 | 40 |
| I-10 | 60 | 40 |
| I-11 | 82 | 60 |
| I-12^{b} | 13 | 40 |
| I-13 | 2 | 43 |
| I-16 | 28 | 50 |
| I-17 | 94 | 45 |
| I-18 | 99 | 51 |
| I-19 | >99 | 48 |
| I-20 | 99 | 48 |
| I-21 | 66 | 65 |
| I-22 | 89 | 47 |
| I-22^{a} | 84 | 30 |

| | | |
|---|---|---|
| ^{a} Substrat: n-Octen-Gemisch, T: 120°C, t: 4 h, p: 20 bar, CO/H₂ (1:1), Solvens: Toluol, 100 ppm Rh, [Olefin]= 2,35 M, Rh/Ligand: 1/2. ^{b}Rh/L = 1/5 | | |

Anhand der in Tabelle 1 dargestellten Ergebnisse konnte gezeigt werden, dass die erfindungsgemäßen Verbindungen I-1 bis I-22 gemäß der ersten bevorzugten Ausgestaltung erfindungsgemäßer Verbindungen, d.h. Phosphite mit einem Methylen-verbrücktem Rückgrat, insbesondere mit einem Diphenylmethan-verbrücktem Rückgrat, und entsprechende Substanzen sowie auch das erfindungsgemäße Verfahren in der Hydroformylierung von Olefinen, hier beispielhaft gezeigt an dem Substrat 2-Penten bzw. einem Octen-Gemisch, hervorragende Resultate zeigen.

Die eingesetzten erfindungsgemäßen Verbindungen I-1 bis I-22 zeigten gute Umsätze von bis zu >99% bei der Hydroformylierung von 2-Penten in die entsprechenden Aldehyde.

Hierbei zeigte sich auch, dass die katalytische Aktivität der eingesetzten Verbindungen I-1 bis I-22 in der Hydroformylierung insbesondere in Kombination mit Rhodium-Metallzentren besonders effektiv ist.

Des Weiteren zeigen die Ergebnisse, dass die erfindungsgemäßen Verbindungen I-1 bis I-22 ebenfalls dazu in der Lage sind, gute bis exzellente Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier beispielhaft untersuchten Substrat 2-Penten, eine besonders schwer einzustellende Größe ist. Folglich sind die mit den erfindungsgemäßen Verbindungen I-1 bis I-22 erzielten n-Selektivitäten von bis zu 65% sehr gute Resultate.

### 2.2) Hydroformylierung mit erfindungsgemäßen Verbindungen 1-23 bis 1-26 - Phosphite mit einem Hydroxyphenylmethanon-Rückgrat:

**Tabelle 2. Ergebnisse erfindungsgemäßer Verbindungen I-23 bis I-26 in der Hydroformylierung von 2-Penten**

| Verbindung | Umsatz (%) | n-Selektivität (%) |
|---|---|---|
| I-23 | 59,4 | 43,2 |
| I-24 | 90,9 | 42,2 |
| I-25 | 89,5 | 42,1 |
| I-26 | 99,0 | 44,7 |

Anhand der in Tabelle 2 dargestellten Ergebnisse konnte gezeigt werden, dass die erfindungsgemäßen Verbindungen I-23 bis I-26 gemäß der zweiten bevorzugten Ausgestaltung erfindungsgemäßer Verbindungen, d.h. Phosphite mit einem Hydroxyphenylmethanon-Rückgrat, und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Die eingesetzten erfindungsgemäßen Verbindungen I-23 bis I-26 zeigten gute Umsätze von bis zu 99% bei der Hydroformylierung von 2-Penten in die entsprechenden Aldehyde.

Hierbei zeigte sich auch, dass die katalytische Aktivität der eingesetzten Verbindungen I-23 bis I-26 in der Hydroformylierung insbesondere in Kombination mit Rhodium-Metallzentren besonders effektiv ist.

Des Weiteren zeigen die Ergebnisse, dass die erfindungsgemäßen Verbindungen I-23 bis I-26 ebenfalls dazu in der Lage sind, gute Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier beispielhaft untersuchten Substrat 2-Penten, eine besonders schwer einzustellende Größe ist. Folglich sind die mit den erfindungsgemäßen Verbindungen I-23 bis I-26 erzielten n-Selektivitäten von bis zu 44,7% sehr gute Resultate.

### 2.3) Hydroformylierung mit erfindungsgemäßen Verbindungen 1-27 bis 1-29 - Phosphite und Phosphine mit Dimethyldioxolan-Rückgrat:

**Tabelle 3. Ergebnisse erfindungsgemäßer Verbindungen I-27 bis I-29 in der Hydroformylierung von 2-Penten**

| Verbindung | Umsatz (%) | n-Selektivität (%) |
|---|---|---|
| I-27 | 19 | 22.7 |
| I-28 | 86 | 33.7 |
| I-29 | 38 | 25.0 |

Anhand der in Tabelle 3 dargestellten Ergebnisse konnte gezeigt werden, dass die erfindungsgemäßen Verbindungen I-27 bis I-29 gemäß der dritten bevorzugten Ausgestaltung erfindungsgemäßer Verbindungen, d.h. Phosphite oder Phosphine mit einem Dimethyldioxolan-Rückgrat, und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Die eingesetzten erfindungsgemäßen Verbindungen I-27 bis I-29 zeigten gute Umsätze von bis zu 86% bei der Hydroformylierung von 2-Penten in die entsprechenden Aldehyde.

Hierbei zeigte sich auch, dass die katalytische Aktivität der eingesetzten Verbindungen I-27 bis I-29 in der Hydroformylierung insbesondere in Kombination mit Rhodium-Metallzentren besonders effektiv ist.

Des Weiteren zeigen die Ergebnisse, dass die erfindungsgemäßen Verbindungen I-27 bis I-29 ebenfalls dazu in der Lage sind, gute Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier beispielhaft untersuchten Substrat 2-Penten, eine besonders schwer einzustellende Größe ist. Folglich sind die mit den erfindungsgemäßen Verbindungen I-27 bis I-29 erzielten n-Selektivitäten von bis zu 33,7% sehr gute Resultate.

### 2.4) Hydroformylierung mit erfindungsgemäßen Verbindungen 1-30 bis 1-39 - Phosphite mit offenem oder geschlossenem Dibenzofuran-Rückgrat:

**Tabelle 4. Ergebnisse erfindungsgemäßer Verbindungen I-30 bis I-39 in der Hydroformylierung von 2-Penten**

| Verbindung | Umsatz (%) | n-Selektivität (%) |
|---|---|---|
| I-30 | 91 | 45,8 |
| I-31 | 95 | 46,3 |
| I-33 | 97,8 | 46,5 |
| I-34 | 87,5 | 54,6 |
| I-35 | 98,5 | 48,6 |
| I-36 | 96 | 44,5 |
| I-37 | 99 | 44,0 |
| I-38 | 0 | - |
| I-39 | 98,5 | 43,7 |

Anhand der in Tabelle 4 dargestellten Ergebnisse konnte gezeigt werden, dass die erfindungsgemäßen Verbindungen I-30 bis I-39 gemäß der vierten bevorzugten Ausgestaltung erfindungsgemäßer Verbindungen, d.h. Phosphite mit offenem oder geschlossenem Dibenzofuran-Rückgrat, und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Die eingesetzten erfindungsgemäßen Verbindungen I-30 bis I-39 zeigten gute Umsätze von bis zu 99% bei der Hydroformylierung von 2-Penten in die entsprechenden Aldehyde.

Hierbei zeigte sich auch, dass die katalytische Aktivität der eingesetzten Verbindungen I-30 bis I-39 in der Hydroformylierung insbesondere in Kombination mit Rhodium-Metallzentren besonders effektiv ist.

Des Weiteren zeigen die Ergebnisse, dass die erfindungsgemäßen Verbindungen I-30 bis I-39 ebenfalls dazu in der Lage sind, gute Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier beispielhaft untersuchten Substrat 2-Penten, eine besonders schwer einzustellende Größe ist. Folglich sind die mit den erfindungsgemäßen Verbindungen I-30 bis I-39 erzielten n-Selektivitäten von bis zu 54,6% sehr gute Resultate.

### 2.5) Hydroformylierung mit erfindungsgemäßen Verbindungen 1-40 bis 1-44 - Phosphite mit Cyclohexan-Bis-Methylphenol-Rückgrat:

**Tabelle 5. Ergebnisse erfindungsgemäßer Verbindungen I-40 bis I-44 in der Hydroformylierung von 2-Penten**

| Verbindung | Umsatz (%) | n-Selektivität (%) |
|---|---|---|
| I-40 | 99 | 40,4 |
| I-41 | 6 | 24,2 |
| I-42 | 94 | 19,9 |
| I-43 | 22 | 29,1 |
| I-44 | 0 | - |

Anhand der in Tabelle 5 dargestellten Ergebnisse konnte gezeigt werden, dass die erfindungsgemäßen Verbindungen I-40 bis I-44 gemäß der fünften bevorzugten Ausgestaltung erfindungsgemäßer Verbindungen, d.h. Phosphite mit Cyclohexan-Bis-Methylphenol-Rückgrat, und entsprechende Substanzen sowie auch das erfindungsgemäße Verfahren in der Hydroformylierung von Olefinen, hier beispielhaft gezeigt an dem Substrat 2-Penten, hervorragende Resultate zeigen.

Die eingesetzten erfindungsgemäßen Verbindungen I-40 bis I-44 zeigten gute Umsätze von bis zu 99% bei der Hydroformylierung von 2-Penten in die entsprechenden Aldehyde.

Hierbei zeigte sich auch, dass die katalytische Aktivität der eingesetzten Verbindungen I-40 bis I-44 in der Hydroformylierung insbesondere in Kombination mit Rhodium-Metallzentren besonders effektiv ist.

Des Weiteren zeigen die Ergebnisse, dass die erfindungsgemäßen Verbindungen I-40 bis I-44 ebenfalls dazu in der Lage sind, gute Resultate in Bezug auf die n-Selektivität bei der Hydroformylierung von Olefinen zu erzielen. Hierbei ist hervorzuheben, dass die n-Selektivität bei der Hydroformylierung von Olefin-Substraten mit innenliegenden Doppelbindungen, wie bei dem hier beispielhaft untersuchten Substrat 2-Penten, eine besonders schwer einzustellende Größe ist. Folglich sind die mit den erfindungsgemäßen Verbindungen I-40 bis I-44 erzielten n-Selektivitäten von bis zu 40,4% sehr gute Resultate.

## Patentansprüche

1. Verbindung, wobei die Verbindung ausgewählt ist aus den folgenden Formeln (I-1) bis (I-44)

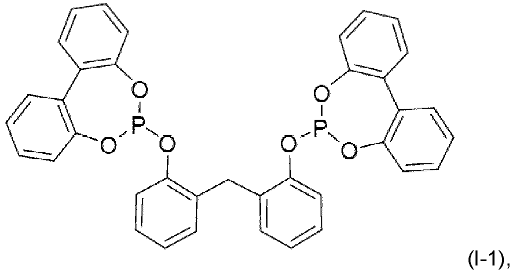

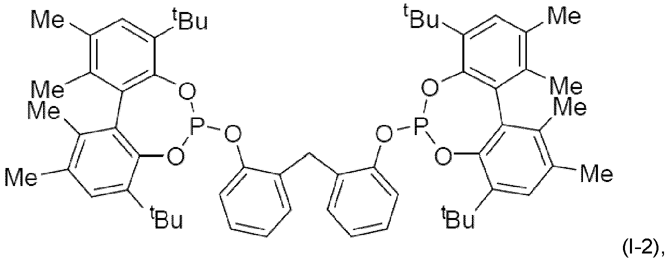

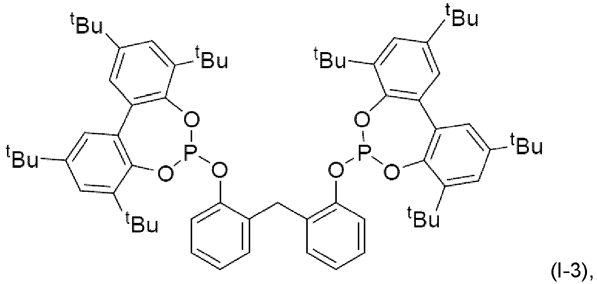

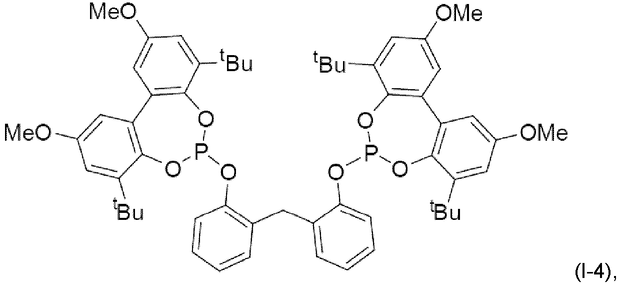

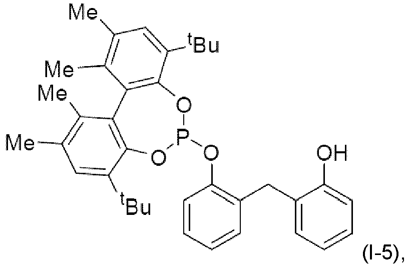

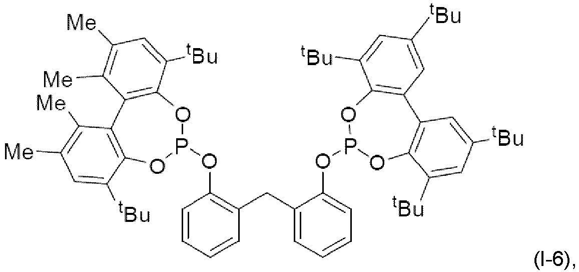

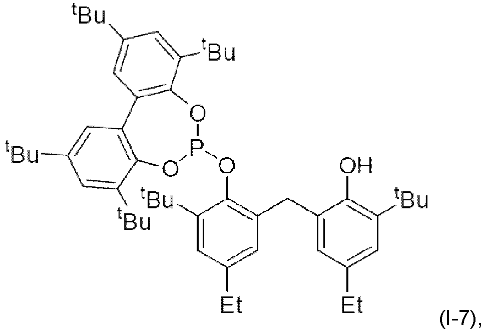

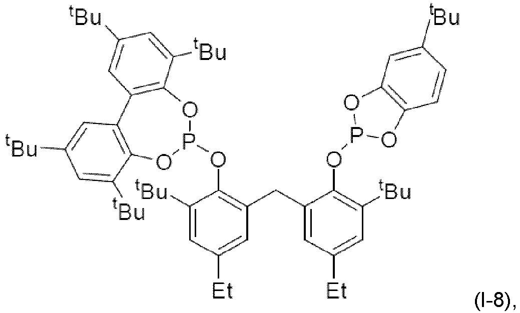

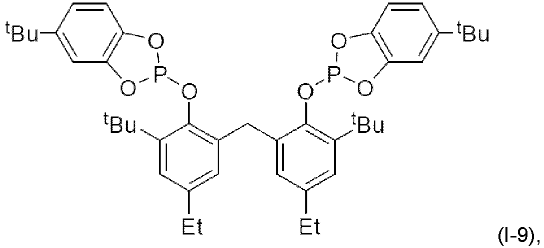

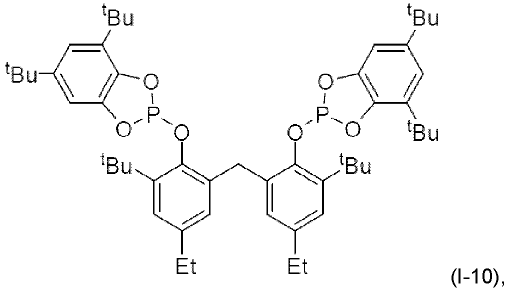

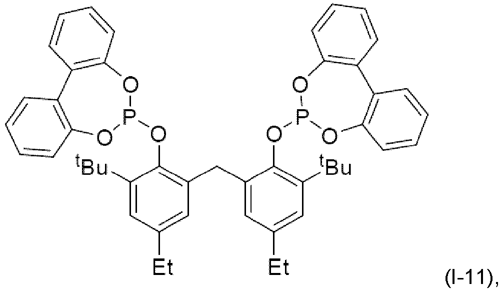

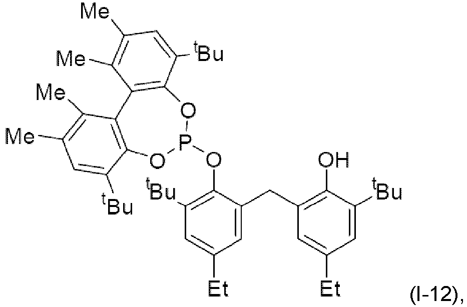

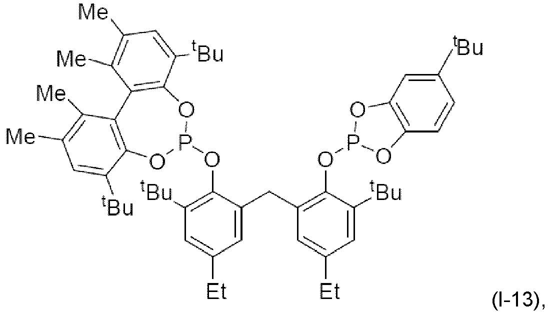

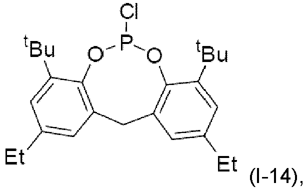

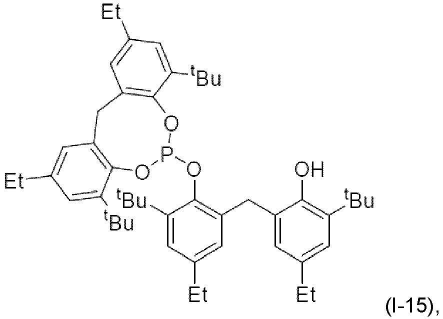

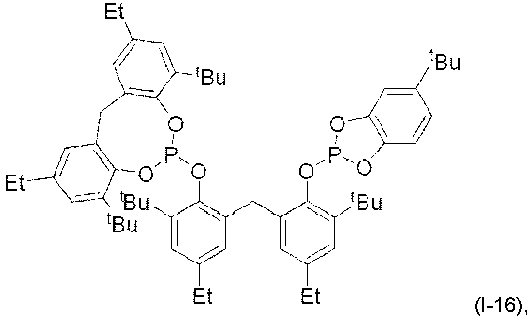

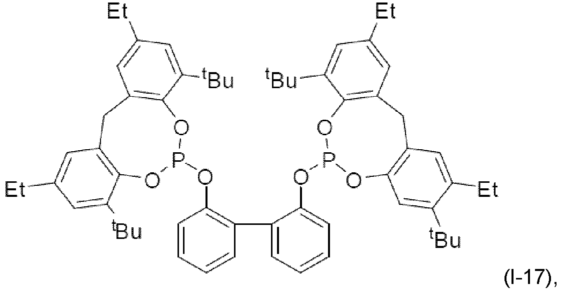

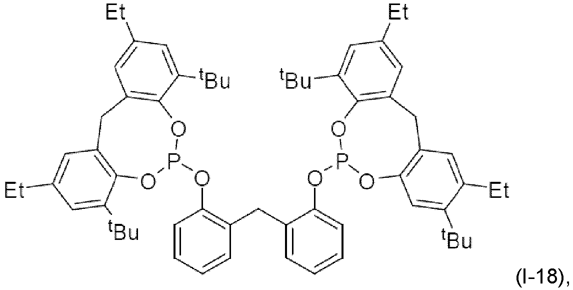

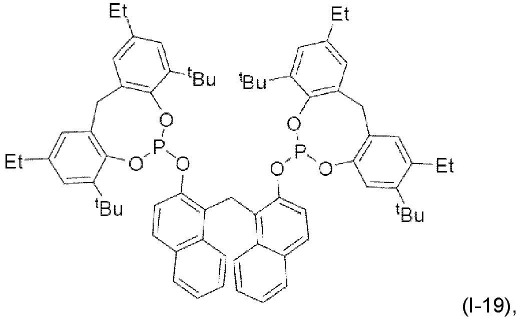

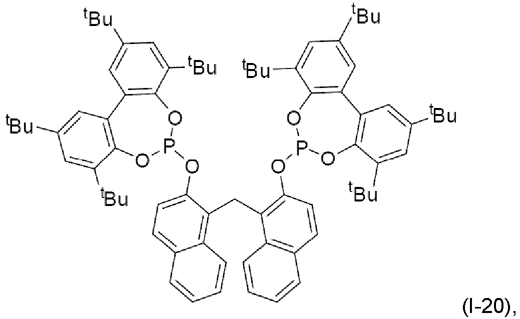

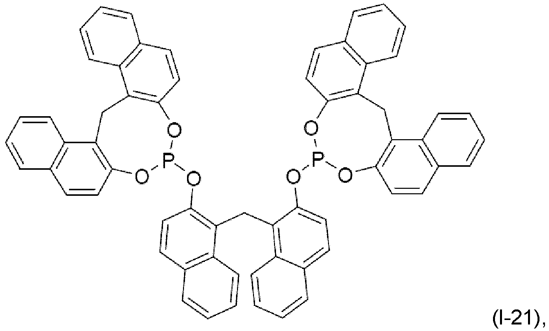

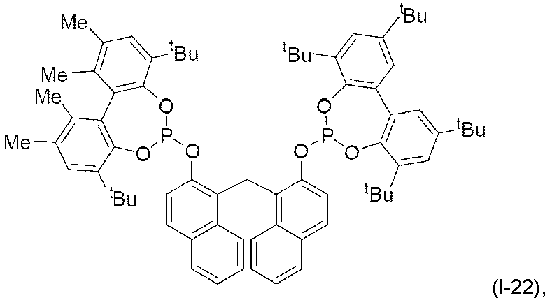

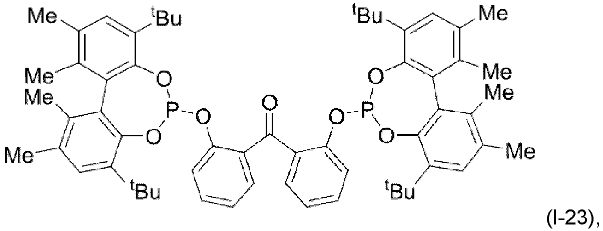

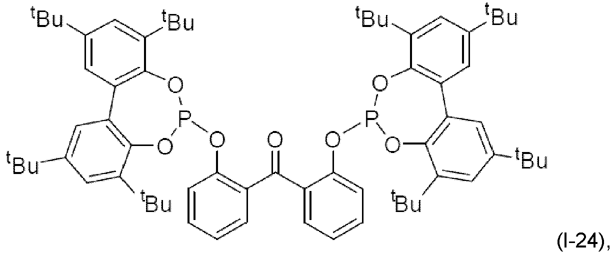

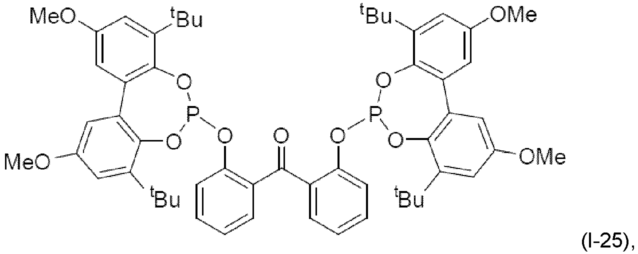

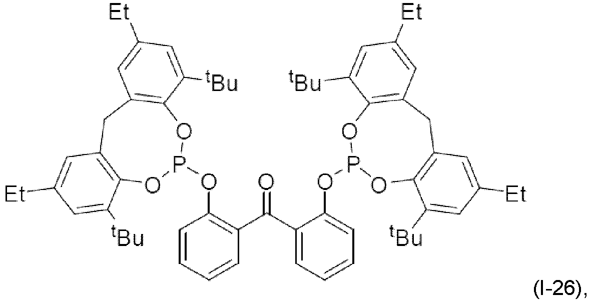

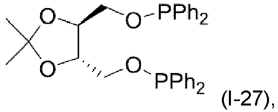

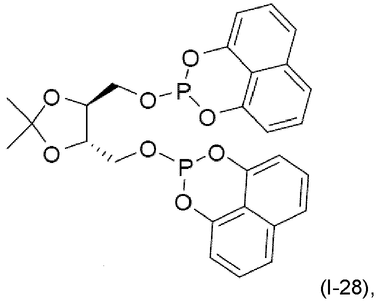

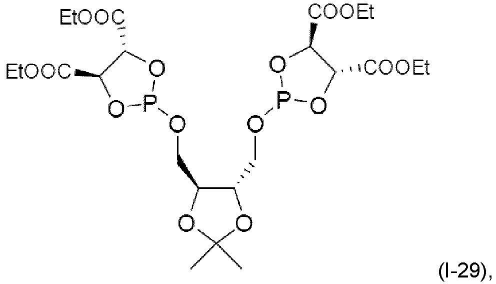

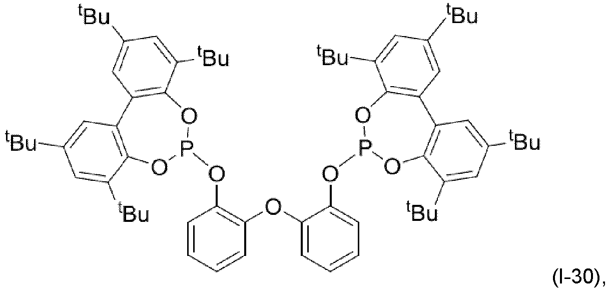

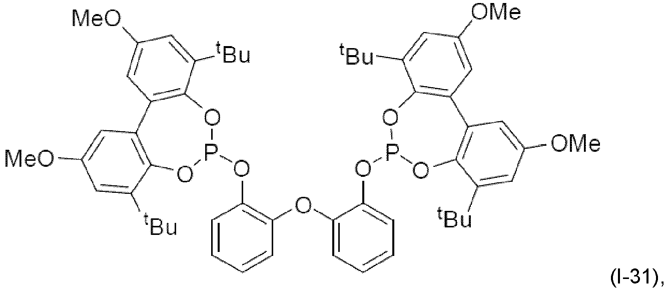

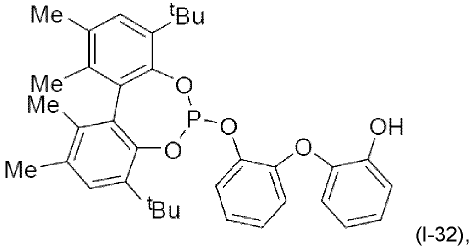

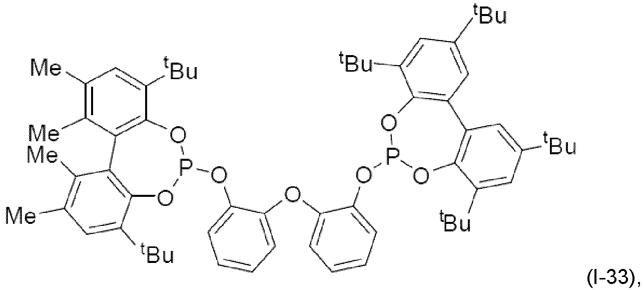

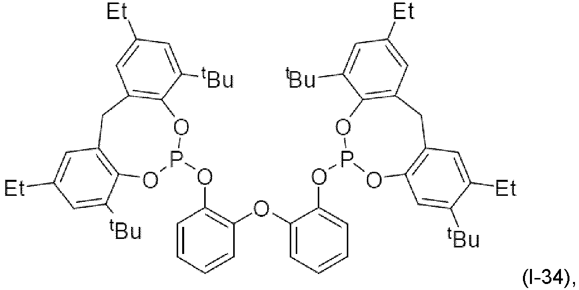

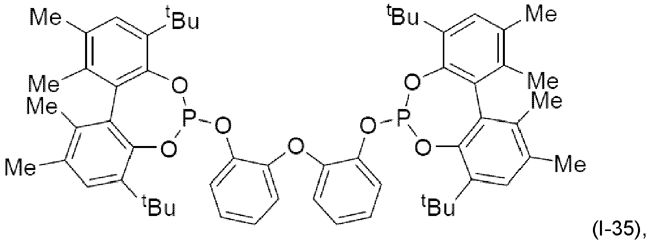

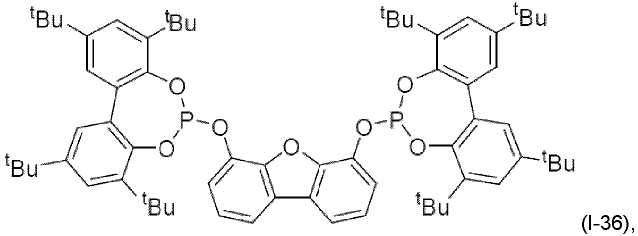

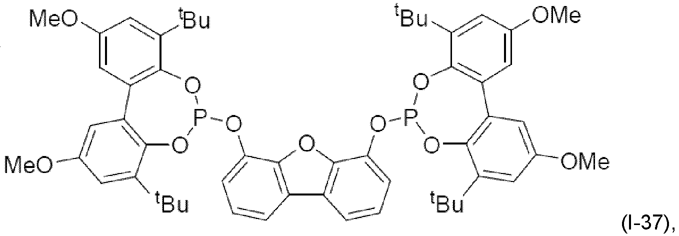

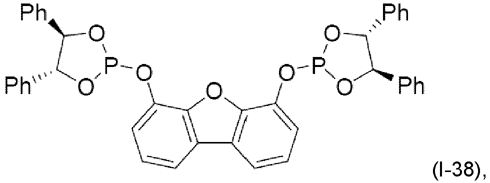

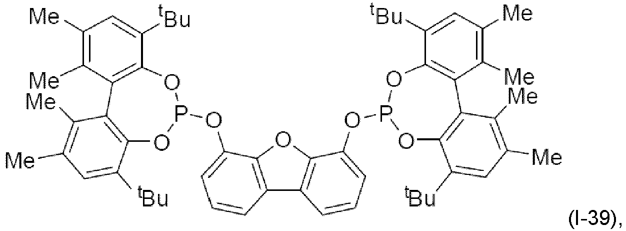

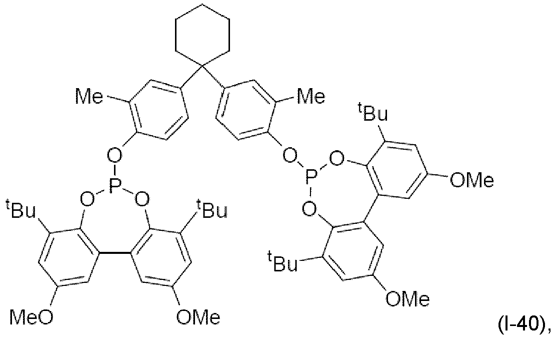

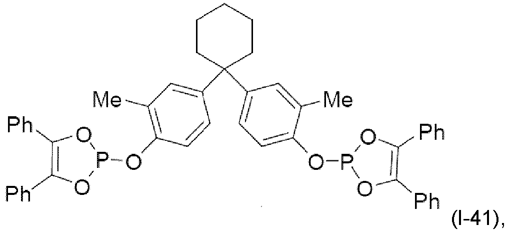

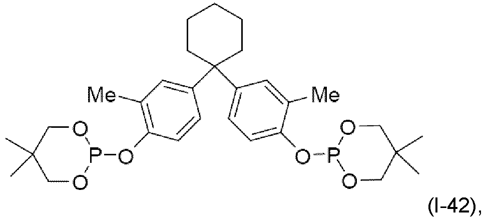

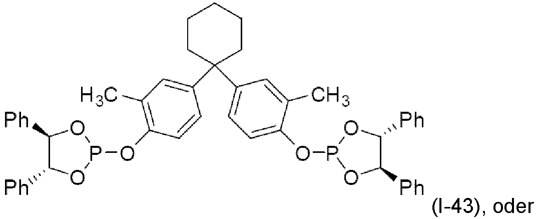

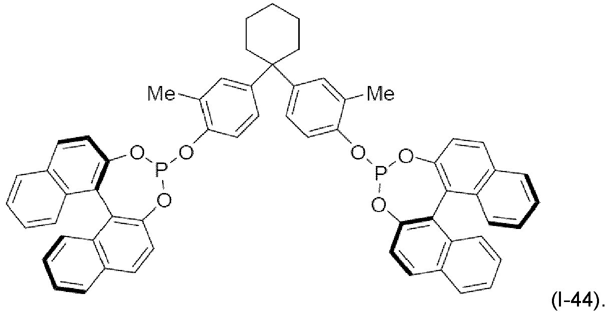

2. Substanz, wobei die Substanz
i) eine Mischung ist, die Mischung umfassend
- eine oder mehrere der Verbindungen ausgewählt aus den Formeln (I-1) bis (I-44) nach Anspruch 1,
und
- eines oder mehrere Metallatome,
und/oder
ii) ein Metallkomplex ist oder einen Metallkomplex enthält, der Metallkomplex umfassend
- eine oder mehrere der Verbindungen ausgewählt aus den Formeln (I-1) bis (I-44) nach Anspruch 1,
und
- eines oder mehrere Metallatome.

3. Substanz nach Anspruch 2, wobei
- das molare Verhältnis von Metallatomen zu Verbindungen gemäß Formeln (I-1) bis (I-44) in der Mischung und/oder im Metallkomplex im Bereich von 0,5:1 bis 1:4 liegt,
bevorzugt im Bereich von 0,9:1 bis 1:2.1 liegt, besonders bevorzugt 1:1 oder 1:2 ist,
und/oder
- das eine oder eines der mehreren Metallatome in der Mischung und/oder im Metallkomplex ausgewählt ist aus der Gruppe bestehend aus Rhodium, Cobalt, Eisen, Ruthenium, Osmium, Iridium, Platin und Zinn,
bevorzugt ist das eine oder eines der mehreren Metallatome Rhodium,
und/oder
- die Mischung als weiteren Bestandteil umfasst
- ein oder mehrere Olefine mit jeweils einer oder mehreren Kohlenstoff-Doppelbindungen,
bevorzugt ausgewählt aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
und/oder
- Lösungsmittel, wobei das Lösungsmittel vorzugsweise Toluol umfasst oder Toluol ist.

4. Verfahren zur Herstellung von Aldehyden umfassend die Verfahrensschritte
a) Herstellen oder Bereitstellen eines Reaktionssystems umfassend
a-1) eine erste Komponente umfassend ein oder mehrere Olefine mit jeweils einer oder mehreren Kohlenstoff-Doppelbindungen,
a-2) eine Katalysatorkomponente umfassend eine Verbindung gemäß Anspruch 1 und eines oder mehrere Metallatome,
a-3) eine zweite Komponente umfassend H₂ und CO
b) Erwärmen des Reaktionssystems, sodass Kohlenstoff-Doppelbindungen des einen oder mindestens eines der mehreren Olefine zu Aldehyden umgesetzt werden.

5. Verfahren nach Anspruch 4, wobei das eine oder die mehreren Olefine ausgewählt ist/sind aus der Gruppe bestehend aus Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen und Di-n-Buten,
bevorzugt ausgewählt aus der Gruppe bestehend aus cis- und/oder trans-2-Penten.

6. Verfahren nach einem der Ansprüche 4 oder 5, wobei
- das eine oder eines der mehreren Metallatome der Katalysatorkomponente ausgewählt ist aus der Gruppe bestehend aus Rhodium, Cobalt, Eisen, Ruthenium, Osmium, Iridium, Platin und Zinn,
bevorzugt ist das eine oder eines der mehreren Metallatome Rhodium,
besonders bevorzugt wird das Rhodium bereitgestellt aus einer der folgenden Verbindungen: Rh(acac)(CO)₂, [(acac)Rh(COD)] und Rh₄CO₁₂,
und/oder
- das molare Verhältnis von Metallatomen zu Verbindungen gemäß Formeln (I-1) bis (I-44) in der Katalysatorkomponente im Bereich von 1:0,8 bis 1:4 liegt,
bevorzugt im Bereich von 1:0,9 bis 1:2,1 liegt,
besonders bevorzugt 1:1 oder 1:2 ist,
und/oder
- die Katalysatorkomponente ein Metallkomplex ist oder einen Metallkomplex enthält, der Metallkomplex umfassend eine oder mehrere der Verbindungen nach Anspruch 1 und Rhodium.

7. Verfahren nach einem der Ansprüche 4 bis 6, wobei die zweite Komponente umfassend H₂ und CO ein Gemisch aus H₂ und CO mit einem Stoffmengenverhältnis von H₂ zu CO im Bereich von 2:1 bis 1:2 ist, bevorzugt im Bereich von 1,5:1 bis 1:1,5, besonders bevorzugt im Bereich von 1,1:1 bis 1:1,1.

8. Verfahren nach einem der Ansprüche 4 bis 7, wobei das Reaktionssystem in Verfahrensschritt b)
- unter einem Druck im Bereich von 1 bis 30 bar steht, bevorzugt unter einem Druck im Bereich von 1,5 bar bis 25 bar steht,
und/oder
- auf eine Temperatur im Bereich von 80 °C bis 160 °C erwärmt wird, bevorzugt auf eine Temperatur im Bereich von 100 °C bis 140 °C erwärmt wird.

9. Verfahren nach einem der Ansprüche 4 bis 8, wobei
- das Reaktionssystem zusätzlich ein Lösungsmittel umfasst, wobei das Lösungsmittel vorzugsweise Toluol umfasst oder Toluol ist,
und/oder
- der Stoffmengenanteil von Rhodium im Reaktionssystem im Bereich von 10 µmol/mol bis 1000 µmol/mol liegt, bevorzugt im Bereich von 20 µmol/mol bis 500 µmol/mol liegt, besonders bevorzugt im Bereich von 50 µmol/mol bis 200 µmol/mol liegt, jeweils bezogen auf die Stoffmenge an Olefinen im Reaktionssystem,
und/oder
- wobei das Stoffmengenverhältnis von Rhodium zu den Verbindungen gemäß Anspruch 1 in der Katalysatorkomponente im Bereich von 1:1 bis 1:3 liegt, bevorzugt im Bereich von 1:1,5 bis 1:2,5 liegt, besonders bevorzugt im Bereich von 1:1,9 bis 1:2,1 liegt.

10. Verfahren nach einem der Ansprüche 4 bis 9, umfassend die Verfahrensschritte
V1) Vorlegen eines Olefins;
V2) Zugabe einer Verbindung gemäß Anspruch 1 und einer Substanz welche Rhodium umfasst;
V3) Zuführen von H₂ und CO;
V4) Erwärmen des Reaktionssystems aus V1) bis V3), wobei das Olefin zu einem Aldehyd umgesetzt wird.

11. Verwendung einer Verbindung nach Anspruch 1 als Ligand eines Katalysators oder einer Substanz nach einem der Ansprüche 2 oder 3 als Katalysator in einer Hydroformylierungsreaktion, bevorzugt in einer Hydroformylierungsreaktion gemäß einem Verfahren wie in den Ansprüchen 4 bis 10 definiert.
